(19)

**Europäisches Patentamt**
**European Patent Office**
**Office européen des brevets**

(11) **EP 4 614 156 A2**

(12) **EUROPEAN PATENT APPLICATION**

(43) Date of publication:
**10.09.2025 Bulletin 2025/37**

(21) Application number: **25191181.4**

(22) Date of filing: **19.10.2021**

(51) International Patent Classification (IPC):
**G01N 33/68** (2006.01)

(52) Cooperative Patent Classification (CPC):
**G01N 33/6887; G01N 33/6893;** G01N 2800/32;
G01N 2800/50; G01N 2800/60

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.10.2020 US 202063093993 P**

(62) Document number(s) of the earlier application(s) in
accordance with Art. 76 EPC:
**21805807.1 / 4 232 825**

(71) Applicant: **SomaLogic Operating Co., Inc.**
**Boulder, CO 80301 (US)**

(72) Inventors:
• **SAMPSON, Laura Mae**
  **Boulder 80301 (US)**
• **HINTERBERG, Michael Albert**
  **Boulder 80301 (US)**

• **JIA, Yi**
  **Boulder 80301 (US)**
• **OSTROFF, Rachel Marie**
  **Boulder 80301 (US)**
• **HAGAR, Yolanda**
  **Boulder 80301 (US)**

(74) Representative: **Mewburn Ellis LLP**
**Aurora Building**
**Counterslip**
**Bristol BS1 6BX (GB)**

Remarks:
This application was filed on 23-07-2025 as a
divisional application to the application mentioned
under INID code 62.

(54) **CARDIOVASCULAR EVENT RISK PREDICTION**

(57) Biomarkers, methods, devices, reagents, systems, and kits used to assess an individual having heart failure with preserved ejection fraction (HFpEF) or having heart failure with reduced ejection fraction (HFrEF) for the prediction of risk of developing a Cardiovascular (CV) Event over a 90 day, 180 day, or 1 year period are provided.

*Fig. 1*

EP 4 614 156 A2

**Description**

**FIELD OF THE INVENTION**

The present application relates generally to the detection of biomarkers and a method of evaluating the risk of a future cardiovascular event in an individual with chronic heart failure and, more specifically, to one or more biomarkers, methods, devices, reagents, systems, and kits used to assess an individual for the prediction of risk of developing a cardiovascular (CV) event. Such events include but are not limited to hospitalization and death.

**BACKGROUND**

Cardiovascular disease is the leading cause of death in the USA. There are a number of existing and important predictors of risk of primary events (D'Agostino, R et al., "General Cardiovascular Risk Profile for Use in Primary Care: The Framingham Heart Study" Circulation 117:743-53 (2008); and Ridker, P. et al., "Development and Validation of Improved Algorithms for the Assessment of Global Cardiovascular Risk in Women" JAMA 297(6):611-619 (2007)) and secondary events (Shlipak, M. et al. "Biomarkers to Predict Recurrent Cardiovascular Disease: The Heart & Soul Study" Am. J. Med. 121:50-57 (2008)) which are widely used in clinical practice and therapeutic trials. Unfortunately, the receiver-operating characteristic curves, hazard ratios, and concordance show that the performance of existing risk factors and biomarkers is modest (AUCs of -0.75 mean that these factors are only halfway between a coin-flip and perfection). In addition to a need for improved diagnostic performance, there is a need for a risk product which is both near-term and personally responsive within individuals to beneficial (and destructive) interventions and lifestyle changes. The commonly utilized Framingham equation has three main problems. Firstly, it is too long term: it gives 10-year risk calculations but humans discount future risks and are reluctant to make behavior and lifestyle modifications based on them. Secondly, it is not very responsive to interventions: it is heavily dependent on chronological age, which cannot decline; and gender, which cannot change. Thirdly, within the high risk population envisioned here, the Framingham factors fail to discriminate well between high and low risk: the hazard ratio between high and low quartiles is only 2, and when one attempts to use Framingham scores to personalize risk by stratifying subjects into finer layers (deciles for example), the observed event rates are similar for many of the deciles.

Risk factors for cardiovascular disease are widely used to drive the intensity and the nature of medical treatments, and their use has undoubtedly contributed to the reduction in cardiovascular morbidity and mortality that has been observed over the past two decades. These factors have routinely been combined into algorithms but unfortunately they do not capture all the risk (the most common initial presentation for heart disease is still death). In fact, they probably only capture half the risk. An area under the ROC curve of -0.76 is typical for such risk factors in primary prevention, with much worse performance in secondary prevention (0.62 is typical), numbers only about one quarter to one half of the performance between a coin-flip at 0.5 and perfection at 1.0.

Moreover, in the Framingham study (Wang et al., "Multiple Biomarkers for the Prediction of First Major Cardiovascular Events and Death" N. Eng. J. Med. 355:2631-2637 (2006)) in 3209 people, the addition of 10 biomarkers (CRP, BNP, NT-proBNP, aldosterone, renin, fibrinogen, D-dimer, plasminogen-activator inhibitor type 1, homocysteine and the urinary albumin to creatinine ratio) did not significantly improve the AUC when added to existing risk factors: the AUC for events 0-5 years was 0.76 with age, sex and conventional risk factors and 0.77 with the best combination of biomarkers added to the mix, and for secondary prevention the situation is worse.

Early identification of patients with higher risk of a cardiovascular event within a 1-year window is important because more aggressive treatment of individuals with elevated risk may improve outcomes. Thus, optimal management requires aggressive intervention to reduce the risk of a cardiovascular event in those patients who are considered to have a higher risk, while patients with a lower risk of a cardiovascular event can be spared expensive and potentially invasive treatments, which are likely to have no beneficial effect to the patient.

Biomarker selection for the prediction of risk of having specific disease state or condition within a defined time period involves first the identification of markers that have a measurable and statistically significant relationship with the probability and/or timing of an event for a specific medical application. Biomarkers can include secreted or shed molecules that are either on the causal pathway to the condition of interest, or which are downstream or parallel to the disease or condition development or progression, or both. They are released into the blood stream from cardiovascular tissue or from other organs and surrounding tissues and circulating cells in response to the biological processes which predispose to a cardiovascular event or they may be reflective of downstream effects of the pathophysiology such as a decline in kidney function. Biomarkers can include small molecules, peptides, proteins, and nucleic acids. Some of the key issues that affect the identification of biomarkers include over-fitting of the available data and bias in the data.

A variety of methods have been utilized in an attempt to identify biomarkers and diagnose or predict the risk of having disease or a condition. For protein-based markers, these include two-dimensional electrophoresis, mass spectrometry, and immunoassay methods. For nucleic acid markers, these include mRNA expression profiles, microRNA profiles, FISH, serial analysis of gene expression (SAGE), large scale gene expression arrays, gene sequencing and genotyping (SNP or small variant analysis).

The utility of two-dimensional electrophoresis is limited by low detection sensitivity; issues with protein solubility, charge, and hydrophobicity; gel reproducibility; and the possibility of a single spot representing multiple proteins. For mass spectrometry, depending on the format used, limitations revolve around the sample processing and separation, sensitivity to low abundance proteins, signal to noise considerations, and inability to immediately identify the detected protein. Limitations in immunoassay approaches to biomarker discovery are centered on the inability of antibody-based multiplex assays to measure a large number of analytes. One might simply print an array of high-quality antibodies and, without sandwiches, measure the analytes bound to those antibodies. (This would be the formal equivalent of using a whole genome of nucleic acid sequences to measure by hybridization all DNA or RNA sequences in an organism or a cell. The hybridization experiment works because hybridization can be a stringent test for identity.) However, even very good antibodies are typically not stringent enough in selecting their binding partners to work in the context of blood or even cell extracts because the protein ensemble in those matrices have widely varying abundances, which can lead to poor signal to noise ratios. Thus, one must use a different approach with immunoassay-based approaches to biomarker discovery - one would need to use multiplexed ELISA assays (that is, sandwiches) to get sufficient stringency to measure many analytes simultaneously to decide which analytes are indeed biomarkers. Sandwich immunoassays do not scale to high content, and thus biomarker discovery using stringent sandwich immunoassays is not possible using standard array formats. Lastly, antibody reagents are subject to substantial lot variability and reagent instability. The instant platform for protein biomarker discovery overcomes this problem.

Many of these methods rely on or require some type of sample fractionation prior to the analysis. Thus, the sample preparation required to run a sufficiently powered study designed to identify and discover statistically relevant biomarkers in a series of well-defined sample populations is extremely difficult, costly, and time consuming. During fractionation, a wide range of variability can be introduced into the various samples. For example, a potential marker could be unstable to the process, the concentration of the marker could be changed, inappropriate aggregation or disaggregation could occur, and inadvertent sample contamination could occur and thus obscure the subtle changes anticipated in early disease.

It is widely accepted that biomarker discovery and detection methods using these technologies have serious limitations for the identification of diagnostic or predictive biomarkers. These limitations include an inability to detect low-abundance biomarkers, an inability to consistently cover the entire dynamic range of the proteome, irreproducibility in sample processing and fractionation, and overall irreproducibility and lack of robustness of the method. Further, these studies have introduced biases into the data and not adequately addressed the complexity of the sample populations, including appropriate controls, in terms of the distribution and randomization required to identify and validate biomarkers within a target disease population.

Although efforts aimed at the discovery of new and effective biomarkers have gone on for several decades, the efforts have been largely unsuccessful. Biomarkers for various diseases typically have been identified in academic laboratories, usually through an accidental discovery while doing basic research on some disease process. Based on the discovery and with small amounts of clinical data, papers were published that suggested the identification of a new biomarker. Most of these proposed biomarkers, however, have not been confirmed as real or useful biomarkers, primarily because the small number of clinical samples tested provide only weak statistical proof that an effective biomarker has in fact been found. That is, the initial identification was not rigorous with respect to the basic elements of statistics.

Based on the history of failed biomarker discovery efforts, theories have been proposed that further promote the general understanding that biomarkers for diagnosis, prognosis or prediction of risk of developing diseases and conditions are rare and difficult to find. Biomarker research based on 2D gels or mass spectrometry supports these notions. Very few useful biomarkers have been identified through these approaches. However, it is usually overlooked that 2D gel and mass spectrometry measure proteins that are present in blood at approximately 1 nM concentrations and higher, and that this ensemble of proteins may well be the least likely to change with disease or the development of a particular condition. Other than the instant biomarker discovery platform, proteomic biomarker discovery platforms that are able to accurately measure protein expression levels at much lower concentrations do not exist.

Much is known about biochemical pathways for complex human biology. Many biochemical pathways culminate in or are started by secreted proteins that work locally within the pathology; for example, growth factors are secreted to stimulate the replication of other cells in the pathology, and other factors are secreted to ward off the immune system, and so on. While many of these secreted proteins work in a paracrine fashion, some operate distally in the body. One skilled in the art with a basic understanding of biochemical pathways would understand that many pathology-specific proteins ought to exist in blood at concentrations below (even far below) the detection limits of 2D gels and mass spectrometry. What must precede the identification of this relatively abundant number of disease biomarkers is a proteomic platform that can analyze proteins at concentrations below those detectable by 2D gels or mass spectrometry.

Chronic heart failure, also known as congestive heart failure (CHF), occurs when the heart cannot pump enough blood and oxygen to support the normal functions of other organs, and is the most common cause of hospitalization in individuals over age 65. Left ventricular ejection fraction (LVEF), measured by echocardiogram, shows how much blood the left ventricle pumps out with each heart contraction. Based on ejection fraction (EF) level, HF can be classified into HF with reduced ejection fraction, HFrEF (LVEF <40%); HF with preserved ejection fraction, HFpEF (LVEF ≥50%); and HF with mid-range ejection fraction, HFmrEF (LVEF of 40% to 49%).

As is discussed above, cardiovascular events may be prevented by aggressive treatment if the propensity for such events can be accurately determined, and by targeting such interventions at the people who need them the most and/or away from people who need them the least, medical resourcing efficiency can be improved and costs may be lowered at the same time. Additionally, when the patient has the knowledge of accurate and near-term information about their personal likelihood of cardiovascular events, this is less deniable than long-term population-based information and will lead to improved lifestyle choices and improved compliance with medication which will add to the benefits. Existing multi-marker tests either require the collection of multiple samples from an individual or require that a sample be partitioned between multiple assays. Optimally, an improved test would require only a single blood, urine or other sample type, and a single assay. Accordingly, a need exists for biomarkers, methods, devices, reagents, systems, and kits that enable the prediction of cardiovascular events within a 1-year period.

SUMMARY OF THE INVENTION

The present application includes biomarkers, methods, reagents, devices, systems, and kits for the prediction of risk of an individual with chronic heart failure, such as stable chronic heart failure, having a Cardiovascular (CV) Event within a 90-day period, a 180-day period, or a 1-year period. The biomarkers of the present application were identified using a multiplex slow off-rate aptamer-based assay which is described in detail herein. By using the multiplex slow off-rate aptamer-based biomarker identification method described herein, this application describes a set of biomarkers that are useful for predicting the likelihood of a CV event in patients with chronic heart failure, such as stable chronic heart failure, within 90 days, 180 days, or 1 year.

In some embodiments, the individual has stable chronic heart failure with preserved ejection fraction (HFpEF). In some embodiments, the individual has reduced ejection fraction (HFrEF). In some embodiments, the 1-year mortality prognosis of the individual is predicted.

Cardiovascular disease involves multiple biological processes and tissues. Examples of biological systems and processes associated with cardiovascular disease are inflammation, thrombosis, disease-associated angiogenesis, platelet activation, macrophage activation, liver acute response, extracellular matrix remodeling, and renal function. These processes can be observed as a function of gender, menopausal status, and age, and according to status of coagulation and vascular function. Since these systems communicate partially through protein based signaling systems, and multiple proteins may be measured in a single blood sample, the invention provides a single sample, single assay multiple protein based test focused on proteins from the specific biological systems and processes involved in chronic heart failure.

In some embodiments, methods for screening a subject for the risk of a cardiovascular event (CV) event are provided, said method comprising forming a biomarker panel having N biomarker proteins, and detecting the level of each of the N biomarker proteins in a sample from the subject, wherein N is at least 2, and wherein a) at least two of the N biomarker proteins are selected from HCC-1, RNAS6, PAP1, SVEP1, and ATL2; or b) at least one of the N biomarker proteins is selected from HCC-1, RNAS6, PAP1, SVEP1, and ATL2, and at least one of the N biomarker proteins is selected from N-terminal pro-BNP, RSPO4, BNP, MIC-1, FABPA, ILRL1, ANGP2, HE4, TAGL, RNAS1, and TSP2.

In some embodiments, methods of predicting the likelihood that a subject will have a CV event are provided, said method comprising forming a biomarker panel having N biomarker proteins, and detecting the level of each of the N biomarker proteins in a sample from the subject, wherein N is at least 2, and wherein a) at least two of the N biomarker proteins are selected from HCC-1, RNAS6, PAP1, SVEP1, and ATL2; or b) at least one of the N biomarker proteins is selected from HCC-1, RNAS6, PAP1, SVEP1, and ATL2, and at least one of the N biomarker proteins is selected from N-terminal pro-BNP, RSPO4, BNP, MIC-1, FABPA, ILRL1, ANGP2, HE4, TAGL, RNAS1, and TSP2.

In some embodiments, at least two of the N biomarker proteins are RNAS6 and PAP1. In some embodiments, at least two of the N biomarker proteins are RNAS6 and ATL2. In some embodiments, at least two of the N biomarker proteins are HCC-1 and PAP1. In some embodiments, at least two of the N biomarker proteins are HCC-1 and ATL2. In some embodiments, at least two of the N biomarker proteins are HCC-1 and RNAS6. In some embodiments, at least two of the N biomarker proteins are PAP1 and SVEP1. In some embodiments, at least two of the N biomarker proteins are HCC-1 and SVEP1. In some embodiments, at least two of the N biomarker proteins are RNAS6 and SVEP1. In some embodiments, at least two of the N biomarker proteins are PAP1 and ATL2. In some embodiments, all of the N biomarker proteins are selected from HCC-1, RNAS6, PAP1, SVEP1, ATL2, N-terminal pro-BNP, RSPO4, BNP, MIC-1, FABPA, ILRL1, ANGP2, HE4, TAGL, RNAS1, and TSP2. In some embodiments, one of the N biomarker proteins is MIC-1. In some embodiments, one of the N biomarker proteins is RNAS1. In any of the foregoing embodiments, N is 2, N is 3, N is 4, N is 5, N is 6, N is 7, N is 8, N is 9, N is 10, N is 11, N is 12, N is 13, N is 14, N is 15, or N is 16. In some embodiments, the subject has heart failure with reduced ejection fraction. In some embodiments, methods of predicting screening a subject for the risk of a a cardiovascular event (CV) event are provided, the method comprising forming a biomarker panel having N biomarker proteins, and detecting the level of each of the N biomarker proteins in a sample from the subject, wherein N is at least 2, wherein at least one of the N biomarker proteins is selected from RET and CRDL1, and at least one of the N biomarker proteins is selected from Tetranectin, N-terminal pro-BNP, TNNT2, CA125, MIC-1, SLPI, HE4, MMP-12, HSPB6, WISP-2, GHR, and IGFBP-2.

In some embodiments, methods of predicting the likelihood that a subject will have a CV event are provided, the method comprising forming a biomarker panel having N biomarker proteins, and detecting the level of each of the N biomarker proteins in a sample from the subject, wherein N is at least 2, wherein at least one of the N biomarker proteins is selected from RET and CRDL1, and at least one of the N biomarker proteins is selected from Tetranectin, N-terminal pro-BNP, TNNT2, CA125, MIC-1, SLPI, HE4, M1VIP-12, HSPB6, WISP-2, GHR, and IGFBP-2.

In some embodiments, at least two of the N biomarker proteins are RET and CRDL1. In some embodiments, one of the N biomarker proteins is MIC-1. In some embodiments, one of the N biomarker proteins is HE4. In some embodiments, one of the N biomarker proteins is Tetranectin. In some embodiments, one of the N biomarker proteins is GHR. In some embodiments, one of the N biomarker proteins is CA125. In some embodiments, one of the N biomarker proteins is N-terminal pro-BNP. In some embodiments, one of the N biomarker proteins is IGFBP-2. In some embodiments, one of the N biomarker proteins is WISP-2. In some embodiments, one of the N biomarker proteins is TNNT2. In some embodiments, one of the N biomarker proteins is HSPB6. In some embodiments, one of the N biomarker proteins is SLPI. In some embodiments, one of the N biomarker proteins is MMP-12. In some embodiments, all of the N biomarker proteins are selected from RET, CRDL1, Tetranectin, N-terminal pro-BNP, TNNT2, CA125, MIC-1, SLPI, HE4, MMP-12, HSPB6, WISP-2, GHR, and IGFBP-2. In any of the foregoing amendments, N is 2, N is 3, N is 4, N is 5, N is 6, N is 7, N is 8, N is 9, N is 10, Nis is 11, Nis is 12, Nis is 13, or N is 14. In some embodiments, the subject has heart failure with preserved ejection fraction.

In various embodiments, the CV event is death.

In some embodiments, the risk or likelihood of the subject having a CV event within 1 year from the date that the sample was taken from the subject is screened or predicted. In some embodiments, the risk or likelihood of the subject having a CV event within 180 days from the date that the sample was taken from the subject is screened or predicted. In some embodiments, the risk or likelihood of the subject having a CV event within 90 days from the date that the sample was taken from the subject is screened or predicted. In some embodiments, the risk or likelihood of the subject having a CV within 1 year, 180 days, or 90 days from the date that the sample was taken from the subject is high if the levels of each of at least 2 of the N biomarker proteins are abnormal relative to a control level of the respective biomarker protein. In some embodiments, the risk or likelihood of the subject having a CV within 1 year, 180 days, or 90 days from the date that the sample was taken from the subject is high if the levels of each of the N biomarker proteins are abnormal relative to a control level of the respective biomarker protein. In some embodiments, the risk or likelihood of the subject having a CV event within 1 year, 180 days, or 90 days from the date that the sample was taken from the subject is calculated as a probability of survival 1 year, 180 days, or 90 days from the date that the sample was taken from the subject.

In some embodiments, the sample is selected from a blood sample, a serum sample, a plasma sample, and a urine sample. In some embodiments, the sample is a blood sample. In some embodiments, the method is performed in vitro.

In some embodiments, the method comprises contacting biomarker proteins of the sample from the subject with a set of capture reagents, wherein each capture reagent of the set of capture reagents specifically binds to one biomarker protein being detected. In some embodiments, two of the capture reagents bind to the same biomarker protein being detected. In some embodiments, two capture reagents specifically bind to SVEP1, and wherein the two capture reagents are aptamers comprising different sequences. In some embodiments, the method comprises contacting biomarker proteins of the sample from the subject with a set of capture reagents, wherein each capture reagent of the set of capture reagents specifically binds to a different biomarker protein being detected. In some embodiments, each capture reagent is an antibody or an aptamer. In some embodiments, each biomarker capture reagent is an aptamer. In some embodiments, at least one aptamer is a slow off-rate aptamer. In some embodiments, at least one slow off-rate aptamer comprises at least one, at least two, at least three, at least four, at least five, at least six, at least seven, at least eight, at least nine, or at least 10 nucleotides with modifications. In some embodiments, each slow off-rate aptamer binds to its target protein with an off rate

($t^{1/2}$) of $\geq$ 30 minutes, $\geq$ 60 minutes, $\geq$ 90 minutes, $\geq$ 120 minutes, $\geq$ 150 minutes, $\geq$ 180 minutes, $\geq$ 210 minutes, or $\geq$ 240 minutes.

In some embodiments, the risk or likelihood of a CV event is based on the detected biomarker levels and at least one item of additional biomedical information selected from a) information corresponding to physical descriptors of the subject, b) information corresponding to a change in weight of the subject, c) information corresponding to the ethnicity of the subject, d) information corresponding to the gender of the subject, e) information corresponding to the subject's smoking history, f) information corresponding to the subject's alcohol use history, g) information corresponding to the subject's occupational history, h) information corresponding to the subject's family history of cardiovascular disease or other circulatory system conditions, i) information corresponding to the presence or absence in the subject of at least one genetic marker correlating with a higher risk of cardiovascular disease in the subject or a family member of the subject, j) information corresponding to clinical symptoms of the subject, k) information corresponding to other laboratory tests, 1) information corresponding to gene expression values of the subject, and m) information corresponding to the subject's consumption of known cardiovascular risk factors such as diet high in saturated fats, high salt, high cholesterol, n) information corresponding to the subject's imaging results obtained by techniques selected from the group consisting of electrocardiogram, echocardiography, carotid ultrasound for intima-media thickness, flow mediated dilation, pulse wave velocity, ankle-brachial index, stress echocardiography, myocardial perfusion imaging, coronary calcium by CT, high resolution CT angiography, MRI imaging, and other imaging modalities, o) information regarding the subject's medications, p) information corresponding to the age of the subject, and q) information regarding the subject's kidney function.

In some embodiments, the at least one item of additional biomedical information is information corresponding to the age of the subject. In some embodiments, the method comprises determining the risk or likelihood of a CV event for the purpose of determining a medical insurance premium or life insurance premium. In some embodiments, the method further comprises determining coverage or premium for medical insurance or life insurance. In some embodiments, the method further comprises using information resulting from the method to predict and/or manage the utilization of medical resources. In some embodiments, the method further comprises using information resulting from the method to enable a decision to acquire or purchase a medical practice, hospital, or company.

In some embodiments, a kit is provided, the kit comprising N biomarker protein capture reagents, wherein N is at least 2, and wherein at least one of the capture reagents binds to HCC-1, RNAS6, PAP1, SVEP1, or ATL2, and at least one of the capture reagents binds to N-terminal pro-BNP, RSPO4, BNP, MIC-1, FABPA, ILRL1, ANGP2, HE4, TAGL, RNAS1, or TSP2. In some embodiments, two of the capture reagents bind to SVEP1 and each of the remaining capture reagents binds to a different protein selected from HCC-1, RNAS6, PAP1, ATL2, N-terminal pro-BNP, RSPO4, BNP, MIC-1, FABPA, ILRL1, ANGP2, HE4, TAGL, RNAS1, and TSP2. In some embodiments, a kit is provided, the kit comprising N biomarker protein capture reagents, wherein N is at least 2, and wherein at least one of the capture reagents binds to RET or CRDL1, and at least one of the capture reagents binds to Tetranectin, N-terminal pro-BNP, TNNT2, CA125, MIC-1, SLPI, HE4, MMP-12, HSPB6, WISP-2, GHR, or IGFBP-2. In some embodiments, each capture reagent binds to a different biomarker protein. In some embodiments, N is 2, N is 3, N is 4, N is 5, N is 6, N is 7, N is 8, N is 9, N is 10, N is 11, N is 12, N is 13, N is 14, N is 15, N is 16, or N is 17. In some embodiments, N is 2, N is 3, or N is 4, or N is 5, or N is 6, or N is 7, or N is 8, or N is 9, or N is 10, or N is 11, or Nis 12, orNis 13, orNis 14.

In some embodiments, each of the N biomarker protein capture reagents specifically binds to a biomarker protein selected from Table 1. In some embodiments, each of the N biomarker protein capture reagents specifically binds to a biomarker protein selected form Table 2. In some embodiments, each of the N biomarker capture reagents is an antibody or an aptamer. In some embodiments, each biomarker capture reagent is an aptamer. In some embodiments, at least one aptamer is a slow off-rate aptamer. In some embodiments, at least one slow off-rate aptamer comprises at least one, at least two, at least three, at least four, at least five, at least six, at least seven, at least eight, at least nine, or at least 10 nucleotides with modifications. In some embodiments,, wherein each slow off-rate aptamer binds to its target protein with an off rate ($t^{1}/_{2}$) of $\geq 30$ minutes, $\geq 60$ minutes, $\geq 90$ minutes, $\geq 120$ minutes, $\geq 150$ minutes, $\geq 180$ minutes, $\geq 210$ minutes, or $\geq 240$ minutes. In some embodiments, the kit is for use in detecting the N biomarker proteins in a sample from a subject. In some embodiments, the kit is for use in determining the subject's risk or likelihood of experiencing a CV event within 1 year from the date that the sample was taken from the subject, wherein the subject has heart failure. In some embodiments, the CV event is death. In some embodiments, the subject has heart failure with reduced ejection fraction. In some embodiments, the subject has heart failure with preserved ejection fraction.

## BRIEF DESCRIPTION OF THE DRAWINGS

Fig. 1 shows the observed Kaplan-Meier survival probability of the training dataset of the HFrEF model, with individuals split into quartiles by predicted event probability at 365 days. The 1st to 4th quartiles are described with top line (Quartile 1), second line down (Quartile 2), third line down (Quartile 3) and bottom line (Quartile 4).

Figs. 2A-2B show Kaplan-Meier survival curves for each quartile for the validation dataset for the HFrEF model. Shaded regions in Fig. 2B represent 95% confidence intervals of the Kaplan-Meier estimates.

Fig. 3 shows the observed Kaplan-Meier survival probability of the training dataset for the HFpEF model, with individuals split into quartiles by predicted event probability at 365 days. The 1st to 4th quartiles are described with top line (Quartile 1), second line down (Quartile 2), third line down (Quartile 3) and bottom line (Quartile 4). Shaded regions represent 95% confidence intervals of the Kaplan-Meier estimates.

Fig. 4 shows Kaplan-Meier survival curves for each quartile of the validation dataset for the HFpEF model. Shaded regions represent 95% confidence intervals of the Kaplan-Meier estimates.

Fig. 5 illustrates a nonlimiting exemplary computer system for use with various computer-implemented methods described herein.

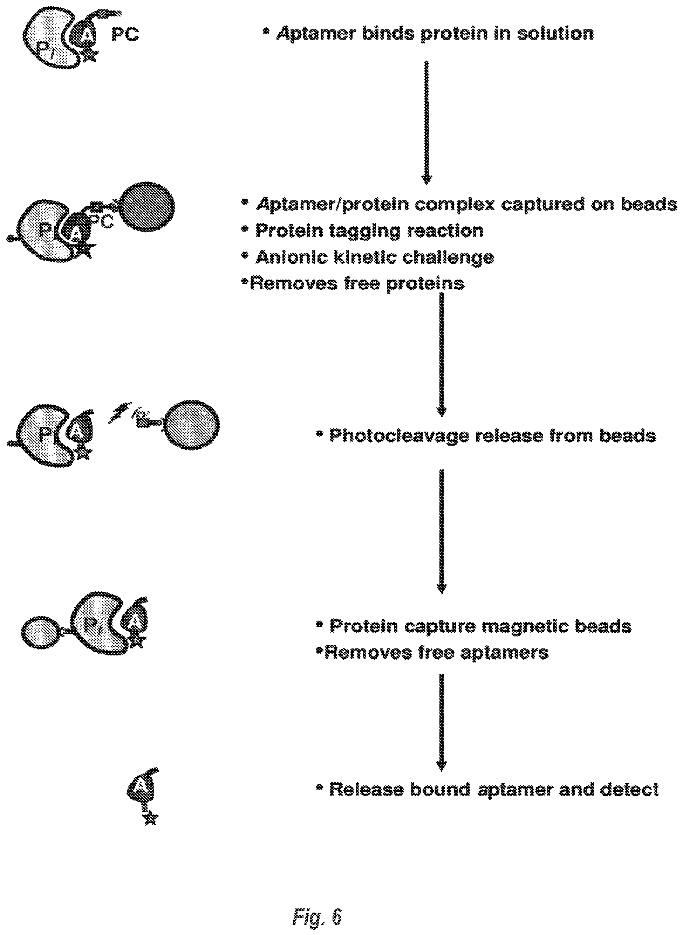

Fig. 6 illustrates a nonlimiting exemplary aptamer assay that can be used to detect one or more biomarkers in a biological sample.

Figs. 7-9 show certain exemplary modified pyrimidines that may be incorporated into aptamers, such as slow off-rate aptamers.

## DETAILED DESCRIPTION

While the invention will be described in conjunction with certain representative embodiments, it will be understood that the invention is defined by the claims, and is not limited to those embodiments.

One skilled in the art will recognize many methods and materials similar or equivalent to those described herein may be used in the practice of the present invention. The present invention is in no way limited to the methods and materials described.

Unless defined otherwise, technical and scientific terms used herein have the meaning commonly understood by one of ordinary skill in the art to which this invention belongs. Although any methods, devices, and materials similar or equivalent to those described herein can be used in the practice of the invention, certain methods, devices, and materials are described herein.

All publications, published patent documents, and patent applications cited herein are hereby incorporated by reference to the same extent as though each individual publication, published patent document, or patent application was specifically and individually indicated as being incorporated by reference.

As used herein, the terms "comprises," "comprising," "includes," "including," "contains," "containing," and any variations thereof, are intended to cover a non-exclusive inclusion, such that a process, method, product-by-process, or composition of matter that comprises, includes, or contains an element or list of elements may include other elements not expressly listed.

The present application includes biomarkers, methods, devices, reagents, systems, and kits for the prediction of risk of near-term CV events within a defined period of time, such as within 90 days, 180 days, or 1 year.

As used herein, a "cardiovascular event" or "CV event" broadly encompasses stroke, a transient ischemic attack (TIA), a myocardial infarction (MI), death, and/or hospitalization for heart failure, in a subject with chronic heart failure. In some embodiments, a "cardiovascular event" is hospitalization for heart failure, or death. In some embodiments, a "cardiovascular event" is hospitalization for heart failure. In some embodiments, a "cardiovascular event" is death.

As used herein, the term "heart failure" or "HF" refers to a complex clinical syndrome that results from any structural or functional impairment of ventricular filling or ejection of blood. The typical manifestations of HF are dyspnea and fatigue, which may limit exercise tolerance and fluid retention, and which may lead to pulmonary and/or splanchnic congestion and/or peripheral edema. The clinical syndrome of HF may result from disorders of the pericardium, myocardium, endocardium, heart valves, or great vessels or from certain metabolic abnormalities. Many patients with HF have symptoms due to impaired left ventricular (LV) myocardial function. (Yancy et al., 2013 ACCF/AHA guideline for the management of heart failure: A report of the American college of cardiology foundation/American heart association task force on practice guidelines. J Amer College Cardiol, 62(16), e147-e239. (2013).) As used herein, the term "chronic heart failure" or "CHF" refers to a stable chronic heart failure, unless indicated otherwise..

As used herein, the term "ejection fraction" or "EF" refers to the fraction (percentage) of outbound blood pumped from the heart with each contraction. It is commonly measured by echocardiogram and serves as a general measure of a subject's cardiac function. It may be measured as the amount of blood being pumped out of the left ventricle of the heart with each contraction. It also may be measured as the amount of blood being pumped out of the right ventricle of the heart to the lungs. As used herein, the term "ejection fraction" or "EF" refers to left ventricular ejection fraction, unless indicated otherwise. Patients with an ejection fraction of 50 percent or higher are classified as having "heart failure with preserved ejection fraction" (HFpEF), and patients with an ejection fraction of and patients with an ejection fraction lower than 40 percent are classified as having "heart failure with reduced ejection fraction" (HFrEF). (Ponikowski P, Voors A, Anker S, et al. 2016 ESC Guidelines for the diagnosis and treatment of acute and chronic heart failure. Eur J Heart Fail. 2016; 37: 2129-200.)

In some embodiments, biomarkers are provided for use either alone or in various combinations to evaluate the risk or likelihood of a future CV event within a 1-year time period with CV events defined as hospitalization for heart failure or death. As described in detail below, exemplary embodiments include the biomarkers provided in Table 1 or in Table 2.

While certain of the described CV event biomarkers may be useful alone for evaluating the risk or likelihood of a CV event, methods are also described herein for the grouping of multiple subsets of the CV event biomarkers, where each grouping or subset selection is useful as a panel of two or more biomarkers, interchangeably referred to herein as a "biomarker panel" and a panel. In some embodiments, the CV event is death. Thus, various embodiments provide combinations comprising at least two, at least three, at least four, at least five, at least six, at least seven, at least eight, at least nine, at least ten, at least eleven, at least twelve, at least thirteen, at least fourteen, at least fifteen, or all sixteen of the biomarkers in Table 1. Other various embodiments provide combinations comprising at least two, at least three, at least four, at least five, at least six, at least seven, at least eight, at least nine, at least ten, at least eleven, at least twelve, at least thirteen, or all fourteen of the biomarkers in Table 2.

"Biological sample", "sample", and "test sample" are used interchangeably herein to refer to any material, biological fluid, tissue, or cell obtained or otherwise derived from an individual. This includes blood (including whole blood, leukocytes, peripheral blood mononuclear cells, buffy coat, plasma, and serum), sputum, tears, mucus, nasal washes, nasal aspirate, urine, saliva, peritoneal washings, ascites, cystic fluid, glandular fluid, lymph fluid, bronchial aspirate, synovial fluid, joint aspirate, organ secretions, cells, a cellular extract, and cerebrospinal fluid. This also includes experimentally separated fractions of all of the preceding. For example, a blood sample can be fractionated into serum, plasma, or into fractions containing particular types of blood cells, such as red blood cells or white blood cells (leukocytes). In some embodiments, a blood sample is a dried blood spot. In some embodiments, a plasma sample is a dried plasma spot. In some embodiments, a sample can be a combination of samples from an individual, such as a combination of a tissue and fluid sample. The term "biological sample" also includes materials containing homogenized solid material, such as from a stool sample, a tissue sample, or a tissue biopsy, for example. The term "biological sample" also includes materials derived from a tissue culture or a cell culture. Any suitable methods for obtaining a biological sample can be employed; exemplary methods include, e.g., phlebotomy, swab (e.g., buccal swab), and a fine needle aspirate biopsy procedure. Exemplary tissues susceptible to fine needle aspiration include lymph node, lung, thyroid, breast, pancreas, and liver. Samples can also be collected, e.g., by micro dissection (e.g., laser capture micro dissection (LCM) or laser micro dissection (LMD)), bladder wash, smear (e.g., a PAP smear), or ductal lavage. A "biological sample" obtained or derived from an individual includes any such sample that has been processed in any suitable manner after being obtained from the individual. In some embodiments, a biological sample is a plasma sample.

Further, in some embodiments, a biological sample may be derived by taking biological samples from a number of individuals and pooling them, or pooling an aliquot of each individual's biological sample. The pooled sample may be treated as described herein for a sample from a single individual, and, for example, if a poor prognosis is established in the pooled sample, then each individual biological sample can be re-tested to determine which individual(s) have an increased or decreased risk of a CV event, such as death.

For purposes of this specification, the phrase "data attributed to a biological sample from an individual" is intended to mean that the data in some form derived from, or were generated using, the biological sample of the individual. The data may have been reformatted, revised, or mathematically altered to some degree after having been generated, such as by conversion from units in one measurement system to units in another measurement system; but, the data are understood to have been derived from, or were generated using, the biological sample.

"Target", "target molecule", and "analyte" are used interchangeably herein to refer to any molecule of interest that may be present in a biological sample. A "molecule of interest" includes any minor variation of a particular molecule, such as, in the case of a protein, for example, minor variations in amino acid sequence, disulfide bond formation, glycosylation, lipidation, acetylation, phosphorylation, or any other manipulation or modification, such as conjugation with a labeling component, which does not substantially alter the identity of the molecule. A "target molecule", "target", or "analyte" refers to a set of copies of one type or species of molecule or multi-molecular structure. Exemplary target molecules include proteins, polypeptides, nucleic acids, carbohydrates, lipids, polysaccharides, glycoproteins, hormones, receptors, antigens, antibodies, affybodies, antibody mimics, viruses, pathogens, toxic substances, substrates, metabolites, transition state analogs, cofactors, inhibitors, drugs, dyes, nutrients, growth factors, cells, tissues, and any fragment or portion of any of the foregoing. In some embodiments, a target molecule is a protein, in which case the target molecule may be referred to as a "target protein."

As used herein, a "capture agent' or "capture reagent" refers to a molecule that is capable of binding specifically to a biomarker. A "target protein capture reagent" refers to a molecule that is capable of binding specifically to a target protein. Nonlimiting exemplary capture reagents include aptamers, antibodies, adnectins, ankyrins, other antibody mimetics and other protein scaffolds, autoantibodies, chimeras, small molecules, nucleic acids, lectins, ligand-binding receptors, imprinted polymers, avimers, peptidomimetics, hormone receptors, cytokine receptors, synthetic receptors, and modifications and fragments of any of the aforementioned capture reagents. In some embodiments, a capture reagent is selected from an aptamer and an antibody.

The term "antibody" refers to full-length antibodies of any species and fragments and derivatives of such antibodies, including Fab fragments, F(ab')2 fragments, single chain antibodies, Fv fragments, and single chain Fv fragments. The term "antibody" also refers to synthetically-derived antibodies, such as phage display-derived antibodies and fragments, affybodies, nanobodies, etc.

As used herein, "marker" and "biomarker" are used interchangeably to refer to a target molecule that indicates or is a sign of a normal or abnormal process in an individual or of a disease or other condition in an individual. More specifically, a "marker" or "biomarker" is an anatomic, physiologic, biochemical, or molecular parameter associated with the presence of a specific physiological state or process, whether normal or abnormal, and, if abnormal, whether chronic or acute. Biomarkers are detectable and measurable by a variety of methods including laboratory assays and medical imaging. In some embodiments, a biomarker is a target protein.

As used herein, "biomarker level" and "level" refer to a measurement that is made using any analytical method for detecting the biomarker in a biological sample and that indicates the presence, absence, absolute amount or concentration, relative amount or concentration, titer, a level, an expression level, a ratio of measured levels, or the like, of, for, or corresponding to the biomarker in the biological sample. The exact nature of the "level" depends on the specific design and components of the particular analytical method employed to detect the biomarker.

When a biomarker indicates or is a sign of an abnormal process or a disease or other condition in an individual, that biomarker is generally described as being either over-expressed or under-expressed as compared to an expression level or value of the biomarker that indicates or is a sign of a normal process or an absence of a disease or other condition in an individual. "Up-regulation", "up-regulated", "over-expression", "over-expressed", and any variations thereof are used interchangeably to refer to a value or level of a biomarker in a biological sample that is greater than a value or level (or range of values or levels) of the biomarker that is typically detected in similar biological samples from healthy or normal individuals. The terms may also refer to a value or level of a biomarker in a biological sample that is greater than a value or level (or range of values or levels) of the biomarker that may be detected at a different stage of a particular disease.

"Down-regulation", "down-regulated", "under-expression", "under-expressed", and any variations thereof are used interchangeably to refer to a value or level of a biomarker in a biological sample that is less than a value or level (or range of values or levels) of the biomarker that is typically detected in similar biological samples from healthy or normal individuals. The terms may also refer to a value or level of a biomarker in a biological sample that is less than a value or level (or range of values or levels) of the biomarker that may be detected at a different stage of a particular disease.

Further, a biomarker that is either over-expressed or under-expressed can also be referred to as being "differentially expressed" or as having a "differential level" or "differential value" as compared to a "normal" expression level or value of the biomarker that indicates or is a sign of a normal process or an absence of a disease or other condition in an individual. Thus, "differential expression" of a biomarker can also be referred to as a variation from a "normal" expression level of the biomarker.

A "control level" of a target molecule refers to the level of the target molecule in the same sample type from an individual that does not have the disease or condition, or from an individual that is not suspected or at risk of having the disease or condition, or from an individual that has had a primary or first cardiovascular event but not a secondary cardiovascular event, or from an individual that has stable cardiovascular disease. Control level may refer to the average level of the target molecule in samples from a population of individuals that does not have the disease or condition, or that is not suspected or at risk of having the disease or condition, or that has had a primary or first cardiovascular event but not a secondary cardiovascular event, or that has stable cardiovascular disease or a combination thereof.

As used herein, "individual," "subject," and "patient" are used interchangeably to refer to a mammal. A mammalian individual can be a human or non-human. In various embodiments, the individual is a human. A healthy or normal individual is an individual in which the disease or condition of interest (including, for example, chronic heart failure and cardiovascular events such as myocardial infarction, stroke and hospitalization for heart failure) is not detectable by conventional diagnostic methods.

"Diagnose", "diagnosing", "diagnosis", and variations thereof refer to the detection, determination, or recognition of a health status or condition of an individual on the basis of one or more signs, symptoms, data, or other information pertaining to that individual. The health status of an individual can be diagnosed as healthy / normal (i.e., a diagnosis of the absence of a disease or condition) or diagnosed as ill / abnormal (i.e., a diagnosis of the presence, or an assessment of the characteristics, of a disease or condition). The terms "diagnose", "diagnosing", "diagnosis", etc., encompass, with respect to a particular disease or condition, the initial detection of the disease; the characterization or classification of the disease; the detection of the progression, remission, or recurrence of the disease; and the detection of disease response after the administration of a treatment or therapy to the individual. The prediction of risk of a CV event includes distinguishing individuals who have an increased risk of a CV event from individuals who do not.

"Pro nose" "prognosing", "prognosis", and variations thereof refer to the prediction of a future course of a disease or condition in an individual who has the disease or condition (e.g., predicting patient survival), and such terms encompass the evaluation of disease or condition response after the administration of a treatment or therapy to the individual.

"Evaluate", "evaluating", "evaluation", and variations thereof encompass both "diagnose" and "prognose" and also encompass determinations or predictions about the future course of a disease or condition in an individual who does not have the disease as well as determinations or predictions regarding the risk that a disease or condition will recur in an individual who apparently has been cured of the disease or has had the condition resolved. The term "evaluate" also encompasses assessing an individual's response to a therapy, such as, for example, predicting whether an individual is likely to respond favorably to a therapeutic agent or is unlikely to respond to a therapeutic agent (or will experience toxic or other undesirable side effects, for example), selecting a therapeutic agent for administration to an individual, or monitoring or determining an individual's response to a therapy that has been administered to the individual. Thus, "evaluating" risk of a CV event can include, for example, any of the following: predicting the future risk of a CV event in an individual; predicting the risk of a CV event in an individual who apparently has no CV issues; predicting a particular type of CV event; predicting the time to a CV event; or determining or predicting an individual's response to a CV treatment or selecting a CV treatment to administer to an individual based upon a determination of the biomarker values derived from the individual's biological sample. Evaluation of risk of a CV event can include embodiments such as the assessment of risk of a CV event on a continuous scale, or classification of risk of a CV event in escalating classifications. Classification of risk includes, for example, classification into two or more classifications such as "No Elevated Risk of a CV Event;" "Elevated Risk of a CV Event;" and/or "Below Average Risk of CV Event." In some embodiments, the evaluation of risk of a CV event is for a defined period. Nonlimiting exemplary defined periods include 90 days, 180 days, and 1 year. In various embodiments, the CV event is death.

As used herein, "additional biomedical information" refers to one or more evaluations of an individual, other than using any of the biomarkers described herein, that are associated with CV risk or, more specifically, CV event risk. "Additional biomedical information" includes any of the following: physical descriptors of an individual, including the height and/or weight of an individual; the age of an individual; the gender of an individual; change in weight; the ethnicity of an individual; occupational history; family history of cardiovascular disease (or other circulatory system disorders); the presence of a genetic marker(s) correlating with a higher risk of cardiovascular disease (or other circulatory system disorders) in the individual or a family member alterations in the carotid intima thickness; clinical symptoms such as chest pain, weight gain or loss gene expression values; physical descriptors of an individual, including physical descriptors observed by radiologic imaging; smoking status; alcohol use history; occupational history; dietary habits - salt, saturated fat and cholesterol intake; caffeine consumption; and imaging information such as electrocardiogram, echocardiography, carotid ultrasound for intima-media thickness, flow mediated dilation, pulse wave velocity, ankle -brachial index, stress echocardiography, myocardial perfusion imaging, coronary calcium by CT, high resolution CT angiography, Mill imaging, and other imaging modalities; and the individual's medications. Testing of biomarker levels in combination with an evaluation of any additional biomedical information, including other laboratory tests (e.g., HDL, LDL testing, CRP levels, Nt-proBNP testing, BNP testing, high sensitivity troponin testing, galectin-3 testing, serum albumin testing, creatine testing), may, for example, improve sensitivity, specificity, and/or AUC for prediction of CV events as compared to biomarker testing alone or evaluating any particular item of additional biomedical information alone (e.g., carotid intima thickness imaging alone). Additional biomedical information can be obtained from an individual using routine techniques known in the art, such as from the individual themselves by use of a routine patient questionnaire or health history questionnaire, etc., or from a medical practitioner, etc. Testing of biomarker levels in combination with an evaluation of any additional biomedical information may, for example, improve sensitivity, specificity, and/or thresholds for prediction of CV events (or other cardiovascular-related uses) as compared to biomarker testing alone or evaluating any particular item of additional biomedical information alone (e.g., CT imaging alone).

As used herein, "detecting" or "determining" with respect to a biomarker value includes the use of both the instrument used to observe and record a signal corresponding to a biomarker level and the material/s required to generate that signal. In various embodiments, the biomarker level is detected using any suitable method, including fluorescence, chemiluminescence, surface plasmon resonance, surface acoustic waves, mass spectrometry, infrared spectroscopy, Raman spectroscopy, atomic force microscopy, scanning tunneling microscopy, electrochemical detection methods, nuclear magnetic resonance, quantum dots, and the like.

"Solid support" refers herein to any substrate having a surface to which molecules may be attached, directly or indirectly, through either covalent or non-covalent bonds. A "solid support" can have a variety of physical formats, which can include, for example, a membrane; a chip (e.g., a protein chip); a slide (e.g., a glass slide or coverslip); a column; a hollow, solid, semi-solid, pore- or cavity- containing particle, such as, for example, a bead; a gel; a fiber, including a fiber optic material; a matrix; and a sample receptacle. Exemplary sample receptacles include sample wells, tubes, capillaries, vials, and any other vessel, groove or indentation capable of holding a sample. A sample receptacle can be contained on a multi-sample platform, such as a microtiter plate, slide, microfluidics device, and the like. A support can be composed of a natural or synthetic material, an organic or inorganic material. The composition of the solid support on which capture reagents are attached generally depends on the method of attachment (e.g., covalent attachment). Other exemplary receptacles include microdroplets and microfluidic controlled or bulk oil/aqueous emulsions within which assays and related manipulations can occur. Suitable solid supports include, for example, plastics, resins, polysaccharides, silica or silica-based materials, functionalized glass, modified silicon, carbon, metals, inorganic glasses, membranes, nylon, natural fibers (such as, for example, silk, wool and cotton), polymers, and the like. The material composing the solid support can include reactive groups such as, for example, carboxy, amino, or hydroxyl groups, which are used for attachment of the capture reagents. Polymeric solid supports can include, e.g., polystyrene, polyethylene glycol tetraphthalate, polyvinyl acetate, polyvinyl chloride, polyvinyl pyrrolidone, polyacrylonitrile, polymethyl methacrylate, polytetrafluoroethylene, butyl rubber, styrenebutadiene rubber, natural rubber, polyethylene, polypropylene, (poly)tetrafluoroethylene, (poly)vinylidenefluoride, polycarbonate, and polymethylpentene. Suitable solid support particles that can be used include, e.g., encoded particles, such as Luminex®-type encoded particles, magnetic particles, and glass particles.

Exemplary Uses of Biomarkers

In various exemplary embodiments, methods are provided for evaluating risk of a CV event in an individual by detecting one or more biomarker values corresponding to one or more biomarkers that are present in the circulation of an individual, such as in blood, serum or plasma, by any number of analytical methods, including any of the analytical methods described herein. These biomarkers are, for example, differentially expressed in individuals with increased risk of a CV event as compared to individuals without increased risk of a CV event. Detection of the differential expression of a biomarker in an individual can be used, for example, to permit the prediction of risk of a CV event within a 90 day, 180 day, or a 1 year time frame. In various embodiments, the CV event is hospitalization for heart failure or death. In various embodiments, the CV event is death.

In addition to testing biomarker levels as a stand-alone diagnostic test, biomarker levels can also be done in conjunction with determination of single nucleotide polymorphisms (SNPs) or other genetic lesions or variability that are indicative of increased risk of susceptibility of disease or condition. (See, e.g., Amos et al., Nature Genetics 40, 616622 (2009)).

Biomarker levels can also be used in conjunction with radiologic screening. Biomarker levels can also be used in conjunction with relevant symptoms or genetic testing. Detection of any of the biomarkers described herein may be useful after the risk of CV event has been evaluated to guide appropriate clinical care of the individual, including increasing to more aggressive levels of care in high risk individuals after the CV event risk has been determined. In addition to testing biomarker levels in conjunction with relevant symptoms or risk factors, information regarding the biomarkers can also be evaluated in conjunction with other types of data, particularly data that indicates an individual's risk for cardiovascular events (e.g., patient clinical history, symptoms, family history of cardiovascular disease, history of smoking or alcohol use, risk factors such as the presence of a genetic marker(s), and/or status of other biomarkers, etc.). These various data can be assessed by automated methods, such as a computer program/software, which can be embodied in a computer or other apparatus/device.

Testing of biomarkers can also be associated with guidelines and cardiovascular risk algorithms currently in use in clinical practice. For example, the Framingham Risk Score uses risk factors to provide a risk score, such risk factors including LDL-cholesterol and HDL-cholesterol levels, impaired glucose levels, smoking, systolic blood pressure, and diabetes. The frequency of high-risk patients increases with age, and men comprise a greater proportion of high-risk patients than women.

Any of the described biomarkers may also be used in imaging tests. For example, an imaging agent can be coupled to any of the described biomarkers, which can be used to aid in prediction of risk of a cardiovascular event, to monitor response to therapeutic interventions, to select for target populations in a clinical trial among other uses.

**Detection and Determination of Biomarkers and Biomarker Levels**

A biomarker level for the biomarkers described herein can be detected using any of a variety of known analytical methods. In one embodiment, a biomarker value is detected using a capture reagent. In various embodiments, the capture reagent can be exposed to the biomarker in solution or can be exposed to the biomarker while the capture reagent is immobilized on a solid support. In other embodiments, the capture reagent contains a feature that is reactive with a secondary feature on a solid support. In these embodiments, the capture reagent can be exposed to the biomarker in solution, and then the feature on the capture reagent can be used in conjunction with the secondary feature on the solid support to immobilize the biomarker on the solid support. The capture reagent is selected based on the type of analysis to be conducted. Capture reagents include but are not limited to aptamers, antibodies, adnectins, ankyrins, other antibody mimetics and other protein scaffolds, autoantibodies, chimeras, small molecules, F(ab')2 fragments, single chain antibody fragments, Fv fragments, single chain Fv fragments, nucleic acids, lectins, ligand-binding receptors, affybodies, nanobodies, imprinted polymers, avimers, peptidomimetics, hormone receptors, cytokine receptors, and synthetic receptors, and modifications and fragments of these.

In some embodiments, a biomarker level is detected using a biomarker/capture reagent complex.

In some embodiments, the biomarker level is derived from the biomarker/capture reagent complex and is detected indirectly, such as, for example, as a result of a reaction that is subsequent to the biomarker/capture reagent interaction, but is dependent on the formation of the biomarker/capture reagent complex.

In some embodiments, the biomarker level is detected directly from the biomarker in a biological sample.

In some embodiments, biomarkers are detected using a multiplexed format that allows for the simultaneous detection of two or more biomarkers in a biological sample. In some embodiments of the multiplexed format, capture reagents are immobilized, directly or indirectly, covalently or non-covalently, in discrete locations on a solid support. In some embodiments, a multiplexed format uses discrete solid supports where each solid support has a unique capture reagent associated with that solid support, such as, for example quantum dots. In some embodiments, an individual device is used for the detection of each one of multiple biomarkers to be detected in a biological sample. Individual devices can be configured to permit each biomarker in the biological sample to be processed simultaneously. For example, a microtiter plate can be used such that each well in the plate is used to uniquely analyze one or more biomarkers to be detected in a biological sample.

In one or more of the foregoing embodiments, a fluorescent tag can be used to label a component of the biomarker/capture reagent complex to enable the detection of the biomarker level. In various embodiments, the fluorescent label can be conjugated to a capture reagent specific to any of the biomarkers described herein using known techniques, and the fluorescent label can then be used to detect the corresponding biomarker level. Suitable fluorescent labels include rare earth chelates, fluorescein and its derivatives, rhodamine and its derivatives, dansyl, allophycocyanin, PBXL-3, Qdot 605, Lissamine, phycoerythrin, Texas Red, and other such compounds.

In some embodiments, the fluorescent label is a fluorescent dye molecule. In some embodiments, the fluorescent dye molecule includes at least one substituted indolium ring system in which the substituent on the 3-carbon of the indolium ring contains a chemically reactive group or a conjugated substance. In some embodiments, the dye molecule includes an AlexFluor molecule, such as, for example, AlexaFluor 488, AlexaFluor 532, AlexaFluor 647, AlexaFluor 680, or AlexaFluor 700. In other embodiments, the dye molecule includes a first type and a second type of dye molecule, such as, e.g., two different AlexaFluor molecules. In some embodiments, the dye molecule includes a first type and a second type of dye molecule, and the two dye molecules have different emission spectra.

Fluorescence can be measured with a variety of instrumentation compatible with a wide range of assay formats. For example, spectrofluorimeters have been designed to analyze microtiter plates, microscope slides, printed arrays, cuvettes, etc. See Principles of Fluorescence Spectroscopy, by J.R. Lakowicz, Springer Science + Business Media, Inc., 2004. See Bioluminescence & Chemiluminescence: Progress & Current Applications; Philip E. Stanley and Larry J. Kricka editors, World Scientific Publishing Company, January 2002.

In one or more embodiments, a chemiluminescence tag can optionally be used to label a component of the biomarker/capture complex to enable the detection of a biomarker level. Suitable chemiluminescent materials include any of oxalyl chloride, Rodamin 6G, Ru(bipy)3$^{2+}$, TMAE (tetrakis(dimethylamino)ethylene), Pyrogallol (1,2,3-trihydroxibenzene), Lucigenin, peroxyoxalates, Aryl oxalates, Acridinium esters, dioxetanes, and others.

In some embodiments, the detection method includes an enzyme/substrate combination that generates a detectable signal that corresponds to the biomarker level. Generally, the enzyme catalyzes a chemical alteration of the chromogenic substrate which can be measured using various techniques, including spectrophotometry, fluorescence, and chemiluminescence. Suitable enzymes include, for example, luciferases, luciferin, malate dehydrogenase, urease, horseradish peroxidase (HRPO), alkaline phosphatase, beta-galactosidase, glucoamylase, lysozyme, glucose oxidase, galactose oxidase, and glucose-6-phosphate dehydrogenase, uricase, xanthine oxidase, lactoperoxidase, microperoxidase, and the like.

In some embodiments, the detection method can be a combination of fluorescence, chemiluminescence, radionuclide or enzyme/substrate combinations that generate a measurable signal. In some embodiments, multimodal signaling could have unique and advantageous characteristics in biomarker assay formats.

In some embodiments, the biomarker levels for the biomarkers described herein can be detected using any analytical methods including, singleplex aptamer assays, multiplexed aptamer assays, singleplex or multiplexed immunoassays, mRNA expression profiling, miRNA expression profiling, mass spectrometric analysis, histological/cytological methods, etc. as discussed below.

Determination of Biomarker Levels using Aptamer-Based Assays

Assays directed to the detection and quantification of physiologically significant molecules in biological samples and other samples are important tools in scientific research and in the health care field. One class of such assays involves the use of a microarray that includes one or more aptamers immobilized on a solid support. The aptamers are each capable of binding to a target molecule in a highly specific manner and with very high affinity. See, e.g., U.S. Patent No. 5,475,096 entitled "Nucleic Acid Ligands"; see also, e.g., U.S. Patent No. 6,242,246, U.S. Patent No. 6,458,543, and U.S. Patent No. 6,503,715, each of which is entitled "Nucleic Acid Ligand Diagnostic Biochip". Once the microarray is contacted with a sample, the aptamers bind to their respective target molecules present in the sample and thereby enable a determination of a biomarker level corresponding to a biomarker.

As used herein, an "aptamer" refers to a nucleic acid that has a specific binding affinity for a target molecule. It is recognized that affinity interactions are a matter of degree; however, in this context, the "specific binding affinity" of an aptamer for its target means that the aptamer binds to its target generally with a much higher degree of affinity than it binds to other components in a test sample. An "aptamer" is a set of copies of one type or species of nucleic acid molecule that comprises a particular nucleotide sequence. An aptamer can include any suitable number of nucleotides, including any number of chemically modified nucleotides. "Aptamers" refers to more than one such set of molecules. Different aptamers can have either the same or different numbers of nucleotides. Aptamers can be DNA or RNA or chemically modified nucleic acids and can be single-stranded, double-stranded, or contain double-stranded regions, and can include higher ordered structures. An aptamer can also be a photoaptamer, where a photoreactive or chemically reactive functional group is included in the aptamer to allow it to be covalently linked to its corresponding target. Any of the aptamer methods disclosed herein can include the use of two or more aptamers that specifically bind the same target molecule. As further described below, an aptamer may include a tag. If an aptamer includes a tag, all copies of the aptamer need not have the same tag. Moreover, if different aptamers each include a tag, these different aptamers can have either the same tag or a different tag.

An aptamer can be identified using any known method, including the SELEX process. Once identified, an aptamer can be prepared or synthesized in accordance with any known method, including chemical synthetic methods and enzymatic synthetic methods.

The terms "SELEX" and "SELEX process" are used interchangeably herein to refer generally to a combination of (1) the selection of aptamers that interact with a target molecule in a desirable manner, for example binding with high affinity to a protein, with (2) the amplification of those selected nucleic acids. The SELEX process can be used to identify aptamers with high affinity to a specific target or biomarker.

SELEX generally includes preparing a candidate mixture of nucleic acids, binding of the candidate mixture to the desired target molecule to form an affinity complex, separating the affinity complexes from the unbound candidate nucleic acids, separating and isolating the nucleic acid from the affinity complex, purifying the nucleic acid, and identifying a specific aptamer sequence. The process may include multiple rounds to further refine the affinity of the selected aptamer. The process can include amplification steps at one or more points in the process. See, e.g., U.S. Patent No. 5,475,096, entitled "Nucleic Acid Ligands". The SELEX process can be used to generate an aptamer that covalently binds its target as well as an aptamer that non-covalently binds its target. See, e.g., U.S. Patent No. 5,705,337 entitled "Systematic Evolution of Nucleic Acid Ligands by Exponential Enrichment: Chemi-SELEX."

The SELEX process can be used to identify high-affinity aptamers containing modified nucleotides that confer improved characteristics on the aptamer, such as, for example, improved *in vivo* stability or improved delivery characteristics. Examples of such modifications include chemical substitutions at the ribose and/or phosphate and/or base positions. SELEX process-identified aptamers containing modified nucleotides are described in U.S. Patent No. 5,660,985, entitled "High Affinity Nucleic Acid Ligands Containing Modified Nucleotides", which describes oligonucleotides containing nucleotide derivatives chemically modified at the 5'- and 2'-positions of pyrimidines. U.S. Patent No. 5,580,737, see supra, describes highly specific aptamers containing one or more nucleotides modified with 2'-amino (2'-NH2), 2'-fluoro (2'-F), and/or 2'-O-methyl (2'-OMe). See also, U.S. Patent Application Publication 20090098549, entitled "SELEX and PHOTOSELEX", which describes nucleic acid libraries having expanded physical and chemical properties and their use in SELEX and photoSELEX.

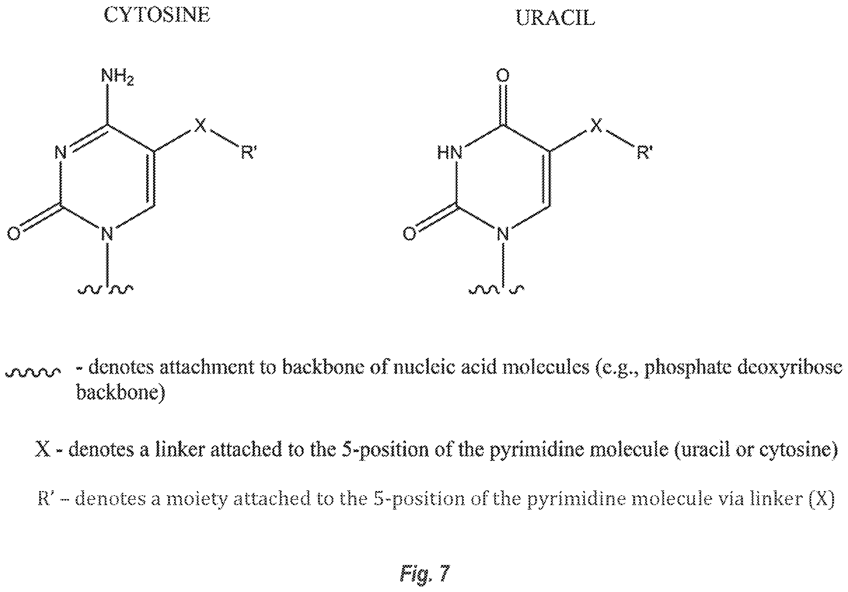

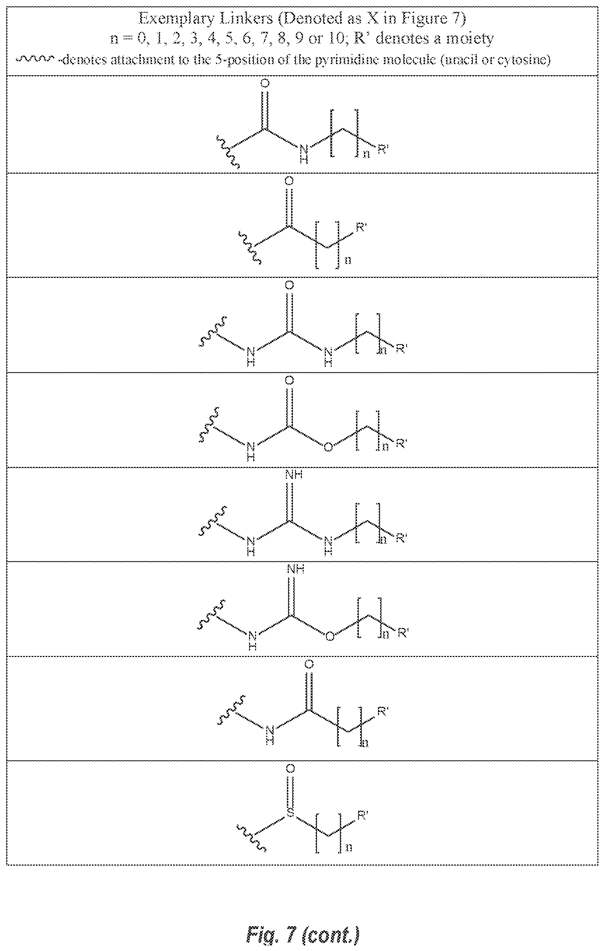

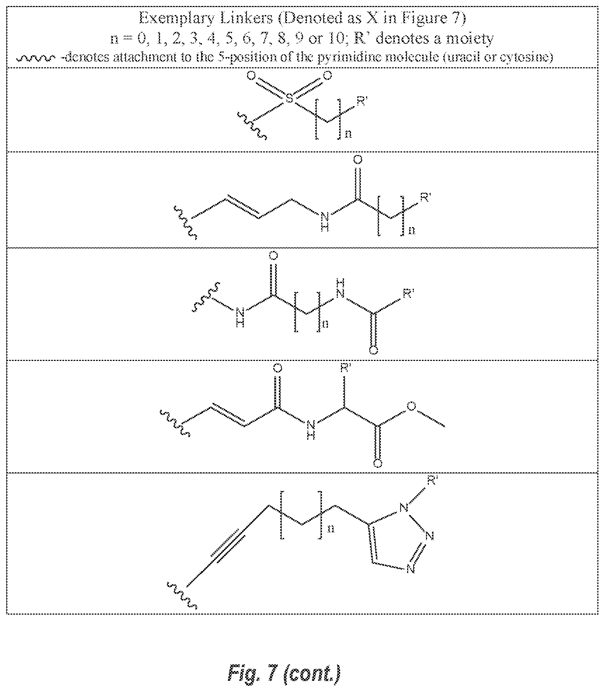

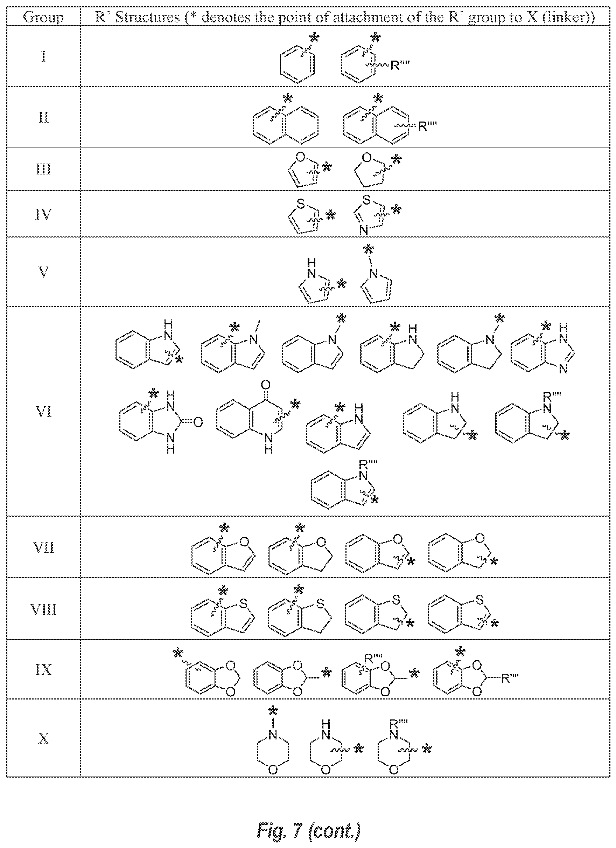

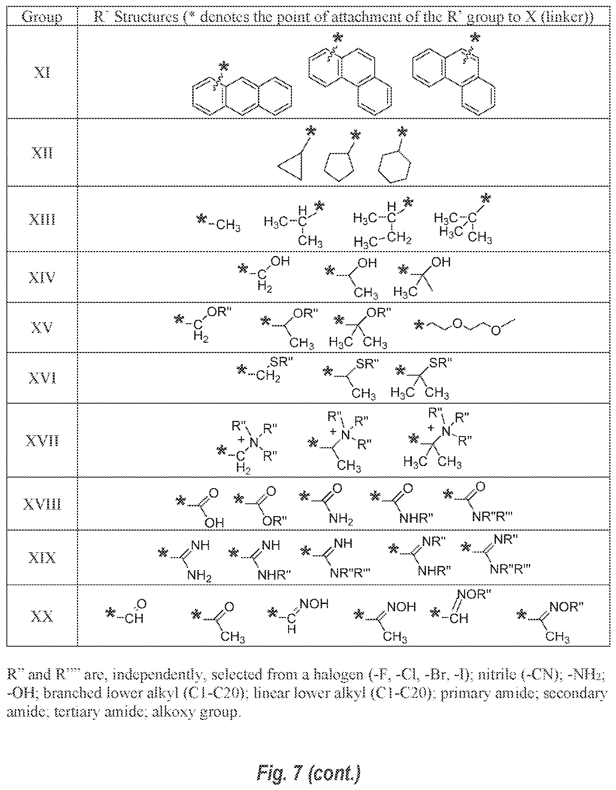

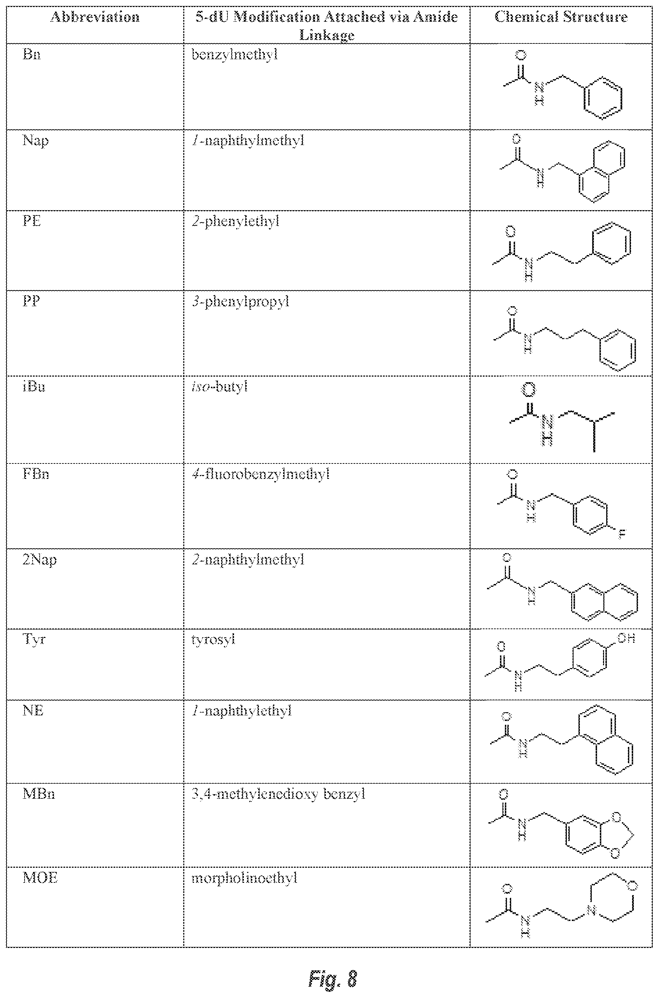

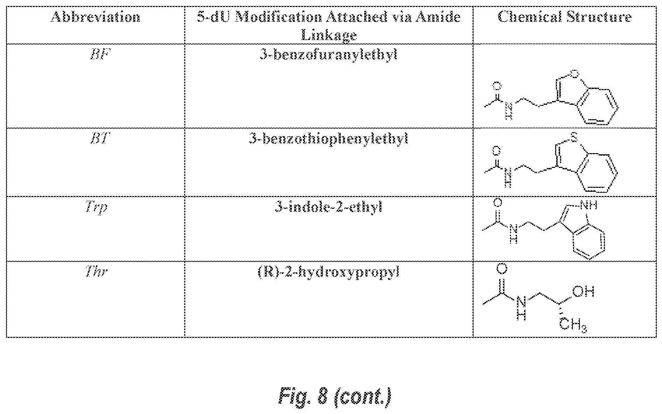

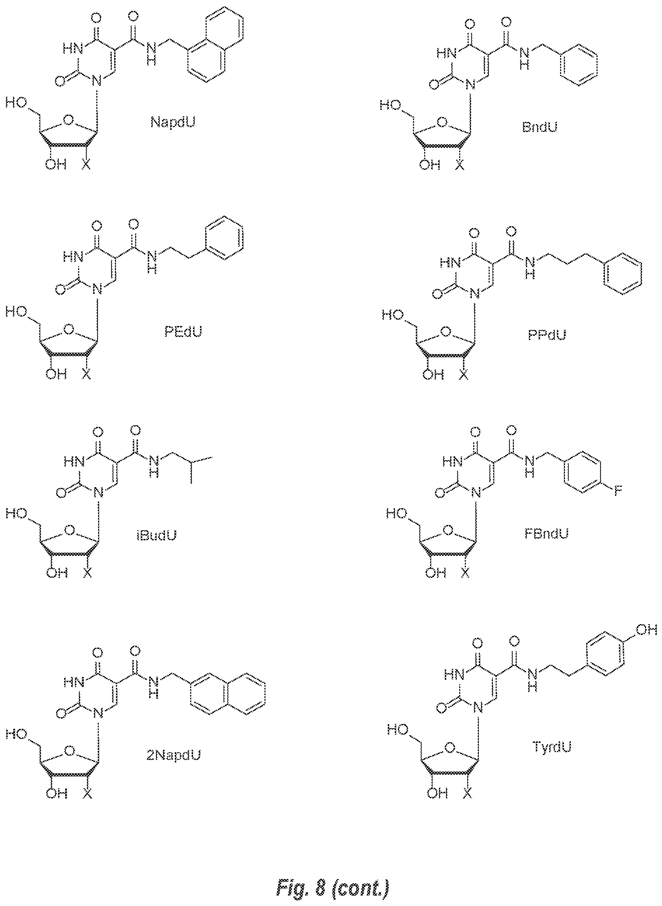

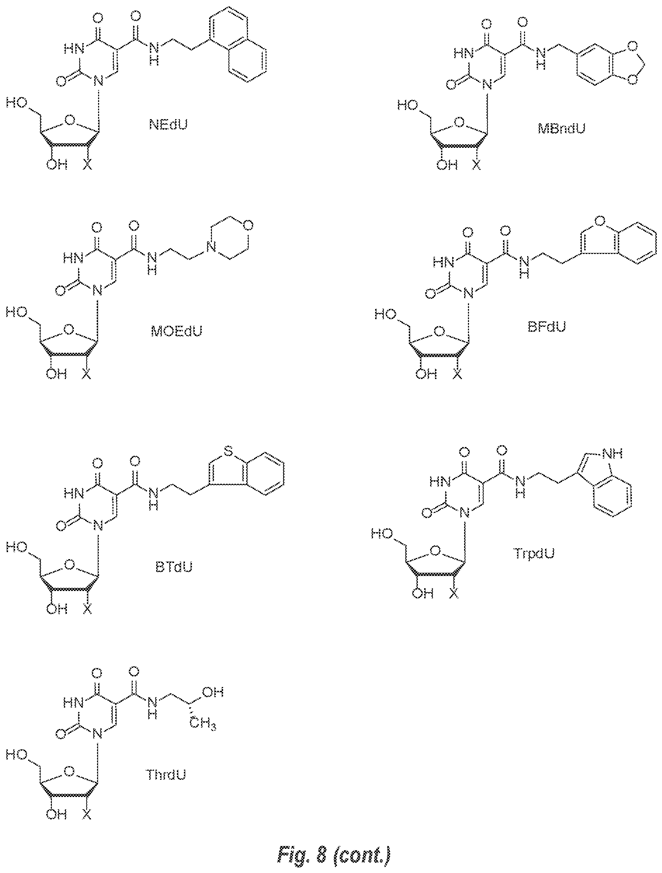

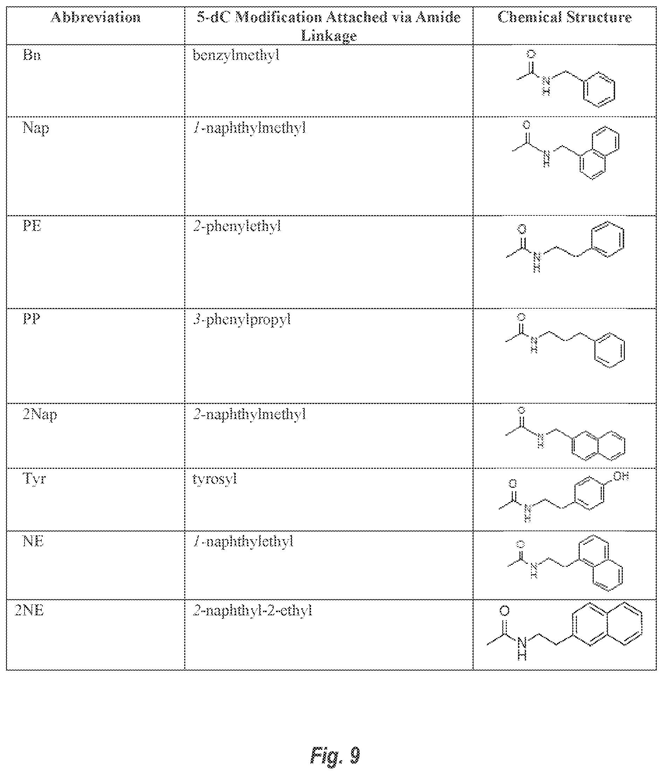

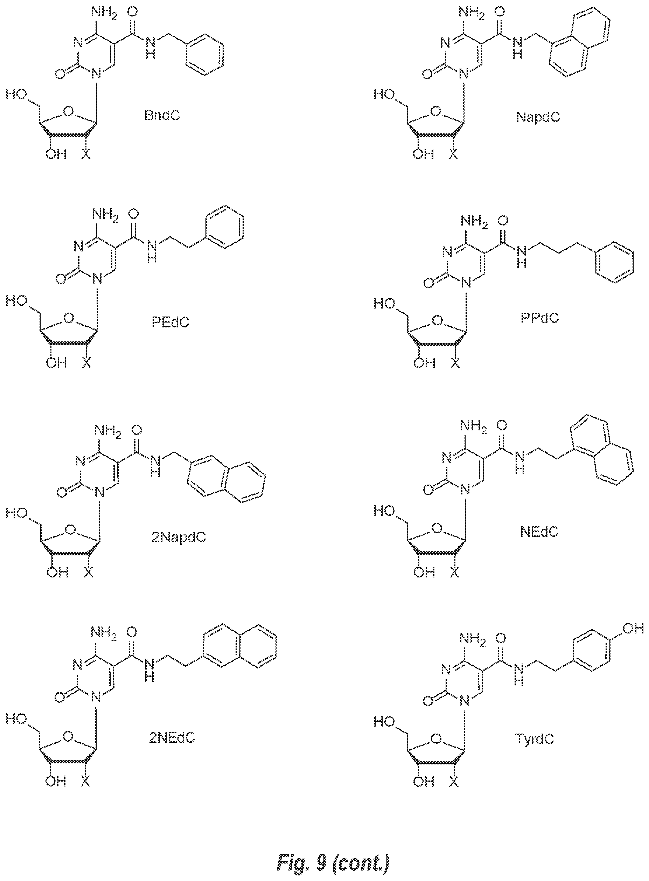

SELEX can also be used to identify aptamers that have desirable off-rate characteristics. See U.S. Publication No. 20090004667, entitled "Method for Generating Aptamers with Improved Off-Rates", which describes improved SELEX methods for generating aptamers that can bind to target molecules. Methods for producing aptamers and photoaptamers having slower rates of dissociation from their respective target molecules are described. The methods involve contacting the candidate mixture with the target molecule, allowing the formation of nucleic acid-target complexes to occur, and performing a slow off-rate enrichment process wherein nucleic acid-target complexes with fast dissociation rates will dissociate and not reform, while complexes with slow dissociation rates will remain intact. Additionally, the methods include the use of modified nucleotides in the production of candidate nucleic acid mixtures to generate aptamers with improved off-rate performance. Nonlimiting exemplary modified nucleotides include, for example, the modified pyrimidines shown in Figs. 7-9. In some embodiments, an aptamer comprises at least one nucleotide with a modification, such as a base modification. In some embodiments, an aptamer comprises at least one nucleotide with a hydrophobic modification, such as a hydrophobic base modification, allowing for hydrophobic contacts with a target protein. Such hydrophobic contacts, in some embodiments, contribute to greater affinity and/or slower off-rate binding by the aptamer. Nonlimiting exemplary nucleotides with hydrophobic modifications are shown in Figure 7. In some embodiments, an aptamer comprises at least two, at least three, at least four, at least five, at least six, at least seven, at least eight, at least nine, or at least 10 nucleotides with hydrophobic modifications, where each hydrophobic modification may be the same or different from the others. In some embodiments, at least one, at least two, at least three, at least four, at least five, at least six, at least seven, at least eight, at least nine, or at least 10 hydrophobic modifications in an aptamer may be independently selected from the hydrophobic modifications shown in Figure 7.

In some embodiments, the aptamer is a slow off-rate aptamer. In some embodiments, a slow off-rate aptamer (including an aptamers comprising at least one nucleotide with a hydrophobic modification) has an off-rate ($t^{1/2}$) of $\geq 30$ minutes, $\geq 60$ minutes, $\geq 90$ minutes, $\geq 120$ minutes, $\geq 150$ minutes, $\geq 180$ minutes, $\geq 210$ minutes, or $\geq 240$ minutes.

In some embodiments, an assay employs aptamers that include photoreactive functional groups that enable the aptamers to covalently bind or "photocrosslink" their target molecules. See, e.g., U.S. Patent No. 6,544,776 entitled

EP 4 614 156 A2

"Nucleic Acid Ligand Diagnostic Biochip". These photoreactive aptamers are also referred to as photoaptamers. See, e.g., U.S. Patent No. 5,763,177, U.S. Patent No. 6,001,577, and U.S. Patent No. 6,291,184, each of which is entitled "Systematic Evolution of Nucleic Acid Ligands by Exponential Enrichment: Photoselection of Nucleic Acid Ligands and Solution SELEX"; see also, e.g., U.S. Patent No. 6,458,539, entitled "Photoselection of Nucleic Acid Ligands". After the microarray is contacted with the sample and the photoaptamers have had an opportunity to bind to their target molecules, the photoaptamers are photoactivated, and the solid support is washed to remove any non-specifically bound molecules. Harsh wash conditions may be used, since target molecules that are bound to the photoaptamers are generally not removed, due to the covalent bonds created by the photoactivated functional group(s) on the photoaptamers. In this manner, the assay enables the detection of a biomarker level corresponding to a biomarker in the test sample.

In some assay formats, the aptamers are immobilized on the solid support prior to being contacted with the sample. Under certain circumstances, however, immobilization of the aptamers prior to contact with the sample may not provide an optimal assay. For example, pre-immobilization of the aptamers may result in inefficient mixing of the aptamers with the target molecules on the surface of the solid support, perhaps leading to lengthy reaction times and, therefore, extended incubation periods to permit efficient binding of the aptamers to their target molecules. Further, when photoaptamers are employed in the assay and depending upon the material utilized as a solid support, the solid support may tend to scatter or absorb the light used to effect the formation of covalent bonds between the photoaptamers and their target molecules. Moreover, depending upon the method employed, detection of target molecules bound to their aptamers can be subject to imprecision, since the surface of the solid support may also be exposed to and affected by any labeling agents that are used. Finally, immobilization of the aptamers on the solid support generally involves an aptamer-preparation step (i.e., the immobilization) prior to exposure of the aptamers to the sample, and this preparation step may affect the activity or functionality of the aptamers.

Aptamer assays that permit an aptamer to capture its target in solution and then employ separation steps that are designed to remove specific components of the aptamer-target mixture prior to detection have also been described (see U.S. Publication No. 20090042206, entitled "Multiplexed Analyses of Test Samples"). The described aptamer assay methods enable the detection and quantification of a non-nucleic acid target (e.g., a protein target) in a test sample by detecting and quantifying a nucleic acid (i.e., an aptamer). The described methods create a nucleic acid surrogate (i.e, the aptamer) for detecting and quantifying a non-nucleic acid target, thus allowing the wide variety of nucleic acid technologies, including amplification, to be applied to a broader range of desired targets, including protein targets.

Aptamers can be constructed to facilitate the separation of the assay components from an aptamer biomarker complex (or photoaptamer biomarker covalent complex) and permit isolation of the aptamer for detection and/or quantification. **In** one embodiment, these constructs can include a cleavable or releasable element within the aptamer sequence. In other embodiments, additional functionality can be introduced into the aptamer, for example, a labeled or detectable component, a spacer component, or a specific binding tag or immobilization element. For example, the aptamer can include a tag connected to the aptamer via a cleavable moiety, a label, a spacer component separating the label, and the cleavable moiety. In one embodiment, a cleavable element is a photocleavable linker. The photocleavable linker can be attached to a biotin moiety and a spacer section, can include an NHS group for derivatization of amines, and can be used to introduce a biotin group to an aptamer, thereby allowing for the release of the aptamer later in an assay method.

Homogenous assays, done with all assay components in solution, do not require separation of sample and reagents prior to the detection of signal. These methods are rapid and easy to use. These methods generate signal based on a molecular capture or binding reagent that reacts with its specific target. In some embodiments of the methods described herein, the molecular capture reagents comprise one or more aptamers and/or antibodies or the like and the specific target of each of the one or more aptamers and/or antibodies or the like may be a biomarker shown in Table 1 or in Table 2.

In some embodiments, a method for signal generation takes advantage of anisotropy signal change due to the interaction of a fluorophore-labeled capture reagent with its specific biomarker target. When the labeled capture reacts with its target, the increased molecular weight causes the rotational motion of the fluorophore attached to the complex to become much slower changing the anisotropy value. By monitoring the anisotropy change, binding events may be used to quantitatively measure the biomarkers in solutions. Other methods include fluorescence polarization assays, molecular beacon methods, time resolved fluorescence quenching, chemiluminescence, fluorescence resonance energy transfer, and the like.

An exemplary solution-based aptamer assay that can be used to detect a biomarker level in a biological sample includes the following: (a) preparing a mixture by contacting the biological sample with an aptamer that includes a first tag and has a specific affinity for the biomarker, wherein an aptamer affinity complex is formed when the biomarker is present in the sample; (b) exposing the mixture to a first solid support including a first capture element, and allowing the first tag to associate with the first capture element; (c) removing any components of the mixture not associated with the first solid support; (d) attaching a second tag to the biomarker component of the aptamer affinity complex; (e) releasing the aptamer affinity complex from the first solid support; (f) exposing the released aptamer affinity complex to a second solid support that includes a second capture element and allowing the second tag to associate with the second capture element; (g)

14

removing any non-complexed aptamer from the mixture by partitioning the non-complexed aptamer from the aptamer affinity complex; (h) eluting the aptamer from the solid support; and (i) detecting the biomarker by detecting the aptamer component of the aptamer affinity complex.

Any means known in the art can be used to detect a biomarker value by detecting the aptamer component of an aptamer affinity complex. A number of different detection methods can be used to detect the aptamer component of an affinity complex, such as, for example, hybridization assays, mass spectroscopy, or QPCR. In some embodiments, nucleic acid sequencing methods can be used to detect the aptamer component of an aptamer affinity complex and thereby detect a biomarker value. Briefly, a test sample can be subjected to any kind of nucleic acid sequencing method to identify and quantify the sequence or sequences of one or more aptamers present in the test sample. In some embodiments, the sequence includes the entire aptamer molecule or any portion of the molecule that may be used to uniquely identify the molecule. In other embodiments, the identifying sequencing is a specific sequence added to the aptamer; such sequences are often referred to as "tags," "barcodes," or "zipcodes." In some embodiments, the sequencing method includes enzymatic steps to amplify the aptamer sequence or to convert any kind of nucleic acid, including RNA and DNA that contain chemical modifications to any position, to any other kind of nucleic acid appropriate for sequencing.

In some embodiments, the sequencing method includes one or more cloning steps. In other embodiments the sequencing method includes a direct sequencing method without cloning.

In some embodiments, the sequencing method includes a directed approach with specific primers that target one or more aptamers in the test sample. In other embodiments, the sequencing method includes a shotgun approach that targets all aptamers in the test sample.

In some embodiments, the sequencing method includes enzymatic steps to amplify the molecule targeted for sequencing. In other embodiments, the sequencing method directly sequences single molecules. An exemplary nucleic acid sequencing-based method that can be used to detect a biomarker value corresponding to a biomarker in a biological sample includes the following: (a) converting a mixture of aptamers that contain chemically modified nucleotides to unmodified nucleic acids with an enzymatic step; (b) shotgun sequencing the resulting unmodified nucleic acids with a massively parallel sequencing platform such as, for example, the 454 Sequencing System (454 Life Sciences/Roche), the Illumina Sequencing System (Illumina), the ABI SOLID Sequencing System (Applied Biosystems), the HeliScope Single Molecule Sequencer (Helicos Biosciences), or the Pacific Biosciences Real Time Single-Molecule Sequencing System (Pacific BioSciences) or the Polonator G Sequencing System (Dover Systems); and (c) identifying and quantifying the aptamers present in the mixture by specific sequence and sequence count.

A nonlimiting exemplary method of detecting biomarkers in a biological sample using aptamers is described in Example 1. *See also* Kraemer et al., 2011, PLoS One 6(10): e26332.

**Determination of Biomarker Levels using Immunoassays**

Immunoassay methods are based on the reaction of an antibody to its corresponding target or analyte and can detect the analyte in a sample depending on the specific assay format. To improve specificity and sensitivity of an assay method based on immuno-reactivity, monoclonal antibodies and fragments thereof are often used because of their specific epitope recognition. Polyclonal antibodies have also been successfully used in various immunoassays because of their increased affinity for the target as compared to monoclonal antibodies. Immunoassays have been designed for use with a wide range of biological sample matrices. Immunoassay formats have been designed to provide qualitative, semi-quantitative, and quantitative results.

Quantitative results are generated through the use of a standard curve created with known concentrations of the specific analyte to be detected. The response or signal from an unknown sample is plotted onto the standard curve, and a quantity or level corresponding to the target in the unknown sample is established.

Numerous immunoassay formats have been designed. ELISA or EIA can be quantitative for the detection of an analyte. This method relies on attachment of a label to either the analyte or the antibody and the label component includes, either directly or indirectly, an enzyme. ELISA tests may be formatted for direct, indirect, competitive, or sandwich detection of the analyte. Other methods rely on labels such as, for example, radioisotopes ($1^{125}$) or fluorescence. Additional techniques include, for example, agglutination, nephelometry, turbidimetry, Western blot, immunoprecipitation, immunocytochemistry, immunohistochemistry, flow cytometry, Luminex assay, and others (see ImmunoAssay: A Practical Guide, edited by Brian Law, published by Taylor & Francis, Ltd., 2005 edition).

Exemplary assay formats include enzyme-linked immunosorbent assay (ELISA), radioimmunoassay, fluorescent, chemiluminescence, and fluorescence resonance energy transfer (FRET) or time resolved-FRET (TR-FRET) immunoassays. Examples of procedures for detecting biomarkers include biomarker immunoprecipitation followed by quantitative methods that allow size and peptide level discrimination, such as gel electrophoresis, capillary electrophoresis, planar electrochromatography, and the like.

Methods of detecting and/or for quantifying a detectable label or signal generating material depend on the nature of the label. The products of reactions catalyzed by appropriate enzymes (where the detectable label is an enzyme; see above) can be, without limitation, fluorescent, luminescent, or radioactive or they may absorb visible or ultraviolet light. Examples of detectors suitable for detecting such detectable labels include, without limitation, x-ray film, radioactivity counters, scintillation counters, spectrophotometers, colorimeters, fluorometers, luminometers, and densitometers.

Any of the methods for detection can be performed in any format that allows for any suitable preparation, processing, and analysis of the reactions. This can be, for example, in multi-well assay plates (e.g., 96 wells or 386 wells) or using any suitable array or microarray. Stock solutions for various agents can be made manually or robotically, and all subsequent pipetting, diluting, mixing, distribution, washing, incubating, sample readout, data collection and analysis can be done robotically using commercially available analysis software, robotics, and detection instrumentation capable of detecting a detectable label.

**Determination of Biomarker Levels using Gene Expression Profiling**

Measuring mRNA in a biological sample may, in some embodiments, be used as a surrogate for detection of the level of the corresponding protein in the biological sample. Thus, in some embodiments, a biomarker or biomarker panel described herein can be detected by detecting the appropriate RNA.

In some embodiments, mRNA expression levels are measured by reverse transcription quantitative polymerase chain reaction (RT-PCR followed with qPCR). RT-PCR is used to create a cDNA from the mRNA. The cDNA may be used in a qPCR assay to produce fluorescence as the DNA amplification process progresses. By comparison to a standard curve, qPCR can produce an absolute measurement such as number of copies of mRNA per cell. Northern blots, microarrays, Invader assays, and RT-PCR combined with capillary electrophoresis have all been used to measure expression levels of mRNA in a sample. See Gene Expression Profiling: Methods and Protocols, Richard A. Shimkets, editor, Humana Press, 2004.

**Detection of Biomarkers Using *In Vivo* Molecular Imaging Technologies**

In some embodiments, a biomarker described herein may be used in molecular imaging tests. For example, an imaging agent can be coupled to a capture reagent, which can be used to detect the biomarker *in vivo.*

*In vivo* imaging technologies provide non-invasive methods for determining the state of a particular disease in the body of an individual. For example, entire portions of the body, or even the entire body, may be viewed as a three dimensional image, thereby providing valuable information concerning morphology and structures in the body. Such technologies may be combined with the detection of the biomarkers described herein to provide information concerning the biomarker *in vivo.*

The use of *In vivo* molecular imaging technologies is expanding due to various advances in technology. These advances include the development of new contrast agents or labels, such as radiolabels and/or fluorescent labels, which can provide strong signals within the body; and the development of powerful new imaging technology, which can detect and analyze these signals from outside the body, with sufficient sensitivity and accuracy to provide useful information. The contrast agent can be visualized in an appropriate imaging system, thereby providing an image of the portion or portions of the body in which the contrast agent is located. The contrast agent may be bound to or associated with a capture reagent, such as an aptamer or an antibody, for example, and/or with a peptide or protein, or an oligonucleotide (for example, for the detection of gene expression), or a complex containing any of these with one or more macromolecules and/or other particulate forms.

The contrast agent may also feature a radioactive atom that is useful in imaging. Suitable radioactive atoms include technetium-99m or iodine-123 for scintigraphic studies. Other readily detectable moieties include, for example, spin labels for magnetic resonance imaging (MM) such as, for example, iodine-123 again, iodine-131, indium-111, fluorine-19, carbon-13, nitrogen-15, oxygen-17, gadolinium, manganese or iron. Such labels are well known in the art and could easily be selected by one of ordinary skill in the art.

Standard imaging techniques include but are not limited to magnetic resonance imaging, computed tomography scanning, positron emission tomography (PET), single photon emission computed tomography (SPECT), and the like. For diagnostic *In vivo* imaging, the type of detection instrument available is a major factor in selecting a given contrast agent, such as a given radionuclide and the particular biomarker that it is used to target (protein, mRNA, and the like). The radionuclide chosen typically has a type of decay that is detectable by a given type of instrument. Also, when selecting a radionuclide for *In vivo* diagnosis, its half-life should be long enough to enable detection at the time of maximum uptake by the target tissue but short enough that deleterious radiation of the host is minimized.

Exemplary imaging techniques include but are not limited to PET and SPECT, which are imaging techniques in which a radionuclide is synthetically or locally administered to an individual. The subsequent uptake of the radiotracer is measured over time and used to obtain information about the targeted tissue and the biomarker. Because of the high-energy (gamma-ray) emissions of the specific isotopes employed and the sensitivity and sophistication of the instruments used to detect them, the two-dimensional distribution of radioactivity may be inferred from outside of the body.

Commonly used positron-emitting nuclides in PET include, for example, carbon-11, nitrogen-13, oxygen-15, and fluorine-18. Isotopes that decay by electron capture and/or gamma-emission are used in SPECT and include, for example iodine-123 and technetium-99m. An exemplary method for labeling amino acids with technetium-99m is the reduction of pertechnetate ion in the presence of a chelating precursor to form the labile technetium-99m-precursor complex, which, in turn, reacts with the metal binding group of a bifunctionally modified chemotactic peptide to form a technetium-99m-chemotactic peptide conjugate.

Antibodies are frequently used for such *in vivo* imaging diagnostic methods. The preparation and use of antibodies for *in vivo* diagnosis is well known in the art. Similarly, aptamers may be used for such *in vivo* imaging diagnostic methods. For example, an aptamer that was used to identify a particular biomarker described herein may be appropriately labeled and injected into an individual to detect the biomarker *in vivo.* The label used will be selected in accordance with the imaging modality to be used, as previously described. Aptamer-directed imaging agents could have unique and advantageous characteristics relating to tissue penetration, tissue distribution, kinetics, elimination, potency, and selectivity as compared to other imaging agents.

Such techniques may also optionally be performed with labeled oligonucleotides, for example, for detection of gene expression through imaging with antisense oligonucleotides. These methods are used for in situ hybridization, for example, with fluorescent molecules or radionuclides as the label. Other methods for detection of gene expression include, for example, detection of the activity of a reporter gene.

Another general type of imaging technology is optical imaging, in which fluorescent signals within the subject are detected by an optical device that is external to the subject. These signals may be due to actual fluorescence and/or to bioluminescence. Improvements in the sensitivity of optical detection devices have increased the usefulness of optical imaging for in vivo diagnostic assays.

For a review of other techniques, see N. Blow, Nature Methods, 6, 465-469, 2009.

**Determination of Biomarker Levels using Mass Spectrometry Methods**

A variety of configurations of mass spectrometers can be used to detect biomarker levels. Several types of mass spectrometers are available or can be produced with various configurations. In general, a mass spectrometer has the following major components: a sample inlet, an ion source, a mass analyzer, a detector, a vacuum system, and instrument-control system, and a data system. Difference in the sample inlet, ion source, and mass analyzer generally define the type of instrument and its capabilities. For example, an inlet can be a capillary-column liquid chromatography source or can be a direct probe or stage such as used in matrix-assisted laser desorption. Common ion sources are, for example, electrospray, including nanospray and microspray or matrix-assisted laser desorption. Common mass analyzers include a quadrupole mass filter, ion trap mass analyzer and time-of-flight mass analyzer. Additional mass spectrometry methods are well known in the art (see Burlingame et al. Anal. Chem. 70:647 R-716R (1998); Kinter and Sherman, New York (2000)).

Protein biomarkers and biomarker levels can be detected and measured by any of the following: electrospray ionization mass spectrometry (ESI-MS), ESI-MS/MS, ESI-MS/(MS)n, matrix-assisted laser desorption ionization time-of-flight mass spectrometry (MALDI-TOF-MS), surface-enhanced laser desorption/ionization time-of-flight mass spectrometry (SELDI-TOF-MS), desorption/ionization on silicon (DIOS), secondary ion mass spectrometry (SIMS), quadrupole time-of-flight (Q-TOF), tandem time-of-flight (TOF/TOF) technology, called ultraflex III TOF/TOF, atmospheric pressure chemical ionization mass spectrometry (APCI-MS), APCI-MS/MS, APCI-(MS)N, atmospheric pressure photoionization mass spectrometry (APPI-MS), APPI-MS/MS, and APPI-(MS)N, quadrupole mass spectrometry, Fourier transform mass spectrometry (FTMS), quantitative mass spectrometry, and ion trap mass spectrometry.

Sample preparation strategies are used to label and enrich samples before mass spectroscopic characterization of protein biomarkers and determination biomarker levels. Labeling methods include but are not limited to isobaric tag for relative and absolute quantitation (iTRAQ) and stable isotope labeling with amino acids in cell culture (SILAC). Capture reagents used to selectively enrich samples for candidate biomarker proteins prior to mass spectroscopic analysis include but are not limited to aptamers, antibodies, nucleic acid probes, chimeras, small molecules, an F(ab')2 fragment, a single chain antibody fragment, an Fv fragment, a single chain Fv fragment, a nucleic acid, a lectin, a ligand-binding receptor, affybodies, nanobodies, ankyrins, domain antibodies, alternative antibody scaffolds (e.g. diabodies etc) imprinted polymers, avimers, peptidomimetics, peptoids, peptide nucleic acids, threose nucleic acid, a hormone receptor, a cytokine receptor, and synthetic receptors, and modifications and fragments of these.

**Determination of Biomarker Levels using a Proximity Ligation Assay**

A proximity ligation assay can be used to determine biomarker values. Briefly, a test sample is contacted with a pair of affinity probes that may be a pair of antibodies or a pair of aptamers, with each member of the pair extended with an oligonucleotide. The targets for the pair of affinity probes may be two distinct determinates on one protein or one determinate on each of two different proteins, which may exist as homo- or hetero-multimeric complexes. When probes bind to the target determinates, the free ends of the oligonucleotide extensions are brought into sufficiently close proximity to hybridize together. The hybridization of the oligonucleotide extensions is facilitated by a common connector oligonucleotide which serves to bridge together the oligonucleotide extensions when they are positioned in sufficient proximity. Once the oligonucleotide extensions of the probes are hybridized, the ends of the extensions are joined together by enzymatic DNA ligation.

Each oligonucleotide extension comprises a primer site for PCR amplification. Once the oligonucleotide extensions are ligated together, the oligonucleotides form a continuous DNA sequence which, through PCR amplification, reveals information regarding the identity and amount of the target protein, as well as, information regarding protein-protein interactions where the target determinates are on two different proteins. Proximity ligation can provide a highly sensitive and specific assay for real-time protein concentration and interaction information through use of real-time PCR. Probes that do not bind the determinates of interest do not have the corresponding oligonucleotide extensions brought into proximity and no ligation or PCR amplification can proceed, resulting in no signal being produced.

The foregoing assays enable the detection of biomarker values that are useful in methods for prediction of risk or likelihood of CV events, where the methods comprise detecting, in a biological sample from an individual, at least two, at least three, at least four, at least five, at least six, at least seven, at least eight, at least nine, at least ten, at least eleven, at least twelve, at least thirteen, at least fourteen, at least fifteen, or all sixteen biomarkers selected from the biomarkers in Table 1; or at least two, at least three, at least four, at least five, at least six, at least seven, at least eight, at least nine, at least ten, at least eleven, at least twelve, at least thirteen, or all fourteen biomarkers selected from the biomarkers in Table 2, wherein a classification, as described below, using the biomarker values indicates whether the individual has elevated risk of a CV event occurring within a 90 day, 180 day, or 1 year time period. In accordance with any of the methods described herein, biomarker values can be **detected** and classified individually or they can be detected and classified collectively, as for example in a multiplex assay format.

Classification of Biomarkers and Calculation of Disease Scores

**In** some embodiments, a biomarker "signature" for a given diagnostic test contains a set of biomarkers, each biomarker having characteristic levels in the populations of interest. Characteristic levels, in some embodiments, may refer to the mean or average of the biomarker levels for the individuals in a particular group. **In** some embodiments, a diagnostic method described herein can be used to assign an unknown sample from an individual into one of two groups, either at increased risk of a CV event or not.

The assignment of a sample into one of two or more groups is known as classification, and the procedure used to accomplish this assignment is known as a classifier or a classification method. Classification methods may also be referred to as scoring methods. There are many classification methods that can be used to construct a diagnostic classifier from a set of biomarker levels. In some instances, classification methods are performed using supervised learning techniques in which a data set is collected using samples obtained from individuals within two (or more, for multiple classification states) distinct groups one wishes to distinguish. Since the class (group or population) to which each sample belongs is known in advance for each sample, the classification method can be trained to give the desired classification response. It is also possible to use unsupervised learning techniques to produce a diagnostic classifier.

Common approaches for developing diagnostic classifiers include decision trees; bagging + boosting + forests; rule inference based learning; Parzen Windows; linear models; logistic; neural network methods; unsupervised clustering; K-means; hierarchical ascending/ descending; semi-supervised learning; prototype methods; nearest neighbor; kernel density estimation; support vector machines; hidden Markov models; Boltzmann Learning; and classifiers may be combined either simply or in ways which minimize particular objective functions. For a review, see, e.g., Pattern Classification, R.O. Duda, et al., editors, John Wiley & Sons, 2nd edition, 2001; see also, The Elements of Statistical Learning - Data Mining, Inference, and Prediction, T. Hastie, et al., editors, Springer Science+Business Media, LLC, 2nd edition, 2009.

To produce a classifier using supervised learning techniques, a set of samples called training data are obtained. In the context of diagnostic tests, training data includes samples from the distinct groups (classes) to which unknown samples will later be assigned. For example, samples collected from individuals in a control population and individuals in a particular disease population can constitute training data to develop a classifier that can classify unknown samples (or, more particularly, the individuals from whom the samples were obtained) as either having the disease or being free from the disease. The development of the classifier from the training data is known as training the classifier. Specific details on classifier training depend on the nature of the supervised learning technique. Training a naive Bayesian classifier is an example of such a supervised learning technique (see, e.g., Pattern Classification, R.O. Duda, et al., editors, John Wiley & Sons, 2nd edition, 2001; see also, The Elements of Statistical Learning - Data Mining, Inference, and Prediction, T. Hastie, et al., editors, Springer Science+Business Media, LLC, 2nd edition, 2009). Training of a naive Bayesian classifier is described, e.g., in U.S. Publication Nos: 2012/0101002 and 2012/0077695.

Since typically there are many more potential biomarker levels than samples in a training set, care must be used to avoid over-fitting. Over-fitting occurs when a statistical model describes random error or noise instead of the underlying relationship. Over-fitting can be avoided in a variety of way, including, for example, by limiting the number of biomarkers used in developing the classifier, by assuming that the biomarker responses are independent of one another, by limiting the complexity of the underlying statistical model employed, and by ensuring that the underlying statistical model conforms to the data.

An illustrative example of the development of a diagnostic test using a set of biomarkers includes the application of a naive Bayes classifier, a simple probabilistic classifier based on Bayes theorem with strict independent treatment of the biomarkers. Each biomarker is described by a class-dependent probability density function (pdf) for the measured RFU values or log RFU (relative fluorescence units) values in each class. The joint pdfs for the set of biomarkers in one class is assumed to be the product of the individual class-dependent pdfs for each biomarker. Training a naive Bayes classifier in this context amounts to assigning parameters ("parameterization") to characterize the class dependent pdfs. Any underlying model for the class-dependent pdfs may be used, but the model should generally conform to the data observed in the training set.

The performance of the naive Bayes classifier is dependent upon the number and quality of the biomarkers used to construct and train the classifier. A single biomarker will perform in accordance with its KS-distance (Kolmogorov-Smirnov). The addition of subsequent biomarkers with good KS distances (>0.3, for example) will, in general, improve the classification performance if the subsequently added biomarkers are independent of the first biomarker. Using the sensitivity plus specificity as a classifier score, many high scoring classifiers can be generated with a variation of a greedy algorithm. (A greedy algorithm is any algorithm that follows the problem solving metaheuristic of making the locally optimal choice at each stage with the hope of finding the global optimum.)

Another way to depict classifier performance is through a receiver operating characteristic (ROC), or simply ROC curve or ROC plot. The ROC is a graphical plot of the sensitivity, or true positive rate, vs. false positive rate (1 - specificity or 1 - true negative rate), for a binary classifier system as its discrimination threshold is varied. The ROC can also be represented equivalently by plotting the fraction of true positives out of the positives (TPR = true positive rate) vs. the fraction of false positives out of the negatives (FPR = false positive rate). Also known as a Relative Operating Characteristic curve, because it is a comparison of two operating characteristics (TPR & FPR) as the criterion changes. The area under the ROC curve (AUC) is commonly used as a summary measure of diagnostic accuracy. It can take values from 0.0 to 1.0. The AUC has an important statistical property: the AUC of a classifier is equivalent to the probability that the classifier will rank a randomly chosen positive instance higher than a randomly chosen negative instance (Fawcett T, 2006. An introduction to ROC analysis. Pattern Recognition Letters .27: 861-874). This is equivalent to the Wilcoxon test of ranks (Hanley, J.A., McNeil, B.J., 1982. The meaning and use of the area under a receiver operating characteristic (ROC) curve. Radiology 143, 29-36.). Another way of describing performance of a diagnostic test in relation to a known reference standard is the net reclassification index: the ability of the new test to correctly upgrade or downgrade risk when compared with the reference standard test. See, e.g., Pencina et al., 2011, Stat. Med. 30: 11-21. While the AUC under the ROC curve is optimal for assessing performance of a 2-class classifier, stratified and personalized medicine relies upon the inference that the population contains more classes than 2. For such comparisons the hazard ratio of the upper vs. lower quartiles (or other stratifications such as deciles) can be used more appropriately.

The risk and likelihood predictions enabled herein may be applied to individuals in primary care or in specialist cardiovascular centers, or even direct to the consumer. In some embodiments, the classifiers used to predict events may involve some calibration to the population to which they are applied - for example there may be variations due to ethnicity or geography. Such calibrations, in some embodiments, may be established in advance from large population studies, so when applied to an individual patient these are incorporated prior to making a risk prediction. A venous blood sample is taken, processed appropriately and analyzed as described herein. Once the analysis is complete, the risk predictions may be made mathematically, with or without incorporating other metadata from medical records described herein such as genetic or demographic. Various forms of output of information are possible depending on the level of expertise of the consumer. For consumers seeking the simplest type of output the information may be, in some embodiments, "is this person likely to have an event in the next x days (where x is 90-365), yes/no" or alternatively akin to a "traffic light" red/orange/green or its verbal or written equivalent such as high/medium/low risk. For consumers seeking greater detail, in some embodiments, the risk may be output as a number or a graphic illustrating the probability of an event per unit time as a continuous score, or a greater number of strata (such as deciles), and/or the average time to event and/or the most likely type of event. In some embodiments, the output may include therapeutic recommendations. Longitudinal monitoring of the same patient over time will enable graphics showing response to interventions or lifestyle changes. In some embodiments, more than one type of output may be provided at the same time to fulfill the needs of the patient and of individual members of the care team with differing levels of expertise.

In some embodiments, the biomarkers shown in Table 1 or Table 2 are detected in a blood sample (such as a plasma sample or a serum sample) from a subject, for example, using aptamers, such as slow off-rate aptamers. The log RFU values are used to calculate an individual's risk or likelihood of having a CV event, or a prognostic index (PI).

Given the PI, the probability that the subject will suffer a cardiovascular event (CV event) in the next "t" days is given by the formula:

$$\Pr[T \leq t] = 1 - e^{-e^{\left(\frac{Log(t) - PI}{S}\right)}},$$

where **PI** is the *prognostic index* (or linear predictor) and s is the associated scale parameter for the extreme value distribution. In various embodiments, "t" is 90 to 365 days.

Kits

Any combination of the biomarkers described herein can be detected using a suitable kit, such as for use in performing the methods disclosed herein. Furthermore, any kit can contain one or more detectable labels as described herein, such as a fluorescent moiety, etc.

In some embodiments, a kit includes (a) one or more capture reagents (such as, for example, at least one aptamer or antibody) for detecting one or more biomarkers in a biological sample, wherein the biomarkers include at least two, at least three, at least four, at least five, at least six, at least seven, at least eight, at least nine, at least ten, at least eleven, at least twelve, at least thirteen, at least fourteen, at least fifteen, or all sixteen biomarkers selected from the biomarkers in Table 1; or at least two, at least three, at least four, at least five, at least six, at least seven, at least eight, at least nine, at least ten, at least eleven, at least twelve, at least thirteen, or all fourteen biomarkers selected from the biomarkers in Table 2, and optionally (b) one or more software or computer program products for classifying the individual from whom the biological sample was obtained as either having or not having increased risk of a CV event or for determining the likelihood that the individual has increased risk of a CV event, as further described herein. Alternatively, rather than one or more computer program products, one or more instructions for manually performing the above steps by a human can be provided.

In some embodiments, a kit comprises a solid support, at least one capture reagent, and a signal generating material. The kit can also include instructions for using the devices and reagents, handling the sample, and analyzing the data. Further the kit may be used with a computer system or software to analyze and report the result of the analysis of the biological sample.

The kits can also contain one or more reagents (e.g., solubilization buffers, detergents, washes, or buffers) for processing a biological sample. Any of the kits described herein can also include, e.g., buffers, blocking agents, mass spectrometry matrix materials, antibody capture agents, positive control samples, negative control samples, software and information such as protocols, guidance and reference data.

In some embodiments kits are provided for the analysis of CV event risk status, wherein the kits comprise PCR primers for one or more aptamers specific to biomarkers described herein. In some embodiments, a kit may further include instructions for use and correlation of the biomarkers with prediction of risk of a CV event. In some embodiments, a kit may also include a DNA array containing the complement of one or more of the aptamers specific for the biomarkers described herein, reagents, and/or enzymes for amplifying or isolating sample DNA. In some embodiments, kits may include reagents for real-time PCR, for example, TaqMan probes and/or primers, and enzymes.

For example, a kit can comprise (a) reagents comprising at least one capture reagent for determining the level of one or more biomarkers in a test sample, and optionally (b) one or more algorithms or computer programs for performing the steps of comparing the amount of each biomarker quantified in the test sample to one or more predetermined cutoffs. In some embodiments, an algorithm or computer program assigns a score for each biomarker quantified based on said comparison and, in some embodiments, combines the assigned scores for each biomarker quantified to obtain a total score. Further, in some embodiments, an algorithm or computer program compares the total score with a predetermined score, and uses the comparison to determine whether an individual has an increased risk of a CV event. Alternatively, rather than one or more algorithms or computer programs, one or more instructions for manually performing the above steps by a human can be provided.

**Biomarker Panels**

In some embodiments, one or more of the biomarkers listed in Table 1 are detected. In some embodiments, the one or more biomarkers listed in Table 1 are detected in a sample from an individual having HFrEF. In some embodiments, all of the biomarkers listed in Table 1 are detected. In some embodiments, the level of each protein listed in Table 1 is detected. In some embodiments, the detecting of the one or more biomarkers or all of the biomarkers is performed in order to determine the risk or likelihood a subject will have a CV event within a defined time period. In some such embodiments, the defined time period is 90 days, 180 days, or one year. In some embodiments, the defined time period is one year. In some embodiments, the CV event is death.

**Table 1**

| Protein | Gene | UniProt ID | Name |
|---|---|---|---|
| N-terminal pro-BNP | NPPB | P 16860 | N-terminal pro-BNP |
| RSPO4 | RSPO4 | Q210M5 | R-spondin-4 |
| ATL2 | ADAMTSL2 | Q86TH1 | ADAMTS-like protein 2 |
| BNP | NPPB | P 16860 | Natriuretic peptides B |
| MIC-1 | GDF15 | Q99988 | Growth/differentiation Factor 15 |
| FABPA | FABP4 | P15090 | Fatty acid-binding protein, adipocyte |
| HCC-1 | CCL14 | Q16627 | C-C motif chemokine 14 |
| ILRL1 | IL1RL1 | Q01638 | Interleukin-1 receptor-like 1 |
| ANGP2 | ANGPT2 | 015123 | Angiopoietin-2 |
| HE4 | WFDC2 | Q14508 | WAP four-disulfide core domain protein 2 |
| TAGL | TAGLN | Q01995 | Transgelin |
| RNAS6 | RNASE6 | Q93091 | Ribonuclease K6 |
| PAP1 | REG3A | Q06141 | Regenerating islet-derived protein 3-alpha |
| RNAS1 | RNASE1 | P07998 | Ribonuclease pancreatic |
| TSP2 | THBS2 | P35442 | Thrombospondin-2 |
| SVEP1 | SVEP1 | Q4LDE5 | Sushi, von Willebrand factor type A, EGF and pentraxin domain-containing protein 1 |

In some embodiments, one or more of the biomarkers listed in Table 2 is detected. In some embodiments, the one or more biomarkers listed in Table 2 are detected in a sample from an individual having HFpEF. In some embodiments, all of the biomarkers listed in the table below are detected. In some embodiments, the level of each protein listed in Table 2 is detected. In some embodiments, the detecting of the one or more biomarkers or all of the biomarkers is performed in order to determine the risk or likelihood a subject will have a CV event within a defined time period. In some such embodiments, the defined time period is 90 days, 180 days, or one year. In some embodiments, the defined time period is one year. In some embodiments, the CV event is death.

**Table 2**

| Protein | Gene | UniProt ID | Name |
|---|---|---|---|
| Tetranectin | CLEC3B | P05452 | Tetranectin |
| N-terminal pro-BNP | NPPB | P16860 | N-terminal pro-BNP |
| TNNT2 | TNNT2 | P45379 | Troponin T, cardiac muscle |
| RET | RET | P07949 | Proto-oncogene tyrosine-protein kinase receptor ret |
| CA125 | MUC16 | Q8WXI7 | Mucin-16 |
| CRDL1 | CHRDL1 | Q9BU40 | Chordin-like protein 1 |
| MIC-1 | GDF15 | Q99988 | Growth/differentiation factor 15 |
| SLPI | SLPI | P03973 | Antileukoproteinase |
| HE4 | WFDC2 | Q14508 | Wap four-disulfide core domain protein 2 |
| MMP-12 | MMP12 | P39900 | Macrophage metalloelastase |
| HSPB6 | HSPB6 | 014558 | Heat shock protein beta-6 |
| WISP-2 | CCNS | O76076 | Wntl-inducible-signaling pathway protein 2 |
| GHR | GHR | P10912 | Growth hormone receptor |
| IGFBP-2 | IGFBP2 | P18065 | Insulin-like growth factor-binding protein 2 |

**Computer Methods and Software**

A method for assessing the risk or likelihood of a CV event in an individual can comprise the following: 1) obtain a biological sample; 2) perform an analytical method to detect and measure a biomarker or set of biomarkers in a panel in the biological sample; 3) optionally perform any data normalization or standardization; 4) determine each biomarker level; and 5) report the results. In some embodiments, the results are calibrated to the population / ethnicity of the subject. In some embodiments, the biomarker levels are combined in some way and a single value for the combined biomarker levels is reported. In this approach, in some embodiments, the score may be a single number determined from the integration of all the biomarkers that is compared to a pre-set threshold value that is an indication of the presence or absence of disease. Or the diagnostic or predictive score may be a series of bars that each represent a biomarker value and the pattern of the responses may be compared to a pre-set pattern for determination of the presence or absence of disease, condition or the increased risk (or not) of an event.

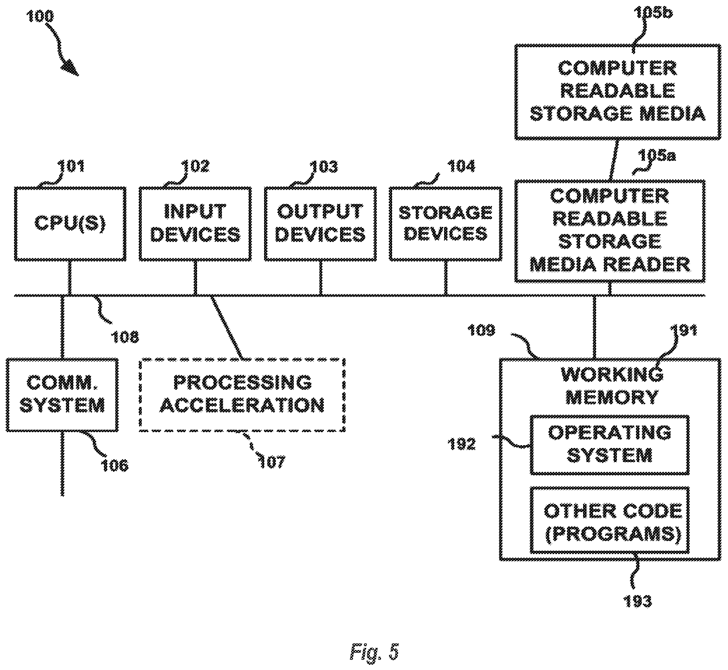

At least some embodiments of the methods described herein can be implemented with the use of a computer. An example of a computer system 100 is shown in Figure 5. With reference to Figure 5, system 100 is shown comprised of hardware elements that are electrically coupled via bus 108, including a processor 101, input device 102, output device 103, storage device 104, computer-readable storage media reader 105a, communications system 106, processing acceleration (e.g., DSP or special-purpose processors) 107 and memory 109. Computer-readable storage media reader 105a is further coupled to computer-readable storage media 105b, the combination comprehensively representing remote, local, fixed and/or removable storage devices plus storage media, memory, etc. for temporarily and/or more permanently containing computer-readable information, which can include storage device 104, memory 109 and/or any other such accessible system 100 resource. System 100 also comprises software elements (shown as being currently located within working memory 191) including an operating system 192 and other code 193, such as programs, data and the like.

With respect to Figure 5, system 100 has extensive flexibility and configurability. Thus, for example, a single architecture might be utilized to implement one or more servers that can be further configured in accordance with currently desirable protocols, protocol variations, extensions, etc. However, it will be apparent to those skilled in the art that embodiments may well be utilized in accordance with more specific application requirements. For example, one or more system elements might be implemented as sub-elements within a system 100 component (e.g., within communications system 106). Customized hardware might also be utilized and/or particular elements might be implemented in hardware, software or both. Further, while connection to other computing devices such as network input/output devices (not shown) may be employed, it is to be understood that wired, wireless, modem, and/or other connection or connections to other computing devices might also be utilized.

In one aspect, the system can comprise a database containing features of biomarkers characteristic of prediction of risk of a CV event. The biomarker data (or biomarker information) can be utilized as an input to the computer for use as part of a computer implemented method. The biomarker data can include the data as described herein.

In one aspect, the system further comprises one or more devices for providing input data to the one or more processors.

The system further comprises a memory for storing a data set of ranked data elements.

In another aspect, the device for providing input data comprises a detector for detecting the characteristic of the data element, e.g., such as a mass spectrometer or gene chip reader.

The system additionally may comprise a database management system. User requests or queries can be formatted in an appropriate language understood by the database management system that processes the query to extract the relevant information from the database of training sets.

The system may be connectable to a network to which a network server and one or more clients are connected. The network may be a local area network (LAN) or a wide area network (WAN), as is known in the art. Preferably, the server includes the hardware necessary for running computer program products (e.g., software) to access database data for processing user requests.

The system may include an operating system (e.g., UNIX or Linux) for executing instructions from a database management system. In one aspect, the operating system can operate on a global communications network, such as the internet, and utilize a global communications network server to connect to such a network.

The system may include one or more devices that comprise a graphical display interface comprising interface elements such as buttons, pull down menus, scroll bars, fields for entering text, and the like as are routinely found in graphical user interfaces known in the art. Requests entered on a user interface can be transmitted to an application program in the system for formatting to search for relevant information in one or more of the system databases. Requests or queries entered by a user may be constructed in any suitable database language.

The graphical user interface may be generated by a graphical user interface code as part of the operating system and can be used to input data and/or to display inputted data. The result of processed data can be displayed in the interface, printed on a printer in communication with the system, saved in a memory device, and/or transmitted over the network or can be provided in the form of the computer readable medium.

The system can be in communication with an input device for providing data regarding data elements to the system (e.g., expression values). In one aspect, the input device can include a gene expression profiling system including, e.g., a mass spectrometer, gene chip or array reader, and the like.

The methods and apparatus for analyzing CV event risk prediction biomarker information according to various embodiments may be implemented in any suitable manner, for example, using a computer program operating on a computer system. A conventional computer system comprising a processor and a random access memory, such as a remotely-accessible application server, network server, personal computer or workstation may be used. Additional computer system components may include memory devices or information storage systems, such as a mass storage system and a user interface, for example a conventional monitor, keyboard and tracking device. The computer system may be a stand-alone system or part of a network of computers including a server and one or more databases.

The CV event risk prediction biomarker analysis system can provide functions and operations to complete data analysis, such as data gathering, processing, analysis, reporting and/or diagnosis. For example, in one embodiment, the computer system can execute the computer program that may receive, store, search, analyze, and report information relating to the CV event risk prediction biomarkers. The computer program may comprise multiple modules performing various functions or operations, such as a processing module for processing raw data and generating supplemental data and an analysis module for analyzing raw data and supplemental data to generate a CV event risk prediction status and/or diagnosis or risk calculation. Calculation of risk status for a CV event may optionally comprise generating or collecting any other information, including additional biomedical information, regarding the condition of the individual relative to the disease, condition or event, identifying whether further tests may be desirable, or otherwise evaluating the health status of the individual.

Some embodiments described herein can be implemented so as to include a computer program product. A computer program product may include a computer readable medium having computer readable program code embodied in the medium for causing an application program to execute on a computer with a database.

As used herein, a "computer program product" refers to an organized set of instructions in the form of natural or programming language statements that are contained on a physical media of any nature (e.g., written, electronic, magnetic, optical or otherwise) and that may be used with a computer or other automated data processing system. Such programming language statements, when executed by a computer or data processing system, cause the computer or data processing system to act in accordance with the particular content of the statements. Computer program products include without limitation: programs in source and object code and/or test or data libraries embedded in a computer readable medium. Furthermore, the computer program product that enables a computer system or data processing equipment device to act in pre-selected ways may be provided in a number of forms, including, but not limited to, original source code, assembly code, object code, machine language, encrypted or compressed versions of the foregoing and any and all equivalents.

In one aspect, a computer program product is provided for evaluation of the risk or likelihood of a CV event. The computer program product includes a computer readable medium embodying program code executable by a processor of a computing device or system, the program code comprising: code that retrieves data attributed to a biological sample from an individual, wherein the data comprises biomarker levels that each correspond to one of the biomarkers in Table 1 or Table 2; and code that executes a classification method that indicates a CV event risk status of the individual as a function of the biomarker values.

In still another aspect, a computer program product is provided for indicating a likelihood or risk of a CV event. The computer program product includes a computer readable medium embodying program code executable by a processor of a computing device or system, the program code comprising: code that retrieves data attributed to a biological sample from an individual, wherein the data comprises a biomarker value corresponding to at least one biomarker in the biological sample selected from the biomarkers provided in Table 1 or 2; and code that executes a classification method that indicates a CV event risk status of the individual as a function of the biomarker value.

While various embodiments have been described as methods or apparatuses, it should be understood that embodiments can be implemented through code coupled with a computer, e.g., code resident on a computer or accessible by the computer. For example, software and databases could be utilized to implement many of the methods discussed above. Thus, in addition to embodiments accomplished by hardware, it is also noted that these embodiments can be accomplished through the use of an article of manufacture comprised of a computer usable medium having a computer readable program code embodied therein, which causes the enablement of the functions disclosed in this description. Therefore, it is desired that embodiments also be considered protected by this patent in their program code means as well. Furthermore, the embodiments may be embodied as code stored in a computer-readable memory of virtually any kind including, without limitation, RAM, ROM, magnetic media, optical media, or magneto-optical media. Even more generally, the embodiments could be implemented in software, or in hardware, or any combination thereof including, but not limited to, software running on a general purpose processor, microcode, programmable logic arrays (PLAs), or application-specific integrated circuits (ASICs).

It is also envisioned that embodiments could be accomplished as computer signals embodied in a carrier wave, as well as signals (e.g., electrical and optical) propagated through a transmission medium. Thus, the various types of information discussed above could be formatted in a structure, such as a data structure, and transmitted as an electrical signal through a transmission medium or stored on a computer readable medium.

It is also noted that many of the structures, materials, and acts recited herein can be recited as means for performing a function or step for performing a function. Therefore, it should be understood that such language is entitled to cover all such structures, materials, or acts disclosed within this specification and their equivalents, including the matter incorporated by reference.

The utilization of the biomarkers disclosed herein, and the various methods for determining biomarker values are described in detail above with respect to evaluation of risk of a CV event. However, the application of the process, the use of identified biomarkers, and the methods for determining biomarker values are fully applicable to other specific types of cardiovascular conditions, to any other disease or medical condition, or to the identification of individuals who may or may not be benefited by an ancillary medical treatment.

**Other Methods**

In some embodiments, the biomarkers and methods described herein are used to determine a medical insurance premium or coverage decision and/or a life insurance premium or coverage decision. In some embodiments, the results of the methods described herein are used to determine a medical insurance premium and/or a life insurance premium. In some such instances, an organization that provides medical insurance or life insurance requests or otherwise obtains information concerning a subject's risk or likelihood of a CV event and uses that information to determine an appropriate medical insurance or life insurance premium for the subject. In some embodiments, the test is requested by, and paid for by, the organization that provides medical insurance or life insurance. In some embodiments, the test is used by the potential acquirer of a practice or health system or company to predict future liabilities or costs should the acquisition go ahead.

In some embodiments, the biomarkers and methods described herein are used to predict and/or manage the utilization of medical resources. In some such embodiments, the methods are not carried out for the purpose of such prediction, but the information obtained from the method is used in such a prediction and/or management of the utilization of medical resources. For example, a testing facility or hospital may assemble information from the present methods for many subjects in order to predict and/or manage the utilization of medical resources at a particular facility or in a particular geographic area.

**EXAMPLES**

The following examples are provided for illustrative purposes only and are not intended to limit the scope of the application as defined by the appended claims. Routine molecular biology techniques described in the following examples can be carried out as described in standard laboratory manuals, such as Sambrook et al., Molecular Cloning: A Laboratory Manual, 3rd. ed., Cold Spring Harbor Laboratory Press, Cold Spring Harbor, N.Y., (2001).

**Example 1: Exemplary Biomarker Detection Using Aptamers**

An exemplary method of detecting one or more biomarker proteins in a sample is described, e.g., in Kraemer et al., PLoS One 6(10): e26332, and is described below. Three different methods of quantification: microarray-based hybridization, a Luminex bead-based method, and qPCR, are described.

Reagents

HEPES, NaCl, KC1, EDTA, EGTA, MgCl2 and Tween-20 may be purchased, e.g., from Fisher Biosciences. Dextran sulfate sodium salt (DxSO4), nominally 8000 molecular weight, may be purchased, e.g., from AIC and is dialyzed against deionized water for at least 20 hours with one exchange. KOD EX DNA polymerase may be purchased, e.g., from VWR. Tetramethylammonium chloride and CAPSO may be purchased, e.g., from Sigma-Aldrich and streptavidin-phycoerythrin (SAPE) may be purchased, e.g., from Moss Inc. 4-(2-Aminoethyl)-benzenesulfonylfluoride hydrochloride (AEBSF) may be purchased, e.g., from Gold Biotechnology. Streptavidin-coated 96-well plates may be purchased, e.g., from Thermo Scientific (Pierce Streptavidin Coated Plates HBC, clear, 96-well, product number 15500 or 15501). NHS-PEO4-biotin may be purchased, e.g., from Thermo Scientific (EZ-Link NHS-PEO4-Biotin, product number 21329), dissolved in anhydrous DMSO, and may be stored frozen in single-use aliquots. IL-8, MIP-4, Lipocalin-2, RANTES, MMP-7, and MMP-9 may be purchased, e.g., from R&D Systems. Resistin and MCP-1 may be purchased, e.g., from PeproTech, and tPA may be purchased, e.g., from VWR.

Nucleic acids

Conventional (including amine- and biotin-substituted) oligodeoxynucleotides may be purchased, e.g., from Integrated DNA Technologies (IDT). Z-Block is a single-stranded oligodeoxynucleotide of sequence 5'- (AC-BnBn)7-AC-3', where Bn indicates a benzyl-substituted deoxyuridine residue. Z-block may be synthesized using conventional phosphoramidite chemistry. Aptamer capture reagents may also be synthesized by conventional phosphoramidite chemistry, and may be purified, for example, on a 21.5x75 mm PRP-3 column, operating at 80°C on a Waters Autopurification 2767 system (or Waters 600 series semi-automated system), using, for example, a timberline TL-600 or TL-150 heater and a gradient of triethylammonium bicarbonate (TEAB) / ACN to elute product. Detection is performed at 260 nm and fractions are collected across the main peak prior to pooling best fractions.

Buffers

Buffer SB18 is composed of 40 mM HEPES, 101 mM NaCl, 5 mM KC1, 5 mM MgC12, and 0.05% (v/v) Tween 20 adjusted to pH 7.5 with NaOH. Buffer SB17 is SB18 supplemented with 1 mM trisodium EDTA. Buffer PB1 is composed of 10 mM HEPES, 101 mM NaCl, 5 mM KC1, 5 mM MgC12, 1 mM trisodium EDTA and 0.05% (v/v) Tween-20 adjusted to pH 7.5 with NaOH. CAPSO elution buffer consists of 100 mM CAPSO pH 10.0 and 1 M NaCl. Neutralization buffer contains of 500 mM HEPES, 500 mM HC1, and 0.05% (v/v) Tween-20. Agilent Hybridization Buffer is a proprietary formulation that is supplied as part of a kit (Oligo aCGH/ChIP-on-chip Hybridization Kit). Agilent Wash Buffer 1 is a proprietary formulation (Oligo aCGH/ChIP-on-chip Wash Buffer 1, Agilent). Agilent Wash Buffer 2 is a proprietary formulation (Oligo aCGH/ChIP-on-chip Wash Buffer 2, Agilent). TMAC hybridization solution consists of 4.5 M tetramethylammonium chloride, 6 mM trisodium EDTA, 75 mM Tris-HC1 (pH 8.0), and 0.15% (v/v) Sarkosyl. KOD buffer (10-fold concentrated) consists of 1200 mM Tris-HC1, 15 mM MgSO4, 100 mM KC1, 60 mM (NH4)2SO4, 1% v/v Triton-X 100 and 1 mg/mL BSA.

Sample preparation

Serum (stored at -80°C in 100 μL aliquots) is thawed in a 25°C water bath for 10 minutes, then stored on ice prior to sample dilution. Samples are mixed by gentle vortexing for 8 seconds. A 6% serum sample solution is prepared by dilution into 0.94x SB17 supplemented with 0.6 mM MgC12, 1 mM trisodium EGTA, 0.8 mM AEBSF, and 2μM Z-Block. A portion of the 6% serum stock solution is diluted 10-fold in SB17 to create a 0.6% serum stock. 6% and 0.6% stocks are used, in some embodiments, to detect high- and low-abundance analytes, respectively.

Capture reagent (aptamer) and streptavidin plate preparation

Aptamers are grouped into 2 mixes according to the relative abundance of their cognate analytes (or biomarkers). Stock concentrations are 4 nM for each aptamer, and the final concentration of each aptamer is 0.5 nM. Aptamer stock mixes are diluted 4-fold in SB17 buffer, heated to 95°C for 5 min and cooled to 37°C over a 15-minute period prior to use. This denaturation-renaturation cycle is intended to normalize aptamer conformer distributions and thus ensure reproducible aptamer activity in spite of variable histories. Streptavidin plates are washed twice with 150 μL buffer PB1 prior to use.

Incubation and plate capture

Heat-cooled 2x Aptamer mixes (55 μL) are combined with an equal volume of 6% or 0.6% serum dilutions, producing mixes containing 3% and 0.3% serum. The plates are sealed with a Silicone Sealing Mat (Axymat Silicone sealing mat, VWR) and incubated for 1.5 h at 37°C. Mixes are then transferred to the wells of a washed 96-well streptavidin plate and further incubated on an Eppendorf Thermomixer set at 37°C, with shaking at 800 rpm, for two hours.

Manual Assay

Unless otherwise specified, liquid is removed by dumping, followed by two taps onto layered paper towels. Wash volumes are 150 μL and all shaking incubations are done on an Eppendorf Thermomixer set at 25°C, 800 rpm. Mixes are removed by pipetting, and plates are washed twice for 1 minute with buffer PB1 supplemented with 1 mM dextran sulfate and 500 μM biotin, then 4 times for 15 seconds with buffer PB1. A freshly made solution of 1 mM NHS-PEO4-biotin in buffer PB1 (150 μL/well) is added, and plates are incubated for 5 minutes with shaking. The NHS-biotin solution is removed, and plates washed 3 times with buffer PB1 supplemented with 20 mM glycine, and 3 times with buffer PB1. Eighty-five μL of buffer PB1 supplemented with 1 mM DxSO4 is then added to each well, and plates are irradiated under a BlackRay UV lamp (nominal wavelength 365 nm) at a distance of 5 cm for 20 minutes with shaking. Samples are transferred to a fresh, washed streptavidin-coated plate, or an unused well of the existing washed streptavidin plate, combining high and low sample dilution mixtures into a single well. Samples are incubated at room temperature with shaking for 10 minutes. Unadsorbed material is removed and the plates washed 8 times for 15 seconds each with buffer PB1 supplemented with 30% glycerol. Plates are then washed once with buffer PB1. Aptamers are eluted for 5 minutes at room temperature with 100 μL CAPSO elution buffer. 90 μL of the eluate is transferred to a 96-well HybAid plate and 10 μL neutralization buffer is added.

Semi-Automated Assay

Streptavidin plates bearing adsorbed equilibration mixes are placed on the deck of a BioTek EL406 plate washer, which is programmed to perform the following steps: unadsorbed material is removed by aspiration, and wells are washed 4 times with 300 μL of buffer PB1 supplemented with 1 mM dextran sulfate and 500 μM biotin. Wells are then washed 3 times with 300 μL buffer PB1. One hundred fifty μL of a freshly prepared (from a 100 mM stock in DMSO) solution of 1 mM NHS-PEO4-biotin in buffer PB1 is added. Plates are incubated for 5 minutes with shaking. Liquid is aspirated, and wells are washed 8 times with 300 μL buffer PB1 supplemented with 10 mM glycine. One hundred μL of buffer PB1 supplemented with 1 mM dextran sulfate are added. After these automated steps, plates are removed from the plate washer and placed on a thermoshaker mounted under a UV light source (BlackRay, nominal wavelength 365 nm) at a distance of 5 cm for 20 minutes. The thermoshaker is set at 800 rpm and 25°C. After 20 minutes irradiation, samples are manually transferred to a fresh, washed streptavidin plate (or to an unused well of the existing washed plate). High-abundance (3% serum+3% aptamer mix) and low-abundance reaction mixes (0.3% serum+0.3% aptamer mix) are combined into a single well at this point. This "Catch-2" plate is placed on the deck of BioTek EL406 plate washer, which is programmed to perform the following steps: the plate is incubated for 10 minutes with shaking. Liquid is aspirated, and wells are washed 21 times with 300 μL buffer PB1 supplemented with 30% glycerol. Wells are washed 5 times with 300 μL buffer PB1, and the final wash is aspirated. One hundred CAPSO elution buffer are added, and aptamers are eluted for 5 minutes with shaking. Following these automated steps, the plate is then removed from the deck of the plate washer, and 90 μL aliquots of the samples are transferred manually to the wells of a HybAid 96-well plate that contains 10 μL neutralization buffer.

Hybridization to custom Agilent 8x15k microarrays

24 μL of the neutralized eluate is transferred to a new 96-well plate and 6μL of 10x Agilent Block (Oligo aCGH/ChIP-on-chip Hybridization Kit, Large Volume, Agilent 5188-5380), containing a set of hybridization controls composed of 10 Cy3 aptamers is added to each well. Thirty μL 2x Agilent Hybridization buffer is added to each sample and mixed. Forty μL of the resulting hybridization solution is manually pipetted into each "well" of the hybridization gasket slide (Hybridization Gasket Slide, 8-microarray per slide format, Agilent). Custom Agilent microarray slides, bearing 10 probes per array complementary to 40 nucleotide random region of each aptamer with a 20x dT linker, are placed onto the gasket slides according to the manufacturers' protocol. The assembly (Hybridization Chamber Kit - SureHyb-enabled, Agilent) is clamped and incubated for 19 hours at 60°C while rotating at 20 rpm.

Post Hybridization Washing

Approximately 400 mL Agilent Wash Buffer 1 is placed into each of two separate glass staining dishes. Slides (no more than two at a time) are disassembled and separated while submerged in Wash Buffer 1, then transferred to a slide rack in a second staining dish also containing Wash Buffer 1. Slides are incubated for an additional 5 minutes in Wash Buffer 1 with stirring. Slides are transferred to Wash Buffer 2 pre-equilibrated to 37°C and incubated for 5 minutes with stirring. Slides are transferred to a fourth staining dish containing acetonitrile, and incubated for 5 minutes with stirring.

Microarray Imaging

Microarray slides are imaged with an Agilent G2565CA Microarray Scanner System, using the Cy3-channel at 5μm resolution at 100% PMT setting, and the XRD option enabled at 0.05. The resulting TIFF images are processed using Agilent feature extraction software version 10.5.1.1 with the GE1 105 Dec08 protocol.

Luminex probe design

Probes immobilized to beads have 40 deoxynucleotides complementary to the 3' end of the 40 nucleotide random region of the target aptamer. The aptamer complementary region is coupled to Luminex Microspheres through a hexaethyleneglycol (HEG) linker bearing a 5' amino terminus. Biotinylated detection deoxyoligonucleotides comprise 17-21 deoxynucleotides complementary to the 5' primer region of target aptamers. Biotin moieties are appended to the 3' ends of detection oligos.

Coupling of probes to Luminex Microspheres

Probes are coupled to Luminex Microplex Microspheres essentially per the manufacturer's instructions, but with the following modifications: amino-terminal oligonucleotide amounts are 0.08 nMol per $2.5 \times 10^6$ microspheres, and the second EDC addition is $5\mu L$ at 10 mg/mL. Coupling reactions are performed in an Eppendorf ThermoShaker set at 25°C and 600 rpm.

Microsphere hybridization

Microsphere stock solutions (about 40000 microspheres/$\mu L$) are vortexed and sonicated in a Health Sonics ultrasonic cleaner (Model: T1.9C) for 60 seconds to suspend the microspheres. Suspended microspheres are diluted to 2000 microspheres per reaction in 1.5x TMAC hybridization solutions and mixed by vortexing and sonication. Thirty-three $\mu L$ per reaction of the bead mixture are transferred into a 96-well HybAid plate. Seven $\mu L$ of 15 nM biotinylated detection oligonucleotide stock in lx TE buffer are added to each reaction and mixed. Ten $\mu L$ of neutralized assay sample are added and the plate is sealed with a silicon cap mat seal. The plate is first incubated at 96°C for 5 minutes and incubated at 50°C without agitation overnight in a conventional hybridization oven. A filter plate (Dura pore, Millipore part number MSBVN1250, 1.2 $\mu m$ pore size) is prewetted with 75 $\mu L$ 1 x TMAC hybridization solution supplemented with 0.5% (w/v) BSA. The entire sample volume from the hybridization reaction is transferred to the filter plate. The hybridization plate is rinsed with 75 $\mu L$ 1 x TMAC hybridization solution containing 0.5% BSA and any remaining material is transferred to the filter plate. Samples are filtered under slow vacuum, with 150 buffer evacuated over about 8 seconds. The filter plate is washed once with 75 $\mu L$ 1 x TMAC hybridization solution containing 0.5% BSA and the microspheres in the filter plate are resuspended in 75 $\mu L$ 1 x TMAC hybridization solution containing 0.5% BSA. The filter plate is protected from light and incubated on an Eppendorf Thermalmixer R for 5 minutes at 1000 rpm. The filter plate is then washed once with 75 $\mu L$ 1 x TMAC hybridization solution containing 0.5% BSA. 75 $\mu L$ of 10 $\mu g$/mL streptavidin phycoerythrin (SAPE-100, MOSS, Inc.) in 1 x TMAC hybridization solution is added to each reaction and incubated on Eppendorf Thermalmixer R at 25°C at 1000 rpm for 60 minutes. The filter plate is washed twice with 75 $\mu L$ 1 x TMAC hybridization solution containing 0.5% BSA and the microspheres in the filter plate are resuspended in 75 $\mu L$ 1 x TMAC hybridization solution containing 0.5% BSA. The filter plate is then incubated protected from light on an Eppendorf Thermalmixer R for 5 minutes, 1000 rpm. The filter plate is then washed once with 75 $\mu L$ lx TMAC hybridization solution containing 0.5% BSA. Microspheres are resuspended in 75 l x TMAC hybridization solution supplemented with 0.5% BSA, and analyzed on a Luminex 100 instrument running XPonent 3.0 software. At least 100 microspheres are counted per bead type, under high PMT calibration and a doublet discriminator setting of 7500 to 18000. QPCR

read-out

Standard curves for qPCR are prepared in water ranging from 108 to 102 copies with 10-fold dilutions and a no-template control. Neutralized assay samples are diluted 40-fold into diH2O. The qPCR master mix is prepared at 2x final concentration (2x KOD buffer, 400 dNTP mix, 400 nM forward and reverse primer mix, 2x SYBR Green I and 0.5 U KOD EX). Ten $\mu L$ of 2x qPCR master mix is added to 10 $\mu L$ of diluted assay sample. qPCR is run on a BioRad MyIQ iCycler with 2 minutes at 96°C followed by 40 cycles of 96°C for 5 seconds and 72°C for 30 seconds.

**Example 2. HFrEF Model and Prediction of Cardiovascular Events**

In order to predict the risk or likelihood that an individual with HFrEF will have a CV event within one year, a model containing a panel of 16 biomarker proteins was developed. The CV event was defined as death. The training analysis was developed using the Bristol Myers Squibb (BMS) Penn Heart Failure Study (PHFS) data set. The PHFS includes 1,345 patients and 360 events. The Henry Ford Heart Failure (HFHF) study includes 620 patients and 222 events. The Atherosclerosis Risk in Communities (ARIC) study includes 44 patients and 25 events. ARIC visit 5 data and the Henry Ford data set were used for verification in refinement.
Table 3A shows stratification of the datasets for HFrEF model development (training and verification) and validation.

TABLE 3A

| Data Use (%) Dataset | Training | Verification | Hold-out Validation |
|---|---|---|---|
| PHFS | 80% | 0% | 20% |
| ARIC visit 5 | 0% | 100% | 0% |
| HFHF | 0% | 20% | 80% |

The HFrEF model is an accelerated failure time (AFT) survival model with a Weibull distribution. This model has 17 aptamers as its features. In total, the 17 aptamers bind 16 different biomarker proteins, which are listed in Table 1. The output of this model is the probability of survival at the specified time point, which is (1-p(all-cause death at the time point)). Thus, the output is a number between 0 and 1, with 0 being the lowest probability of survival (highest risk) and 1 being the highest probability of survival (lowest risk). The feature list was refined using several iterations of repeated Lasso AFT survival models, and the final model was trained using unpenalized AFT regression methods.

Overall Results

Each model fit is an accelerated failure time regression model with Weibull distribution, using a fit of a linear combination of the respective reagent values . The concordance index (C-index) was calculated by comparing the concordance of the predicted 1-year risk probabilities from each respective model to the time to death or censoring in the training data.
The C-index and area under the ROC curve (AUC) values for use of the model in predicting death within the specified time frame are given in Table 3B below.

Table 3B

| Data Set | C-index (95% CI) | AUC (95% CI) at 1 year | AUC (95% CI) at 180 days |
|---|---|---|---|
| Training | 0.754 (0.73, 0.78) | 0.790 (0.76, 0.83) | 0.783 (0.75, 0.83) |
| Verification | 0.747 (0.69, 0.83) | 0.847 (0.70, 0.97) | 0.773 (0.55, 0.95) |
| Validation | 0.75 (0.72, 0.776) | 0.762 (0.693, 0.829) | 0.781 (0.74, 0.824) |
| *C-index is not time specific, so the C-Index validation result will be the same for both the 6-month and 1-year timepoints. | | | |

Model Development

Table 4A shows the number of individuals who experience death by 6 months, 1 year, at any point in the study, and at no point in the study, broken down by training/verification/validation and by dataset.

**TABLE 4A**

| Study Stage | Dataset | Death at 180 days | Death at 365 days | Death by end of study | Death not observed |
|---|---|---|---|---|---|
| **Training** | | 82 | 138 | 649 | 428 |
| | **PHFS** | | | | |
| **Verification** | | 6 | 11 | 45 | 90 |
| | **HFHF** | | | | |
| | **ARIC** | 2 | 3 | 25 | 19 |
| | **Combined** | 8 | 14 | 70 | 99 |

(continued)

| Study Stage | Dataset | Death at 180 days | Death at 365 days | Death by end of study | Death not observed |
|---|---|---|---|---|---|
| Validation | PHFS | 21 | 33 | 71 | 196 |
| | HFHF | 24 | 39 | 177 | 318 |
| | Combined | 45 | 72 | 248 | 514 |

The demographics of the portion of the PHFS development cohort used in the training data set, the ARIC visit 5 data set and the Henry Ford data set used in the verification, and the PHFS and Henry Ford (HFHF) data set used in the validation are shown in Table 4B-4D below.

T/US2021/055537 **Table 4B: PHFS development cohort for training, verification and validation data sets**

| Data set | Covariate | Measure | Total | Death within 1 year | Survival at 1 year |
|---|---|---|---|---|---|
| HFrEF population of the PHFS training data set | Sample Size | | 1,077 | 649 (60.2%) | 428 (39.7%) |
| | Age | Mean (SD) | 55.74 (7.5) | 57.5 (13.5) | 53.0 (14.2) |
| | | Median | 57.52 | 59.0 | 54.2 |
| | | Range | 18.4-91.1 | 18.4-91.1 | 19.0-84.2 |
| | Sex | Male | 769 (71.4%) | 477 (73.4%) | 292 (68%) |
| | | Female | 308 (28.6%) | 172 (26.6%) | 136 (32%) |
| | Ethnicity | Caucasian | 725 (67.3%) | 425 (65.5%) | 300 (70%) |
| | | Black | 253 (23.5%) | 163 (25.1%) | 90 (21%) |
| | | Other | 99 (9.2%) | 61 (9.4%) | 38 (9%) |
| | Diabetes | Yes | 323 (29.9%) | 221 (34%) | 102 (24%) |
| | | No | 754 (70.1%) | 428 (66%) | 326 (76%) |
| | eGFR | Mean (SD) | 57.9 (24.0) | 54.18 (23.6) | 63.4 (23.5) |
| | | Median | 56.9 | 52.46 | 62.5 |
| | | Range | 4.3-184.7 | 6.23-184.7 | 4.3-143.7 |
| | BMI | Mean (SD) | 29.9 (6.96) | 29.7 (6.94) | 30.0 (7.0) |
| | | Median | 28.7 | 28.7 | 28.7 |
| | | Range | 16.3-63.5 | 16.3-63.5 | 17.0-61.8 |

(continued)

| Data set | Covariate | Measure | Total | Death within 1 year | Survival at 1 year |
|---|---|---|---|---|---|
| **ARIC** Visit 5, 20% of **HFHF** of the verification dataset | Sample Size | Number | 169 | 70 | 99 |
| | Death by 180 days | Number | 169 | 8 | 161 |
| | Death by 365 days | Number | 169 | 14 | 155 |
| | Age | Mean (SD) | 69.4 (10.4) | 71.9 (9.8) | 67.7 (10.5) |
| | | Median | 71 | 72 | 70 |
| | | Range | 43 - 92 | 43 - 92 | 45 - 87 |
| | Gender | Male | 122 (72%) | 52 (74%) | 70 (71%) |
| | | Female | 47 (28%) | 18 (26%) | 29 (29%) |
| | Ethnicity | Caucasian | 80 (47%) | 30 (43%) | 50 (51%) |
| | | Black | 84 (50%) | 38 (54%) | 46 (46%) |
| | | Other | 5 (3%) | 2 (3%) | 3 (4%) |
| | Diabetes | Yes | 81 (48%) | 38 (54%) | 43 (43%) |
| | | No | 88 (52%) | 32 (46%) | 56 (57%) |
| | BMI | Mean (SD) | 29.8 (6.7) | 28.8 (6.2) | 30.6 (6.9) |
| | | Median | 29.1 | 28.2 | 29.4 |
| | | Range | 16.6 - 63.6 | 16.6 - 54.9 | 18.4 - 63.6 |
| HFrEF population of the PHFS validation data set | Sample Size | Number | 267 | 71 (26.6%) | 196 (73.4%) |
| | Age | Mean (SD) | 57 (13) | 63.6 (12.2) | 54.6 (12.5) |
| | | Median | 58 | 63.7 | 56.4 |
| | | Range | (20.2, 83.4) | (20.8, 82.9) | (20.2, 83.4) |
| | Gender | Male | 184 (69%) | 51 (72%) | 133 (68%) |
| | | Female | 83 (31%) | 20 (28%) | 63 (32%) |
| | Ethnicity | Caucasian | 179 (67%) | 48 (68%) | 131 (67%) |
| | | Black | 60 (23%) | 17 (24%) | 43 (22%) |
| | | Other | 26 (10%) | 6 (8%) | 20 (10%) |
| | Diabetes | Yes | 85 (32%) | 29 (41%) | 56 (29%) |
| | | No | 182 (68%) | 42 (59%) | 140 (71%) |
| | eGFR | Mean (SD) | 57.9 (23.2) | 49.4 (22.1) | 61 (22.9) |
| | | Median | 58.2 | 46.9 | 62.7 |
| | | Range | 3.9 - 126.8 | 14.8 - 103.9 | 3.9 - 126.8 |
| | BMI | Mean (SD) | 30.3 (7.1) | 28.6 (5.7) | 30.9 (7.4) |
| | | Median | 29.1 | 27.6 | 29.9 |
| | | Range | 17.8-61.8 | 19.5-43.9 | 17.8-61.8 |
| HFrEF population of the HFHF validation data set | Sample Size | Number | 495 | 177 (36) | 318 (64%) |
| | Age | Mean (SD) | 67.33 (12.56) | 71.06 (12.64) | 65.23 (12.05) |
| | | Median | 67 | 74 | 65 |
| | | Range | (20, 96) | (29, 96) | (20, 91) |

(continued)

| Data set | Covariate | Measure | Total | Death within 1 year | Survival at 1 year |
|---|---|---|---|---|---|
| | Gender | Male | 326 (66%) | 119 (67%) | 207 (65%) |
| | | Female | 169 (34%) | 58 (33%) | 111 (35%) |
| | Ethnicity | Caucasian | 230 (46%) | 77 (44%) | 153 (48%) |
| | | Black | 248 (50%) | 94 (53%) | 154 (48%) |
| | | Other | 17 (4%) | 6 (3%) | 11 (4%) |
| | Diabetes | Yes | 191 (39%) | 66 (37%) | 125 (39%) |
| | | No | 304 (61%) | 111 (63%) | 193 (61%) |
| | BMI | Mean (SD) | 30.3 (7.5) | 29.4 (7.6) | 30.8 (7.3) |
| | | Median | 29.1 | 27.6 | 29.6 |
| | | Range | (16.2, 68.2) | (17, 57.8) | (16.2, 68.2) |

To ensure quality of the data, pre-processing steps were performed before the data were analyzed. The pre-processing steps included data quality control (QC) and pre-analytics.

Data QC on the PHFS data showed that 29 samples failed row-check, meaning at least one of the hybridization or three median scale factors were outside the 0.4 to 2.5 range, indicating technical issues (e.g., clogs) with that particular sample that would not be fixed by running the sample again. Additionally, there were 12 outlier samples with at least 5% of measurements more than 6 MADs from the median signal. These 41 samples in total (1.1%) were removed from further analyses. Finally, all analytes that did not pass target confirmation specificity testing were removed from the data set.

Data QC on the Henry Ford data showed that 24 samples failed row-check and were removed. This is 3.7% of the 644 HFrEF patients in that set. There were no outlier samples in this data set.

Data QC on the ARIC Visit 5 data showed no row-check failures or outliers, and so no samples were removed.

Pre-analytics did not show evidence of strong relationships between any of the clinical variables explored (age, sex, ethnicity, diabetes status, BMI, HFpEF/HFrEF status, event status, and eGFR) and the normalization scale factors in any of the datasets.

After data quality control and pre-analytics, model development was completed in two steps, 1) proof of concept (POC), and 2) refinement.

Only the training data was used in the POC step. The preliminary models explored were Cox with elastic net regularization, and AFT with elastic net regularization using Weibull and log-logistic distributions all using 10 repeats of 5-fold cross-validation. For POC analysis, sex and age were included in the development of the initial HFrEF only models for the composite endpoint.

Models developed in refinement used the PFHS, ARIC visit 5, and Henry Ford data sets. The PHFS data was split 80/20 training/validation, as in POC. All ARIC data (44 samples) was used for verification only. The Henry Ford data was split 20/80 verification/validation.

The final model is an AFT model with a Weibull distribution, and has 17 features, which are 17 aptamers. This model type was chosen because of its performance in POC.

The feature list was developed using several rounds of repeated Lasso, with lambda = 100 and alpha= 0.125, using AFT elastic net models with three repeats of five-fold cross validation. The model was initiated with the top 100 univariate features by rank from the POC analysis. After each round of model fitting, a threshold for coefficient size was identified by hand, and features with the smallest coefficients (in absolute value) were dropped from the feature list. This was repeated until the resulting model metrics (C-Index and AUC at one year (365 days)) began to drop in the verification set. The final list of 17 aptamers was then used to fit a standard AFT survival model with no penalization parameters.

POC Results

Initial model performance criteria were met for the HFrEF model with all-cause death as the endpoint, providing sufficient evidence to move this test into model refinement. Neither the all-comers model nor the HFrEF model for the composite endpoint passed initial performance criteria and were not recommended to move into refinement.

The POC results showed a number of analytes significant at different false-discovery rate (FDR) adjusted p-value levels for the Cox model.

Those numbers and percentages can be observed in Table 5 for the Heart Failure prognosis of all-cause death - reduced ejection fraction test.

Table 5

| FDR | # analytes (%) ≤ FDR level |
|---|---|
| 0.10 | 1853 (35.1%) |
| 0.05 | 1584 (30.0%) |
| 0.01 | 1228 (23.2%) |

The model that performed the best was a Cox model with elastic net regression penalty parameters, which achieved a C-Index of 0.751. The best AFT log-logistic model achieved a C-Index of 0.71. Both of these exceed the feasibility threshold of C-Index > 0.67. Because of the greater interpretability of the AFT model, this was the preferred method to use in refinement. The AUC at one year for this model was 0.782.

Adding age and sex to the model in POC did not improve model performance and were not included in any of the models in Refinement.

Refinement Results

The final model developed in refinement for the HFrEF population is a 17-aptamer AFT survival model using a Weibull distribution. The final model does not include regularization parameters (alpha and lambda).

A model with 31 features achieved slightly better metrics on the training and verification data but was not nearly as stable during model hardening, and so was rejected.

The model was trained on the 80% of PHFS data that was used for POC. The verification metrics were calculated on all of the ARIC visit 5 patients with HFrEF and 20% of the Henry Ford patients with HFrEF. The rest of the data (20% of PHFS and 80% of Henry Ford) were held out for validation. The C-index and AUC results for use of the model in predicting death or for predicting the composite endpoint at one year are shown in Table 3B above.

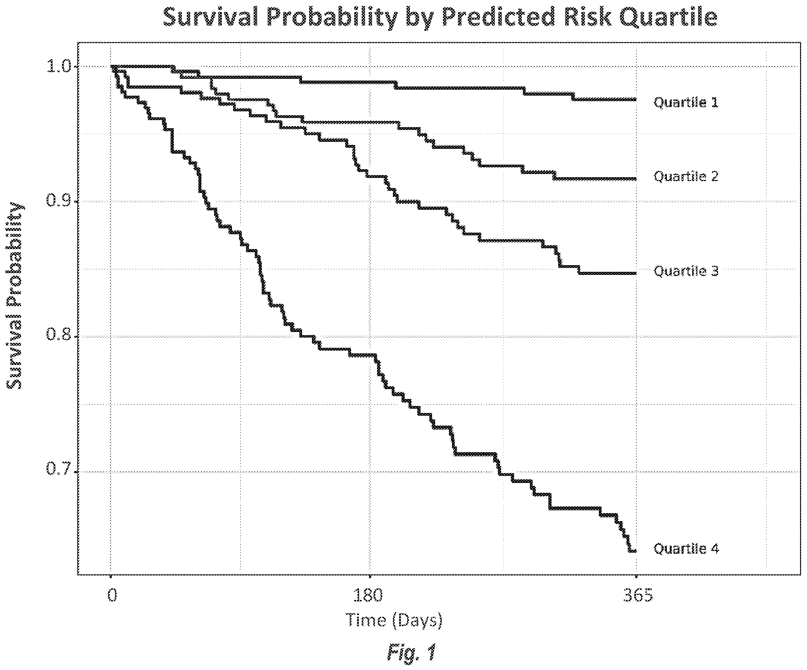

Figure 1 shows the observed Kaplan-Meier probability of the training dataset, with individuals split into quartiles by predicted event probability at 365 days. The lines are well separated at 180 and 365 days as expected for a well-performing model.

Validation

Validation of the model was assessed on the 20% of the PHFS and 80% of the Henry Ford data that were not used in the model development. The predictions are the survival probability at one year. Minimum values of 0.7 for the C-index and AUC are required in order to pass validation.

The AUC at 1 year (365 days) and 6 months (180 days), and the C-Index are shown in Table 6 for the training, verification, and validation sets. All validation metrics are higher than required to pass validation. (C-Index > 0.7 and AUC > 0.7 at one year and six months). There is a slight drop in all three metrics from training to validation data, as expected.

TABLE 6

| Data set | AUC (1 year) | AUC (95% CI) (1 year) | AUC (6 months) | AUC (95% CI) (6 months) | C-Index | C-Index (95% CI) |
|---|---|---|---|---|---|---|
| Training | 0.79 | (0.76, 0.83) | 0.783 | (0.747, 0.827) | 0.754 | 0.73 - 0.78 |
| Verification | 0.847 | (0.7, 0.97) | 0.773 | (0.55, 0.952) | 0.747 | 0.69 - 0.83 |
| Validation (entire dataset) | 0.781 | (0.74, 0.824) | 0.762 | (0.693, 0.829) | 0.75 | 0.72 - 0.776 |
| Validation (PHFS) | 0.710 | (0.614, 0.807) | 0.707 | (0.497, 0.812) | 0.714 | 0.657 - 0.771 |
| Validation (HFHF) | 0.834 | (0.768, 0.895) | 0.775 | (0.673, 0.866) | 0.755 | 0.711 - 0.793 |

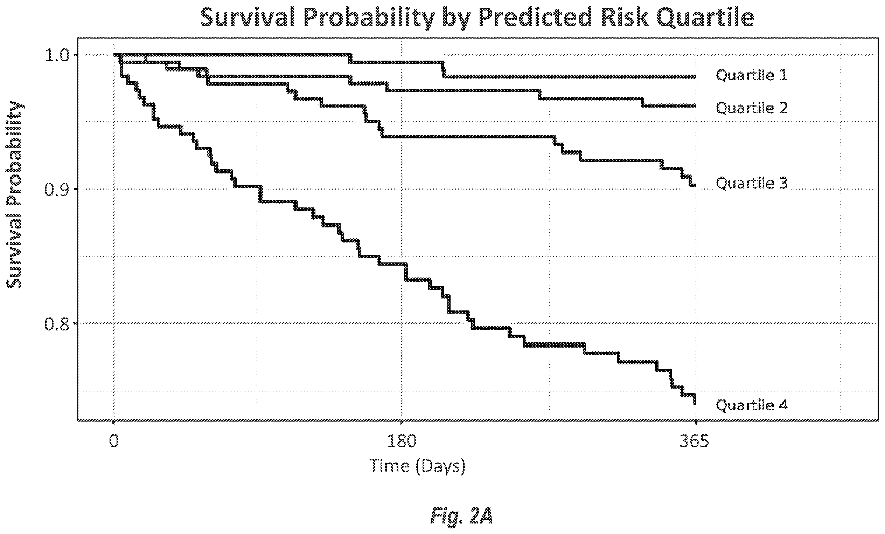

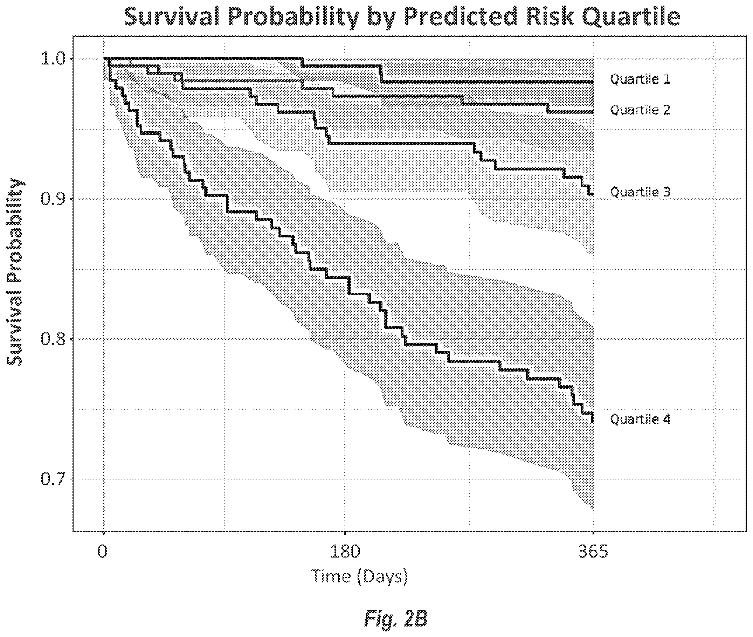

Figure 2A shows the observed Kaplan-Meier probability of the validation dataset, with individuals split into quartiles by predicted event probability at 365 days. The lines are well separated at 180 and 365 days, as we would expect to see for a model that is performing well. Furthermore, the lines do not cross after -45 days, which is another indicator of a model behaving as expected. Figure 2B shows the observed survival probability in validation data when stratified by predicted risk quartiles, with 95% confidence intervals for the different Kaplan-Meier curves. The survival probability cutoff for each quartile is Q4: p < 0.813 , Q3: p < 0.9; Q2 <0.942 , and Q1 is > 0.942.

The interference testing data were evaluated for putative interference using the final model. Albumin at 1000 and 2000 mg/dL, Hemoglobin at 500 and 1000 mg/dL, Cholesterol, and Valsartan failed the first step of interference testing at 365 days and 180 days, but none had a measurable effect on the C-index of the model. Out-of-range RFU values were imputed via winsorization during model development. Overall, the model hardening tools results, including interference testing and assay noise simulation, on the validation data were satisfactory meeting both C-Index and AUC metric goals at both 365 days and 180 days.

**Example 3: Analysis of HFrEF Biomarker Panel Model**

Model biomarker panels comprising various combinations of the biomarkers listed in Table 1 were analyzed to determine the C-index for all causes of death within one year of each panel. Tables 7 and 8 below show the model results when various combinations comprising 1 to 8 biomarker proteins were measured.

The results in Table 7 show that panels comprising at least two of CCL14, RNASE6, REG3A, and SVEP1, or at least two of CCL14, RNASE6, REG3A, and ADAMTSL2 performed adequately, with a C-index above 0.700.

The results in Table 8 also show that many panels comprising ADAMTSL2, CCL14, REG3A, RNASE6, or SVEP1 and comprising at least one of GDF15, THBS2, SVEP1, RNASE1, TAGLN, RSPO4, and WFDC2 performed adequately, with a C-index above 0.700. In panels that comprise SVEP1 twice, SVEP1 was measured using two different aptamers that bind SVEP1.

**Table 7: Performance of panels comprising proteins expressed from the indicated genes**

| CCL14 | RNASE6 | REG3A | SVEP1 | ADAMTSL2 | C-index |
|---|---|---|---|---|---|
| X | | X | X | X | 0.733 |
| X | X | X | | X | 0.733 |
| X | X | X | X | | 0.732 |
| | X | X | X | X | 0.732 |
| X | X | | X | X | 0.731 |
| X | | X | | X | 0.729 |
| X | X | | X | | 0.729 |
| | X | X | | X | 0.727 |
| | X | X | X | | 0.726 |
| | | X | X | X | 0.726 |
| X | | X | X | | 0.725 |
| X | | | X | X | 0.722 |
| X | X | | | X | 0.721 |
| | X | | X | X | 0.720 |
| X | X | X | | | 0.717 |
| | | X | | X | 0.720 |
| | X | | X | | 0.720 |
| X | | | X | | 0.718 |
| | | X | X | | 0.713 |
| X | X | | | | 0.711 |
| X | | | | X | 0.710 |
| X | | X | | | 0.708 |
| | X | | | X | 0.707 |

(continued)

| CCL14 | | RNASE6 | | REG3A | | SVEP1 | | ADAMTSL2 | | C-index |
|---|---|---|---|---|---|---|---|---|---|---|
| | | X | | X | | | | | | 0.707 |
| | | | | | | X | | X | | 0.694 |
| X | | | | | | | | | | 0.690 |
| | | X | | | | | | | | 0.690 |
| | | | | | | | | | | X **0.687** |
| | | | | | | | | | | X **0.680** |
| | | | | | | | | | | X **0.658** |

Table 8: Performance of panels comprising proteins expressed from the indicated genes

| | | | | | | | | |
|---|---|---|---|---|---|---|---|---|
| ADAMTSL2 | GDF15 | THBS2 | SVEP1 | RNASE1 | TAGLN | RSPO4 | WFDC2 | C-index |
| ADAMTSL2 | GDF15 | THBS2 | | | TAGLN | RSPO4 | | 0.741 |
| ADAMTSL2 | GDF15 | THBS2 | | | TAGLN | RSPO4 | WFDC2 | 0.741 |
| ADAMTSL2 | GDF15 | THBS2 | | RNASE1 | TAGLN | RSPO4 | WFDC2 | 0.741 |
| ADAMTSL2 | GDF15 | THBS2 | | RNASE1 | TAGLN | RSPO4 | | 0.741 |
| ADAMTSL2 | GDF15 | THBS2 | SVEP1 | | TAGLN | RSPO4 | | 0.741 |
| ADAMTSL2 | GDF15 | THBS2 | SVEP1 | | TAGLN | RSPO4 | WFDC2 | 0.741 |
| ADAMTSL2 | GDF15 | | | | TAGLN | RSPO4 | | 0.740 |
| ADAMTSL2 | GDF15 | THBS2 | SVEP1 | RNASE1 | TAGLN | RSPO4 | | 0.740 |
| ADAMTSL2 | GDF 15 | THBS2 | SVEP1 | RNASE1 | TAGLN | RSPO4 | WFDC2 | 0.740 |
| ADAMTSL2 | GDF15 | THBS2 | | RNASE1 | | RSPO4 | | 0.740 |
| ADAMTSL2 | GDF15 | THBS2 | | RNASE1 | | RSPO4 | WFDC2 | 0.740 |
| ADAMTSL2 | GDF15 | | | RNASE1 | TAGLN | RSPO4 | | 0.740 |
| ADAMTSL2 | GDF15 | | | RNASE1 | TAGLN | RSPO4 | WFDC2 | 0.740 |
| ADAMTSL2 | GDF15 | | | | TAGLN | RSPO4 | WFDC2 | 0.740 |
| ADAMTSL2 | GDF15 | THBS2 | SVEP1 | RNASE1 | | RSPO4 | | 0.740 |
| ADAMTSL2 | GDF15 | | SVEP1 | | TAGLN | RSPO4 | | 0.740 |
| ADAMTSL2 | GDF15 | THBS2 | SVEP1 | RNASE1 | | RSPO4 | WFDC2 | 0.740 |
| ADAMTSL2 | GDF15 | | SVEP1 | RNASE1 | TAGLN | RSPO4 | | 0.740 |
| ADAMTSL2 | GDF15 | | SVEP1 | RNASE1 | TAGLN | RSPO4 | WFDC2 | 0.740 |
| ADAMTSL2 | GDF15 | | SVEP1 | | TAGLN | RSPO4 | WFDC2 | 0.739 |
| ADAMTSL2 | GDF15 | | | RNASE1 | | RSPO4 | | 0.739 |
| ADAMTSL2 | GDF15 | | | RNASE1 | | RSPO4 | WFDC2 | 0.739 |
| ADAMTSL2 | GDF15 | | SVEP1 | RNASE1 | | RSPO4 | | 0.739 |
| ADAMTSL2 | GDF15 | | SVEP1 | RNASE1 | | RSPO4 | WFDC2 | 0.739 |
| ADAMTSL2 | GDF15 | THBS2 | | | | RSPO4 | WFDC2 | 0.739 |
| ADAMTSL2 | GDF15 | THBS2 | | | | RSPO4 | | 0.739 |
| ADAMTSL2 | GDF15 | THBS2 | SVEP1 | | TAGLN | | | 0.738 |
| ADAMTSL2 | GDF15 | THBS2 | SVEP1 | RNASE1 | TAGLN | | | 0.738 |
| ADAMTSL2 | GDF15 | THBS2 | SVEP1 | | | RSPO4 | WFDC2 | 0.738 |

(continued)

| ADAMTSL2 | GDF15 | THBS2 | SVEP1 | RNASE1 | TAGLN | RSPO4 | WFDC2 | C-index |
|---|---|---|---|---|---|---|---|---|
| ADAMTSL2 | GDF15 | THBS2 | | | TAGLN | | | 0.738 |
| ADAMTSL2 | GDF15 | | SVEP1 | | TAGLN | | | 0.738 |
| ADAMTSL2 | GDF15 | THBS2 | SVEP1 | | | RSPO4 | | 0.738 |
| ADAMTSL2 | GDF15 | THBS2 | | RNASE1 | TAGLN | | | 0.738 |
| ADAMTSL2 | GDF15 | THBS2 | | | TAGLN | | WFDC2 | 0.738 |
| ADAMTSL2 | GDF15 | THBS2 | SVEP1 | RNASE1 | TAGLN | | WFDC2 | 0.738 |
| ADAMTSL2 | GDF15 | THBS2 | SVEP1 | | TAGLN | | WFDC2 | 0.738 |
| ADAMTSL2 | GDF15 | THBS2 | | RNASE1 | TAGLN | | WFDC2 | 0.738 |
| ADAMTSL2 | GDF15 | | SVEP1 | | TAGLN | | WFDC2 | 0.738 |
| ADAMTSL2 | GDF15 | | SVEP1 | RNASE1 | TAGLN | | | 0.738 |
| ADAMTSL2 | GDF15 | | | | TAGLN | | | 0.737 |
| ADAMTSL2 | GDF15 | | SVEP1 | RNASE1 | TAGLN | | WFDC2 | 0.737 |
| ADAMTSL2 | GDF15 | | | RNASE1 | TAGLN | | | 0.737 |
| ADAMTSL2 | GDF15 | | | | | RSPO4 | | 0.737 |
| ADAMTSL2 | GDF15 | | | RNASE1 | TAGLN | | WFDC2 | 0.737 |
| ADAMTSL2 | GDF15 | | | | TAGLN | | WFDC2 | 0.737 |
| ADAMTSL2 | GDF15 | | | | | RSPO4 | WFDC2 | 0.737 |
| ADAMTSL2 | GDF15 | | SVEP1 | | | RSPO4 | WFDC2 | 0.737 |
| ADAMTSL2 | GDF15 | | SVEP1 | | | RSPO4 | | 0.737 |
| ADAMTSL2 | GDF15 | THBS2 | SVEP1 | RNASE1 | | | WFDC2 | 0.736 |
| ADAMTSL2 | GDF15 | THBS2 | SVEP1 | RNASE1 | | | | 0.736 |
| ADAMTSL2 | GDF15 | | SVEP1 | RNASE1 | | | WFDC2 | 0.735 |
| ADAMTSL2 | GDF15 | | SVEP1 | RNASE1 | | | | 0.735 |
| ADAMTSL2 | | THBS2 | SVEP1 | RNASE1 | TAGLN | RSPO4 | | 0.735 |
| ADAMTSL2 | | THBS2 | | RNASE1 | TAGLN | RSPO4 | | 0.735 |
| ADAMTSL2 | GDF15 | THBS2 | | RNASE1 | | | | 0.735 |
| ADAMTSL2 | GDF15 | THBS2 | | RNASE1 | | | WFDC2 | 0.735 |
| ADAMTSL2 | | THBS2 | | RNASE1 | TAGLN | RSPO4 | WFDC2 | 0.734 |
| ADAMTSL2 | GDF15 | THBS2 | SVEP1 | | | | WFDC2 | 0.734 |
| ADAMTSL2 | GDF15 | THBS2 | SVEP1 | | | | | 0.734 |
| ADAMTSL2 | | THBS2 | | | TAGLN | RSPO4 | WFDC2 | 0.734 |
| ADAMTSL2 | GDF15 | THBS2 | | | | | WFDC2 | 0.734 |
| ADAMTSL2 | | THBS2 | SVEP1 | RNASE1 | TAGLN | RSPO4 | WFDC2 | 0.734 |
| ADAMTSL2 | GDF15 | | SVEP1 | | | | WFDC2 | 0.734 |
| ADAMTSL2 | GDF15 | THBS2 | | | | | | 0.734 |
| ADAMTSL2 | GDF15 | | SVEP1 | | | | | 0.734 |
| ADAMTSL2 | | THBS2 | | | TAGLN | RSPO4 | | 0.733 |
| ADAMTSL2 | | THBS2 | SVEP1 | | TAGLN | RSPO4 | WFDC2 | 0.733 |
| ADAMTSL2 | | THBS2 | SVEP1 | RNASE1 | TAGLN | | | 0.733 |

(continued)

| ADAMTSL2 | GDF15 | THBS2 | SVEP1 | RNASE1 | TAGLN | RSPO4 | WFDC2 | C-index |
|---|---|---|---|---|---|---|---|---|
| ADAMTSL2 | GDF15 | | | RNASE1 | | | | 0.733 |
| ADAMTSL2 | GDF15 | | | RNASE1 | | | WFDC2 | 0.733 |
| ADAMTSL2 | | THBS2 | | RNASE1 | | RSPO4 | WFDC2 | 0.733 |
| ADAMTSL2 | | THBS2 | SVEP1 | | TAGLN | RSPO4 | | 0.733 |
| ADAMTSL2 | | THBS2 | SVEP1 | RNASE1 | | RSPO4 | WFDC2 | 0.732 |
| ADAMTSL2 | | | SVEP1 | RNASE1 | TAGLN | RSPO4 | | 0.732 |
| ADAMTSL2 | | THBS2 | SVEP1 | | TAGLN | | WFDC2 | 0.732 |
| ADAMTSL2 | GDF15 | | | | | | WFDC2 | 0.732 |
| ADAMTSL2 | | THBS2 | | RNASE1 | TAGLN | | | 0.732 |
| ADAMTSL2 | | THBS2 | | | TAGLN | | WFDC2 | 0.732 |
| ADAMTSL2 | | THBS2 | SVEP1 | RNASE1 | TAGLN | | WFDC2 | 0.732 |
| ADAMTSL2 | | | SVEP1 | RNASE1 | TAGLN | RSPO4 | WFDC2 | 0.732 |
| ADAMTSL2 | | THBS2 | | RNASE1 | TAGLN | | WFDC2 | 0.732 |
| ADAMTSL2 | GDF15 | | | | | | | 0.732 |
| ADAMTSL2 | | | | RNASE1 | TAGLN | RSPO4 | | 0.731 |
| ADAMTSL2 | | | | | TAGLN | RSPO4 | WFDC2 | 0.731 |
| ADAMTSL2 | | | | RNASE1 | TAGLN | RSPO4 | WFDC2 | 0.731 |
| ADAMTSL2 | | | SVEP1 | | TAGLN | RSPO4 | WFDC2 | 0.731 |
| ADAMTSL2 | | THBS2 | SVEP1 | RNASE1 | | RSPO4 | | 0.731 |
| ADAMTSL2 | | THBS2 | SVEP1 | | TAGLN | | | 0.731 |
| ADAMTSL2 | | | SVEP1 | RNASE1 | TAGLN | | | 0.731 |
| ADAMTSL2 | | THBS2 | | RNASE1 | | RSPO4 | | 0.731 |
| ADAMTSL2 | | THBS2 | | | TAGLN | | | 0.731 |
| ADAMTSL2 | | THBS2 | | | | RSPO4 | WFDC2 | 0.730 |
| ADAMTSL2 | | | SVEP1 | RNASE1 | | RSPO4 | WFDC2 | 0.730 |
| ADAMTSL2 | | | SVEP1 | RNASE1 | TAGLN | | WFDC2 | 0.730 |
| ADAMTSL2 | | THBS2 | SVEP1 | | | RSPO4 | WFDC2 | 0.730 |
| ADAMTSL2 | | | SVEP1 | | TAGLN | | WFDC2 | 0.730 |
| ADAMTSL2 | | | | | TAGLN | RSPO4 | | 0.729 |
| ADAMTSL2 | | | SVEP1 | | TAGLN | RSPO4 | | 0.729 |
| ADAMTSL2 | | | | RNASE1 | TAGLN | | | 0.729 |
| ADAMTSL2 | | | SVEP1 | RNASE1 | | RSPO4 | | 0.729 |
| ADAMTSL2 | | | SVEP1 | | TAGLN | | | 0.729 |
| ADAMTSL2 | | | | | TAGLN | | WFDC2 | 0.728 |
| ADAMTSL2 | | | | RNASE1 | TAGLN | | WFDC2 | 0.728 |
| ADAMTSL2 | | | | RNASE1 | | RSPO4 | WFDC2 | 0.728 |
| ADAMTSL2 | | THBS2 | SVEP1 | RNASE1 | | | WFDC2 | 0.728 |
| ADAMTSL2 | | | | | TAGLN | | | 0.727 |
| ADAMTSL2 | | THBS2 | SVEP1 | RNASE1 | | | | 0.727 |

(continued)

| ADAMTSL2 | GDF15 | THBS2 | SVEP1 | RNASE1 | TAGLN | RSPO4 | WFDC2 | C-index |
|---|---|---|---|---|---|---|---|---|
| ADAMTSL2 | | | SVEP1 | | | RSPO4 | WFDC2 | 0.726 |
| ADAMTSL2 | | THBS2 | SVEP1 | | | | WFDC2 | 0.726 |
| ADAMTSL2 | | THBS2 | | RNASE1 | | | WFDC2 | 0.725 |
| ADAMTSL2 | | | SVEP1 | RNASE1 | | | WFDC2 | 0.725 |
| ADAMTSL2 | | | | | | RSPO4 | WFDC2 | 0.725 |
| ADAMTSL2 | | | | RNASE1 | | RSPO4 | | 0.725 |
| ADAMTSL2 | | THBS2 | | | | | WFDC2 | 0.724 |
| ADAMTSL2 | | | SVEP1 | RNASE1 | | | | 0.724 |
| ADAMTSL2 | | | SVEP1 | | | | WFDC2 | 0.723 |
| ADAMTSL2 | | THBS2 | | RNASE1 | | | | 0.722 |
| ADAMTSL2 | | | | RNASE1 | | | WFDC2 | 0.719 |
| ADAMTSL2 | | | | | | | WFDC2 | 0.717 |
| ADAMTSL2 | | THBS2 | SVEP1 | | | RSPO4 | | 0.712 |
| ADAMTSL2 | | | | RNASE1 | | | | 0.712 |
| ADAMTSL2 | | THBS2 | | | | RSPO4 | | 0.709 |
| ADAMTSL2 | | THBS2 | SVEP1 | | | | | 0.705 |
| ADAMTSL2 | | | S VEP1 | | | RSPO4 | | 0.701 |
| ADAMTSL2 | | THBS2 | | | | | | 0.694 |
| ADAMTSL2 | | | SVEP1 | | | | | 0.694 |
| ADAMTSL2 | | | | | | RSPO4 | | 0.688 |
| ADAMTSL2 | | | | | | | | 0.658 |
| **CCL14** | **GDF15** | **THBS2** | **SVEP1** | **RNASE1** | **TAGLN** | **RSPO4** | **WFDC2** | **C-index** |
| CCL14 | GDF15 | THBS2 | | RNASE1 | | RSPO4 | | 0.746 |
| CCL14 | GDF15 | THBS2 | SVEP1 | | | RSPO4 | | 0.746 |
| CCL14 | GDF15 | THBS2 | | | | RSPO4 | | 0.746 |
| CCL14 | GDF15 | THBS2 | SVEP1 | RNASE1 | | RSPO4 | | 0.746 |
| CCL14 | GDF15 | THBS2 | SVEP1 | RNASE1 | | RSPO4 | WFDC2 | 0.746 |
| CCL14 | GDF15 | THBS2 | | RNASE1 | | RSPO4 | WFDC2 | 0.746 |
| CCL14 | GDF15 | THBS2 | | | | RSPO4 | WFDC2 | 0.746 |
| CCL14 | GDF15 | THBS2 | SVEP1 | | | RSPO4 | WFDC2 | 0.746 |
| CCL14 | GDF15 | THBS2 | SVEP1 | | TAGLN | RSPO4 | | 0.745 |
| CCL14 | GDF15 | THBS2 | SVEP1 | | TAGLN | RSPO4 | WFDC2 | 0.745 |
| CCL14 | GDF15 | THBS2 | | | TAGLN | RSPO4 | WFDC2 | 0.745 |
| CCL14 | GDF15 | THBS2 | | | TAGLN | RSPO4 | | 0.745 |
| CCL14 | GDF15 | THBS2 | | RNASE1 | TAGLN | RSPO4 | WFDC2 | 0.744 |
| CCL14 | GDF15 | THBS2 | SVEP1 | RNASE1 | TAGLN | RSPO4 | WFDC2 | 0.744 |
| CCL14 | GDF15 | THBS2 | | RNASE1 | TAGLN | RSPO4 | | 0.744 |
| CCL14 | GDF15 | THBS2 | SVEP1 | RNASE1 | TAGLN | RSPO4 | | 0.744 |

(continued)

| | | | | | | | | |
|---|---|---|---|---|---|---|---|---|
| ADAMTSL2 | | | | | | | | 0.658 |
| CCL14 | GDF15 | | SVEP1 | RNASE1 | | RSPO4 | WFDC2 | 0.743 |
| CCL14 | GDF15 | | SVEP1 | | | RSPO4 | WFDC2 | 0.743 |
| CCL14 | GDF15 | | SVEP1 | RNASE1 | | RSPO4 | | 0.743 |
| CCL14 | GDF15 | | SVEP1 | | | RSPO4 | | 0.743 |
| CCL14 | GDF15 | THBS2 | SVEP1 | | | | | 0.742 |
| CCL14 | GDF15 | THBS2 | SVEP1 | | | | WFDC2 | 0.742 |
| CCL14 | GDF15 | THBS2 | SVEP1 | RNASE1 | | | WFDC2 | 0.742 |
| CCL14 | GDF15 | THBS2 | SVEP1 | RNASE1 | | | | 0.742 |
| CCL14 | GDF15 | | SVEP1 | | TAGLN | RSPO4 | WFDC2 | 0.742 |
| CCL14 | GDF15 | | SVEP1 | | TAGLN | RSPO4 | | 0.742 |
| CCL14 | GDF15 | THBS2 | SVEP1 | | TAGLN | | | 0.742 |
| CCL14 | GDF15 | THBS2 | SVEP1 | | TAGLN | | WFDC2 | 0.741 |
| CCL14 | GDF15 | | | RNASE1 | | RSPO4 | WFDC2 | 0.741 |
| CCL14 | GDF15 | | SVEP1 | RNASE1 | TAGLN | RSPO4 | WFDC2 | 0.741 |
| CCL14 | GDF15 | THBS2 | SVEP1 | RNASE1 | TAGLN | | WFDC2 | 0.741 |
| CCL14 | GDF15 | | | | | RSPO4 | WFDC2 | 0.741 |
| CCL14 | GDF15 | THBS2 | SVEP1 | RNASE1 | TAGLN | | | 0.741 |
| CCL14 | GDF15 | THBS2 | | RNASE1 | TAGLN | | WFDC2 | 0.741 |
| CCL14 | GDF15 | THBS2 | | RNASE1 | TAGLN | | | 0.741 |
| CCL14 | GDF15 | THBS2 | | RNASE1 | | | WFDC2 | 0.741 |
| CCL14 | GDF15 | THBS2 | | RNASE1 | | | | 0.741 |
| CCL14 | GDF15 | | | | | RSPO4 | | 0.741 |
| CCL14 | GDF15 | THBS2 | | | | | | 0.741 |
| CCL14 | GDF15 | THBS2 | | | | | WFDC2 | 0.741 |
| CCL14 | GDF15 | THBS2 | | | TAGLN | | WFDC2 | 0.741 |
| CCL14 | GDF15 | | SVEP1 | RNASE1 | | | WFDC2 | 0.741 |
| CCL14 | GDF15 | THBS2 | | | TAGLN | | | 0.741 |
| CCL14 | GDF15 | | | RNASE1 | | RSPO4 | | 0.741 |
| CCL14 | GDF15 | | SVEP1 | RNASE1 | TAGLN | RSPO4 | | 0.741 |
| CCL14 | GDF15 | | | | TAGLN | RSPO4 | WFDC2 | 0.741 |
| CCL14 | GDF15 | | SVEP1 | | | | WFDC2 | 0.741 |
| CCL14 | GDF15 | | | RNASE1 | TAGLN | RSPO4 | WFDC2 | 0.740 |
| CCL14 | GDF15 | | SVEP1 | | | | | 0.740 |
| CCL14 | GDF15 | | SVEP1 | RNASE1 | | | | 0.740 |
| CCL14 | GDF15 | | | | TAGLN | RSPO4 | | 0.740 |
| CCL14 | GDF15 | | SVEP1 | | TAGLN | | WFDC2 | 0.740 |
| CCL14 | GDF15 | | SVEP1 | | TAGLN | | | 0.740 |
| CCL14 | GDF15 | | | RNASE1 | TAGLN | RSPO4 | | 0.740 |
| CCL14 | GDF15 | | SVEP1 | RNASE1 | TAGLN | | WFDC2 | 0.740 |

(continued)

| | | | | | | | | |
|---|---|---|---|---|---|---|---|---|
| ADAMTSL2 | | | | | | | | 0.658 |
| CCL14 | GDF15 | | SVEP1 | RNASE1 | TAGLN | | | 0.739 |
| CCL14 | | THBS2 | | RNASE1 | TAGLN | RSPO4 | | 0.739 |
| CCL14 | | THBS2 | SVEP1 | RNASE1 | TAGLN | RSPO4 | | 0.739 |
| CCL14 | GDF15 | | | RNASE1 | TAGLN | | WFDC2 | 0.739 |
| CCL14 | | THBS2 | SVEP1 | RNASE1 | | RSPO4 | WFDC2 | 0.738 |
| CCL14 | GDF15 | | | | TAGLN | | WFDC2 | 0.738 |
| CCL14 | | THBS2 | | | TAGLN | RSPO4 | | 0.738 |
| CCL14 | | THBS2 | | RNASE1 | | RSPO4 | WFDC2 | 0.738 |
| CCL14 | | THBS2 | SVEP1 | | TAGLN | RSPO4 | | 0.738 |
| CCL14 | | THBS2 | SVEP1 | RNASE1 | TAGLN | RSPO4 | WFDC2 | 0.738 |
| CCL14 | | THBS2 | | | TAGLN | RSPO4 | WFDC2 | 0.738 |
| CCL14 | | THBS2 | SVEP1 | | TAGLN | RSPO4 | WFDC2 | 0.738 |
| CCL14 | | THBS2 | SVEP1 | RNASE1 | TAGLN | | | 0.738 |
| CCL14 | | THBS2 | SVEP1 | RNASE1 | | RSPO4 | | 0.738 |
| CCL14 | | THBS2 | | RNASE1 | TAGLN | RSPO4 | WFDC2 | 0.738 |
| CCL14 | | THBS2 | SVEP1 | | | RSPO4 | WFDC2 | 0.738 |
| CCL14 | | THBS2 | | | | RSPO4 | WFDC2 | 0.738 |
| CCL14 | GDF15 | | | RNASE1 | TAGLN | | | 0.738 |
| CCL14 | | THBS2 | SVEP1 | | TAGLN | | | 0.738 |
| CCL14 | GDF15 | | | RNASE1 | | | WFDC2 | 0.737 |
| CCL14 | GDF15 | | | | | | WFDC2 | 0.737 |
| CCL14 | | THBS2 | | RNASE1 | | RSPO4 | | 0.737 |
| CCL14 | | THBS2 | | | TAGLN | | | 0.737 |
| CCL14 | | THBS2 | | RNASE1 | TAGLN | | | 0.737 |
| CCL14 | GDF15 | | | RNASE1 | | | | 0.737 |
| CCL14 | GDF15 | | | | | | | 0.737 |
| CCL14 | GDF15 | | | | TAGLN | | | 0.737 |
| CCL14 | | THBS2 | SVEP1 | RNASE1 | TAGLN | | WFDC2 | 0.736 |
| CCL14 | | THBS2 | SVEP1 | | TAGLN | | WFDC2 | 0.736 |
| CCL14 | | THBS2 | | RNASE1 | TAGLN | | WFDC2 | 0.736 |
| CCL14 | | THBS2 | | | TAGLN | | WFDC2 | 0.736 |
| CCL14 | | THBS2 | SVEP1 | | | | WFDC2 | 0.736 |
| CCL14 | | THBS2 | SVEP1 | RNASE1 | | | WFDC2 | 0.735 |
| CCL14 | | THBS2 | SVEP1 | RNASE1 | | | | 0.735 |
| CCL14 | | THBS2 | | RNASE1 | | | WFDC2 | 0.733 |
| CCL14 | | THBS2 | | | | | WFDC2 | 0.733 |
| CCL14 | | | SVEP1 | RNASE1 | | RSPO4 | WFDC2 | 0.733 |
| CCL14 | | | SVEP1 | RNASE1 | TAGLN | RSPO4 | | 0.732 |
| CCL14 | | | SVEP1 | RNASE1 | | RSPO4 | | 0.732 |

(continued)

| | | | | | | | | |
|---|---|---|---|---|---|---|---|---|
| ADAMTSL2 | | | | | | | | 0.658 |
| CCL14 | | | SVEP1 | RNASE1 | TAGLN | | | 0.731 |
| CCL14 | | | SVEP1 | RNASE1 | TAGLN | RSPO4 | WFDC2 | 0.731 |
| CCL14 | | | SVEP1 | | | RSPO4 | WFDC2 | 0.731 |
| CCL14 | | THBS2 | | RNASE1 | | | | 0.731 |
| CCL14 | | | SVEP1 | | TAGLN | RSPO4 | WFDC2 | 0.730 |
| CCL14 | | THBS2 | SVEP1 | | | RSPO4 | | 0.730 |
| CCL14 | | | SVEP1 | RNASE1 | | | WFDC2 | 0.730 |
| CCL14 | | | SVEP1 | RNASE1 | TAGLN | | WFDC2 | 0.729 |
| CCL14 | | | SVEP1 | | TAGLN | | WFDC2 | 0.729 |
| CCL14 | | | SVEP1 | RNASE1 | | | | 0.729 |
| CCL14 | | | SVEP1 | | TAGLN | RSPO4 | | 0.729 |
| CCL14 | | THBS2 | | | | RSPO4 | | 0.729 |
| CCL14 | | | SVEP1 | | | | WFDC2 | 0.728 |
| CCL14 | | | S VEP1 | | TAGLN | | | 0.728 |
| CCL14 | | THBS2 | SVEP1 | | | | | 0.727 |
| CCL14 | | | | RNASE1 | TAGLN | RSPO4 | | 0.726 |
| CCL14 | | | | RNASE1 | TAGLN | RSPO4 | WFDC2 | 0.726 |
| CCL14 | | | | | TAGLN | RSPO4 | WFDC2 | 0.725 |
| CCL14 | | | | RNASE1 | | RSPO4 | WFDC2 | 0.725 |
| CCL14 | | | | RNASE1 | TAGLN | | | 0.725 |
| CCL14 | | | | | | RSPO4 | WFDC2 | 0.724 |
| CCL14 | | | | RNASE1 | | RSPO4 | | 0.723 |
| CCL14 | | | | | TAGLN | | WFDC2 | 0.723 |
| CCL14 | | | | | TAGLN | RSPO4 | | 0.723 |
| CCL14 | | THBS2 | | | | | | 0.723 |
| CCL14 | | | | | TAGLN | | | 0.722 |
| CCL14 | | | | RNASE1 | TAGLN | | WFDC2 | 0.721 |
| CCL14 | | | SVEP1 | | | RSPO4 | | 0.721 |
| CCL14 | | | SVEP1 | | | | | 0.717 |
| CCL14 | | | | | | | WFDC2 | 0.714 |
| CCL14 | | | | RNASE1 | | | WFDC2 | 0.714 |
| CCL14 | | | | RNASE1 | | | | 0.713 |
| CCL14 | | | | | | RSPO4 | | 0.709 |
| CCL14 | | | | | | | | 0.690 |
| REG3A | GDF15 | THBS2 | SVEP1 | RNASE1 | TAGLN | RSPO4 | WEDC2 | C-index |
| REG3A | GDF 15 | THBS2 | | RNASE1 | | RSPO4 | WFDC2 | 0.743 |
| REG3A | GDF 15 | THBS2 | SVEP1 | RNASE1 | | RSPO4 | WFDC2 | 0.743 |
| REG3A | GDF15 | THBS2 | | RNASE1 | | RSPO4 | | 0.743 |
| REG3A | GDF15 | THBS2 | SVEP1 | RNASE1 | | RSPO4 | | 0.743 |

40

(continued)

| | | | | | | | | |
|---|---|---|---|---|---|---|---|---|
| ADAMTSL2 | | | | | | | | 0.658 |
| REG3A | GDF15 | THBS2 | | | | RSPO4 | WFDC2 | 0.742 |
| REG3A | GDF15 | THBS2 | SVEP1 | | | RSPO4 | WFDC2 | 0.742 |
| REG3A | GDF15 | THBS2 | SVEP1 | | | RSPO4 | | 0.742 |
| REG3A | GDF15 | THBS2 | SVEP1 | RNASE1 | TAGLN | RSPO4 | WFDC2 | 0.742 |
| REG3A | GDF15 | THBS2 | | | | RSPO4 | | 0.742 |
| REG3A | GDF15 | THBS2 | | RNASE1 | TAGLN | RSPO4 | WFDC2 | 0.742 |
| REG3A | GDF15 | THBS2 | SVEP1 | | TAGLN | RSPO4 | WFDC2 | 0.742 |
| REG3A | GDF15 | THBS2 | SVEP1 | | TAGLN | RSPO4 | | 0.742 |
| REG3A | GDF15 | THBS2 | | | TAGLN | RSPO4 | WFDC2 | 0.742 |
| REG3A | GDF15 | THBS2 | | | TAGLN | RSPO4 | | 0.742 |
| REG3A | GDF15 | THBS2 | SVEP1 | RNASE1 | TAGLN | RSPO4 | | 0.742 |
| REG3A | GDF15 | THBS2 | | RNASE1 | TAGLN | RSPO4 | | 0.742 |
| REG3A | GDF15 | THBS2 | SVEP1 | RNASE1 | | | WFDC2 | 0.740 |
| REG3A | GDF15 | THBS2 | SVEP1 | RNASE1 | | | | 0.740 |
| REG3A | GDF15 | THBS2 | SVEP1 | | | | | 0.740 |
| REG3A | GDF15 | THBS2 | SVEP1 | | | | WFDC2 | 0.740 |
| REG3A | GDF15 | THBS2 | SVEP1 | | TAGLN | | | 0.739 |
| REG3A | GDF15 | THBS2 | | RNASE1 | TAGLN | | WFDC2 | 0.739 |
| REG3A | GDF15 | THBS2 | | | TAGLN | | WFDC2 | 0.739 |
| REG3A | GDF15 | THBS2 | SVEP1 | | TAGLN | | WFDC2 | 0.739 |
| REG3A | GDF15 | THBS2 | SVEP1 | RNASE1 | TAGLN | | WFDC2 | 0.739 |
| REG3A | GDF15 | THBS2 | | RNASE1 | | | WFDC2 | 0.739 |
| REG3A | GDF15 | THBS2 | | | TAGLN | | | 0.739 |
| REG3A | GDF15 | THBS2 | SVEP1 | RNASE1 | TAGLN | | | 0.739 |
| REG3A | | THBS2 | | RNASE1 | TAGLN | RSPO4 | | 0.739 |
| REG3A | GDF15 | THBS2 | | | | | WFDC2 | 0.739 |
| REG3A | GDF15 | THBS2 | | RNASE1 | TAGLN | | | 0.739 |
| REG3A | GDF15 | THBS2 | | | | | | 0.739 |
| REG3A | GDF15 | THBS2 | | RNASE1 | | | | 0.739 |
| REG3A | | THBS2 | | | TAGLN | RSPO4 | | 0.739 |
| REG3A | GDF15 | | SVEP1 | RNASE1 | | RSPO4 | WFDC2 | 0.739 |
| REG3A | | THBS2 | SVEP1 | RNASE1 | TAGLN | RSPO4 | | 0.739 |
| REG3A | | THBS2 | SVEP1 | | TAGLN | RSPO4 | | 0.739 |
| REG3A | | THBS2 | | RNASE1 | TAGLN | | | 0.739 |
| REG3A | | THBS2 | | | TAGLN | | | 0.739 |
| REG3A | | THBS2 | SVEP1 | RNASE1 | | RSPO4 | | 0.738 |
| REG3A | | THBS2 | | RNASE1 | | RSPO4 | | 0.738 |
| REG3A | | THBS2 | SVEP1 | RNASE1 | | RSPO4 | WFDC2 | 0.738 |
| REG3A | | THBS2 | SVEP1 | RNASE1 | TAGLN | | | 0.738 |

(continued)

| | | | | | | | | |
|---|---|---|---|---|---|---|---|---|
| ADAMTSL2 | | | | | | | | 0.658 |
| REG3A | | THBS2 | | RNASE1 | | RSPO4 | WFDC2 | 0.738 |
| REG3A | GDF15 | | SVEP1 | RNASE1 | | RSPO4 | | 0.738 |
| REG3A | | THBS2 | SVEP1 | | TAGLN | | | 0.738 |
| REG3A | GDF15 | | SVEP1 | RNASE1 | TAGLN | RSPO4 | WFDC2 | 0.738 |
| REG3A | | THBS2 | | RNASE1 | TAGLN | RSPO4 | WFDC2 | 0.737 |
| REG3A | | THBS2 | SVEP1 | RNASE1 | TAGLN | RSPO4 | WFDC2 | 0.737 |
| REG3A | | THBS2 | | | TAGLN | RSPO4 | WFDC2 | 0.737 |
| REG3A | GDF15 | | | RNASE1 | | RSPO4 | WFDC2 | 0.737 |
| REG3A | | THBS2 | SVEP1 | | TAGLN | RSPO4 | WFDC2 | 0.737 |
| REG3A | | THBS2 | SVEP1 | | | RSPO4 | WFDC2 | 0.737 |
| REG3A | | THBS2 | | | | RSPO4 | WFDC2 | 0.737 |
| REG3A | | THBS2 | SVEP1 | RNASE1 | | | | 0.736 |
| REG3A | GDF15 | | SVEP1 | | | RSPO4 | WFDC2 | 0.736 |
| REG3A | GDF15 | | SVEP1 | | TAGLN | RSPO4 | WFDC2 | 0.736 |
| REG3A | GDF15 | | SVEP1 | RNASE1 | TAGLN | RSPO4 | | 0.736 |
| REG3A | GDF15 | | SVEP1 | | | RSPO4 | | 0.736 |
| REG3A | GDF15 | | | RNASE1 | TAGLN | RSPO4 | WFDC2 | 0.736 |
| REG3A | GDF15 | | SVEP1 | | TAGLN | RSPO4 | | 0.736 |
| REG3A | GDF15 | | SVEP1 | RNASE1 | | | WFDC2 | 0.736 |
| REG3A | | THBS2 | | | TAGLN | | WFDC2 | 0.736 |
| REG3A | | THBS2 | SVEP1 | | TAGLN | | WFDC2 | 0.736 |
| REG3A | GDF15 | | | RNASE1 | | RSPO4 | | 0.736 |
| REG3A | | THBS2 | | RNASE1 | | | | 0.735 |
| REG3A | | THBS2 | | RNASE1 | TAGLN | | WFDC2 | 0.735 |
| REG3A | | THBS2 | SVEP1 | RNASE1 | TAGLN | | WFDC2 | 0.735 |
| REG3A | GDF15 | | | | TAGLN | RSPO4 | WFDC2 | 0.735 |
| REG3A | | THBS2 | SVEP1 | RNASE1 | | | WFDC2 | 0.735 |
| REG3A | GDF15 | | SVEP1 | RNASE1 | | | | 0.735 |
| REG3A | | THBS2 | SVEP1 | | | | WFDC2 | 0.735 |
| REG3A | GDF15 | | SVEP1 | | TAGLN | | WFDC2 | 0.735 |
| REG3A | GDF15 | | | RNASE1 | TAGLN | RSPO4 | | 0.734 |
| REG3A | GDF15 | | SVEP1 | | | | WFDC2 | 0.734 |
| REG3A | GDF15 | | | | | RSPO4 | WFDC2 | 0.734 |
| REG3A | GDF15 | | | | TAGLN | RSPO4 | | 0.734 |
| REG3A | GDF15 | | SVEP1 | RNASE1 | TAGLN | | WFDC2 | 0.734 |
| REG3A | GDF15 | | SVEP1 | | | | | 0.734 |
| REG3A | | THBS2 | | | | RSPO4 | | 0.734 |
| REG3A | GDF15 | | | | | RSPO4 | | 0.734 |
| REG3A | GDF15 | | SVEP1 | | TAGLN | | | 0.734 |

(continued)

| | | | | | | | | |
|---|---|---|---|---|---|---|---|---|
| ADAMTSL2 | | | | | | | | 0.658 |
| REG3A | | THBS2 | SVEP1 | | | RSPO4 | | 0.734 |
| REG3A | | THBS2 | | RNASE1 | | | WFDC2 | 0.733 |
| REG3A | | THBS2 | | | | | WFDC2 | 0.733 |
| REG3A | GDF15 | | SVEP1 | RNASE1 | TAGLN | | | 0.733 |
| REG3A | | THBS2 | | | | | | 0.732 |
| REG3A | | THBS2 | SVEP1 | | | | | 0.732 |
| REG3A | GDF15 | | | RNASE1 | TAGLN | | WFDC2 | 0.732 |
| REG3A | GDF15 | | | | TAGLN | | WFDC2 | 0.731 |
| REG3A | GDF15 | | | RNASE1 | TAGLN | | | 0.730 |
| REG3A | GDF15 | | | | TAGLN | | | 0.730 |
| REG3A | GDF15 | | | RNASE1 | | | WFDC2 | 0.730 |
| REG3A | | | SVEP1 | RNASE1 | | RSPO4 | WFDC2 | 0.729 |
| REG3A | | | SVEP1 | RNASE1 | | RSPO4 | | 0.729 |
| REG3A | GDF15 | | | RNASE1 | | | | 0.729 |
| REG3A | | | SVEP1 | RNASE1 | TAGLN | RSPO4 | | 0.729 |
| REG3A | GDF15 | | | | | | WFDC2 | 0.728 |
| REG3A | | | SVEP1 | RNASE1 | TAGLN | RSPO4 | WFDC2 | 0.728 |
| REG3A | GDF15 | | | | | | | 0.728 |
| REG3A | | | SVEP1 | RNASE1 | TAGLN | | | 0.726 |
| REG3A | | | SVEP1 | RNASE1 | | | WFDC2 | 0.726 |
| REG3A | | | SVEP1 | RNASE1 | | | | 0.725 |
| REG3A | | | SVEP1 | | TAGLN | RSPO4 | WFDC2 | 0.725 |
| REG3A | | | SVEP1 | RNASE1 | TAGLN | | WFDC2 | 0.725 |
| REG3A | | | SVEP1 | | | RSPO4 | WFDC2 | 0.724 |
| REG3A | | | | RNASE1 | TAGLN | RSPO4 | WFDC2 | 0.723 |
| REG3A | | | | RNASE1 | TAGLN | RSPO4 | | 0.723 |
| REG3A | | | SVEP1 | | TAGLN | | WFDC2 | 0.723 |
| REG3A | | | SVEP1 | | TAGLN | RSPO4 | | 0.723 |
| REG3A | | | SVEP1 | | TAGLN | | | 0.722 |
| REG3A | | | | RNASE1 | | RSPO4 | WFDC2 | 0.722 |
| REG3A | | | SVEP1 | | | | WFDC2 | 0.721 |
| REG3A | | | | | TAGLN | RSPO4 | WFDC2 | 0.720 |
| REG3A | | | | RNASE1 | | RSPO4 | | 0.720 |
| REG3A | | | | RNASE1 | TAGLN | | | 0.718 |
| REG3A | | | | | TAGLN | RSPO4 | | 0.718 |
| REG3A | | | | RNASE1 | TAGLN | | WFDC2 | 0.718 |
| REG3A | | | | | TAGLN | | WFDC2 | 0.717 |
| REG3A | | | SVEP1 | | | RSPO4 | | 0.716 |
| REG3A | | | | | TAGLN | | | 0.715 |

(continued)

| RNASE6 | GDF15 | THBS2 | SVEP1 | RNASE1 | TAGLN | RSPO4 | WFDC2 | C-index |
|---|---|---|---|---|---|---|---|---|
| ADAMTSL2 | | | | | | | | 0.658 |
| REG3A | | | | | | RSPO4 | WFDC2 | 0.715 |
| REG3A | | | SVEP1 | | | | | 0.713 |
| REG3A | | | | RNASE1 | | | WFDC2 | 0.708 |
| REG3A | | | | RNASE1 | | | | 0.706 |
| REG3A | | | | | | | WFDC2 | 0.702 |
| REG3A | | | | | | RSPO4 | | 0.702 |
| REG3A | | | | | | | | 0.680 |
| **RNASE6** | **GDF15** | **THBS2** | **SVEP1** | **RNASE1** | **TAGLN** | **RSPO4** | **WFDC2** | **C-index** |
| RNASE6 | GDF15 | THBS2 | | RNASE1 | | RSPO4 | | 0.745 |
| RNASE6 | GDF15 | THBS2 | SVEP1 | RNASE1 | | RSPO4 | | 0.745 |
| RNASE6 | GDF15 | THBS2 | SVEP1 | | | RSPO4 | | 0.745 |
| RNASE6 | GDF15 | THBS2 | | | | RSPO4 | | 0.745 |
| RNASE6 | GDF15 | THBS2 | SVEP1 | RNASE1 | | RSPO4 | WFDC2 | 0.745 |
| RNASE6 | GDF15 | THBS2 | | | | RSPO4 | WFDC2 | 0.745 |
| RNASE6 | GDF15 | THBS2 | | RNASE1 | | RSPO4 | WFDC2 | 0.745 |
| RNASE6 | GDF15 | THBS2 | SVEP1 | | | RSPO4 | WFDC2 | 0.745 |
| RNASE6 | GDF15 | THBS2 | SVEP1 | | TAGLN | RSPO4 | WFDC2 | 0.744 |
| RNASE6 | GDF15 | THBS2 | | | TAGLN | RSPO4 | WFDC2 | 0.744 |
| RNASE6 | GDF15 | THBS2 | SVEP1 | | TAGLN | RSPO4 | | 0.744 |
| RNASE6 | GDF15 | THBS2 | | | TAGLN | RSPO4 | | 0.744 |
| RNASE6 | GDF15 | THBS2 | SVEP1 | RNASE1 | TAGLN | RSPO4 | WFDC2 | 0.744 |
| RNASE6 | GDF15 | THBS2 | | RNASE1 | TAGLN | RSPO4 | WFDC2 | 0.744 |
| RNASE6 | GDF15 | THBS2 | SVEP1 | RNASE1 | TAGLN | RSPO4 | | 0.743 |
| RNASE6 | GDF15 | THBS2 | | RNASE1 | TAGLN | RSPO4 | | 0.743 |
| RNASE6 | GDF15 | | SVEP1 | RNASE1 | | RSPO4 | | 0.742 |
| RNASE6 | GDF15 | | SVEP1 | RNASE1 | | RSPO4 | WFDC2 | 0.742 |
| RNASE6 | GDF15 | THBS2 | SVEP1 | RNASE1 | | | WFDC2 | 0.742 |
| RNASE6 | GDF15 | THBS2 | SVEP1 | | | | WFDC2 | 0.742 |
| RNASE6 | GDF15 | | SVEP1 | | | RSPO4 | | 0.742 |
| RNASE6 | GDF15 | THBS2 | SVEP1 | RNASE1 | | | | 0.742 |
| RNASE6 | GDF15 | THBS2 | SVEP1 | | | | | 0.741 |
| RNASE6 | GDF15 | | SVEP1 | | | RSPO4 | WFDC2 | 0.741 |
| RNASE6 | GDF15 | THBS2 | SVEP1 | | TAGLN | | WFDC2 | 0.741 |
| RNASE6 | GDF15 | THBS2 | SVEP1 | RNASE1 | TAGLN | | WFDC2 | 0.741 |
| RNASE6 | GDF15 | THBS2 | | RNASE1 | TAGLN | | WFDC2 | 0.741 |
| RNASE6 | GDF15 | THBS2 | SVEP1 | | TAGLN | | | 0.741 |
| RNASE6 | | THBS2 | | RNASE1 | TAGLN | RSPO4 | | 0.741 |
| RNASE6 | GDF15 | | SVEP1 | | TAGLN | RSPO4 | WFDC2 | 0.741 |
| RNASE6 | | THBS2 | SVEP1 | | TAGLN | RSPO4 | | 0.741 |

(continued)

| | | | | | | | | |
|---|---|---|---|---|---|---|---|---|
| ADAMTSL2 | | | | | | | | 0.658 |
| RNASE6 | GDF15 | THBS2 | SVEP1 | RNASE1 | TAGLN | | | 0.741 |
| RNASE6 | GDF15 | THBS2 | | | TAGLN | | WFDC2 | 0.741 |
| RNASE6 | GDF15 | THBS2 | | RNASE1 | TAGLN | | | 0.741 |
| RNASE6 | | THBS2 | | | TAGLN | RSPO4 | | 0.741 |
| RNASE6 | GDF15 | | SVEP1 | | TAGLN | RSPO4 | | 0.741 |
| RNASE6 | | THBS2 | SVEP1 | RNASE1 | TAGLN | RSPO4 | | 0.741 |
| RNASE6 | GDF15 | | SVEP1 | RNASE1 | TAGLN | RSPO4 | WFDC2 | 0.740 |
| RNASE6 | GDF15 | THBS2 | | | TAGLN | | | 0.740 |
| RNASE6 | GDF15 | | | | TAGLN | RSPO4 | WFDC2 | 0.740 |
| RNASE6 | GDF15 | THBS2 | | | | | WFDC2 | 0.740 |
| RNASE6 | GDF15 | THBS2 | | RNASE1 | | | WFDC2 | 0.740 |
| RNASE6 | GDF15 | | | RNASE1 | | RSPO4 | WFDC2 | 0.740 |
| RNASE6 | GDF15 | THBS2 | | RNASE1 | | | | 0.740 |
| RNASE6 | GDF15 | THBS2 | | | | | | 0.740 |
| RNASE6 | GDF15 | | | | | RSPO4 | WFDC2 | 0.740 |
| RNASE6 | | THBS2 | SVEP1 | RNASE1 | | RSPO4 | WFDC2 | 0.740 |
| RNASE6 | GDF15 | | | RNASE1 | TAGLN | RSPO4 | WFDC2 | 0.739 |
| RNASE6 | | THBS2 | SVEP1 | RNASE1 | TAGLN | RSPO4 | WFDC2 | 0.739 |
| RNASE6 | GDF15 | | SVEP1 | RNASE1 | TAGLN | RSPO4 | | 0.739 |
| RNASE6 | | THBS2 | SVEP1 | | TAGLN | | | 0.739 |
| RNASE6 | | THBS2 | SVEP1 | | | RSPO4 | WFDC2 | 0.739 |
| RNASE6 | | THBS2 | SVEP1 | | TAGLN | RSPO4 | WFDC2 | 0.739 |
| RNASE6 | | THBS2 | SVEP1 | RNASE1 | | RSPO4 | | 0.739 |
| RNASE6 | | THBS2 | | | TAGLN | RSPO4 | WFDC2 | 0.739 |
| RNASE6 | | THBS2 | | RNASE1 | TAGLN | | | 0.739 |
| RNASE6 | | THBS2 | | RNASE1 | TAGLN | RSPO4 | WFDC2 | 0.739 |
| RNASE6 | GDF15 | | | RNASE1 | | RSPO4 | | 0.739 |
| RNASE6 | | THBS2 | | RNASE1 | | RSPO4 | WFDC2 | 0.739 |
| RNASE6 | GDF15 | | | | | RSPO4 | | 0.739 |
| RNASE6 | | THBS2 | | | TAGLN | | | 0.739 |
| RNASE6 | | THBS2 | | RNASE1 | | RSPO4 | | 0.739 |
| RNASE6 | GDF15 | | | | TAGLN | RSPO4 | | 0.739 |
| RNASE6 | | THBS2 | SVEP1 | RNASE1 | TAGLN | | | 0.739 |
| RNASE6 | | THBS2 | | | | RSPO4 | WFDC2 | 0.739 |
| RNASE6 | GDF15 | | SVEP1 | RNASE1 | | | WFDC2 | 0.739 |
| RNASE6 | GDF15 | | | RNASE1 | TAGLN | RSPO4 | | 0.738 |
| RNASE6 | GDF15 | | SVEP1 | | | | | 0.738 |
| RNASE6 | GDF15 | | SVEP1 | | | | WFDC2 | 0.738 |
| RNASE6 | GDF15 | | SVEP1 | RNASE1 | | | | 0.738 |

(continued)

| | | | | | | | | |
|---|---|---|---|---|---|---|---|---|
| ADAMTSL2 | | | | | | | | 0.658 |
| RNASE6 | GDF15 | | SVEP1 | | TAGLN | | WFDC2 | 0.738 |
| RNASE6 | GDF15 | | SVEP1 | RNASE1 | TAGLN | | WFDC2 | 0.737 |
| RNASE6 | GDF15 | | SVEP1 | | TAGLN | | | 0.737 |
| RNASE6 | | THBS2 | SVEP1 | RNASE1 | TAGLN | | WFDC2 | 0.737 |
| RNASE6 | | THBS2 | SVEP1 | | TAGLN | | WFDC2 | 0.737 |
| RNASE6 | | THBS2 | | | TAGLN | | WFDC2 | 0.736 |
| RNASE6 | | THBS2 | | RNASE1 | TAGLN | | WFDC2 | 0.736 |
| RNASE6 | | THBS2 | SVEP1 | | | | WFDC2 | 0.736 |
| RNASE6 | GDF15 | | SVEP1 | RNASE1 | TAGLN | | | 0.736 |
| RNASE6 | | THBS2 | SVEP1 | | | RSPO4 | | 0.736 |
| RNASE6 | | THBS2 | SVEP1 | RNASE1 | | | WFDC2 | 0.736 |
| RNASE6 | GDF15 | | | | TAGLN | | WFDC2 | 0.735 |
| RNASE6 | | THBS2 | SVEP1 | RNASE1 | | | | 0.735 |
| RNASE6 | | THBS2 | | | | RSPO4 | | 0.735 |
| RNASE6 | GDF15 | | | RNASE1 | TAGLN | | WFDC2 | 0.735 |
| RNASE6 | GDF15 | | | RNASE1 | TAGLN | | | 0.734 |
| RNASE6 | GDF15 | | | | TAGLN | | | 0.734 |
| RNASE6 | | THBS2 | | | | | WFDC2 | 0.734 |
| RNASE6 | | THBS2 | | RNASE1 | | | | 0.733 |
| RNASE6 | | THBS2 | | RNASE1 | | | WFDC2 | 0.733 |
| RNASE6 | GDF15 | | | | | | WFDC2 | 0.733 |
| RNASE6 | | THBS2 | SVEP1 | | | | | 0.732 |
| RNASE6 | GDF15 | | | RNASE1 | | | WFDC2 | 0.732 |
| RNASE6 | GDF15 | | | | | | | 0.732 |
| RNASE6 | | | SVEP1 | RNASE1 | | RSPO4 | WFDC2 | 0.732 |
| RNASE6 | | | SVEP1 | RNASE1 | TAGLN | RSPO4 | | 0.732 |
| RNASE6 | GDF15 | | | RNASE1 | | | | 0.732 |
| RNASE6 | | | SVEP1 | RNASE1 | | RSPO4 | | 0.732 |
| RNASE6 | | | SVEP1 | RNASE1 | TAGLN | RSPO4 | WFDC2 | 0.731 |
| RNASE6 | | | SVEP1 | | TAGLN | RSPO4 | WFDC2 | 0.730 |
| RNASE6 | | | SVEP1 | | TAGLN | RSPO4 | | 0.730 |
| RNASE6 | | | SVEP1 | | | RSPO4 | WFDC2 | 0.730 |
| RNASE6 | | | SVEP1 | RNASE1 | TAGLN | | | 0.730 |
| RNASE6 | | THBS2 | | | | | | 0.729 |
| RNASE6 | | | SVEP1 | | TAGLN | | | 0.729 |
| RNASE6 | | | SVEP1 | RNASE1 | TAGLN | | WFDC2 | 0.728 |
| RNASE6 | | | SVEP1 | RNASE1 | | | WFDC2 | 0.728 |
| RNASE6 | | | SVEP1 | | TAGLN | | WFDC2 | 0.728 |
| RNASE6 | | | SVEP1 | RNASE1 | | | | 0.728 |

(continued)

| | | | | | | | | |
|---|---|---|---|---|---|---|---|---|
| ADAMTSL2 | | | | | | | | 0.658 |
| RNASE6 | | | | RNASE1 | TAGLN | RSPO4 | | 0.727 |
| RNASE6 | | | SVEP1 | | | | WFDC2 | 0.727 |
| RNASE6 | | | | RNASE1 | TAGLN | RSPO4 | WFDC2 | 0.725 |
| RNASE6 | | | | RNASE1 | TAGLN | | | 0.725 |
| RNASE6 | | | | | TAGLN | RSPO4 | | 0.724 |
| RNASE6 | | | SVEP1 | | | RSPO4 | | 0.724 |
| RNASE6 | | | | | TAGLN | RSPO4 | WFDC2 | 0.724 |
| RNASE6 | | | | RNASE1 | | RSPO4 | WFDC2 | 0.723 |
| RNASE6 | | | | | TAGLN | | | 0.723 |
| RNASE6 | | | | RNASE1 | | RSPO4 | | 0.723 |
| RNASE6 | | | | | TAGLN | | WFDC2 | 0.721 |
| RNASE6 | | | | RNASE1 | TAGLN | | WFDC2 | 0.721 |
| RNASE6 | | | | | | RSPO4 | WFDC2 | 0.720 |
| RNASE6 | | | SVEP1 | | | | | 0.720 |
| RNASE6 | | | | RNASE1 | | | WFDC2 | 0.710 |
| RNASE6 | | | | | | RSPO4 | | 0.709 |
| RNASE6 | | | | RNASE1 | | | | 0.709 |
| RNASE6 | | | | | | | WFDC2 | 0.707 |
| RNASE6 | | | | | | | | 0.690 |
| SVEP1 | GDF15 | THRS2 | SVFP1 | PNASE1 | TAGLN | RSPO4 | WEDC2 | C-index |
| SVEP1 | GDF15 | THBS2 | SVEP1 | RNASE1 | | RSPO4 | WFDC2 | 0.742 |
| SVEP1 | GDF15 | THBS2 | | RNASE1 | | RSPO4 | WFDC2 | 0.742 |
| SVEP1 | GDF15 | THBS2 | SVEP1 | RNASE1 | | RSPO4 | | 0.742 |
| SVEP1 | GDF15 | THBS2 | | RNASE1 | | RSPO4 | | 0.741 |
| SVEP1 | GDF15 | THBS2 | | | TAGLN | RSPO4 | | 0.741 |
| SVEP1 | GDF15 | THBS2 | SVEP1 | | TAGLN | RSPO4 | | 0.741 |
| SVEP1 | GDF15 | THBS2 | SVEP1 | RNASE1 | TAGLN | RSPO4 | WFDC2 | 0.741 |
| SVEP1 | GDF15 | THBS2 | SVEP1 | RNASE1 | TAGLN | RSPO4 | | 0.741 |
| SVEP1 | GDF15 | THBS2 | | RNASE1 | TAGLN | RSPO4 | WFDC2 | 0.741 |
| SVEP1 | GDF15 | THBS2 | | RNASE1 | TAGLN | RSPO4 | | 0.741 |
| SVEP1 | GDF15 | THBS2 | | | TAGLN | RSPO4 | WFDC2 | 0.741 |
| SVEP1 | GDF15 | THBS2 | SVEP1 | | TAGLN | RSPO4 | WFDC2 | 0.741 |
| SVEP1 | GDF15 | THBS2 | | | | RSPO4 | WFDC2 | 0.739 |
| SVEP1 | GDF15 | THBS2 | SVEP1 | | | RSPO4 | WFDC2 | 0.739 |
| SVEP1 | GDF15 | THBS2 | SVEP1 | | | RSPO4 | | 0.739 |
| SVEP1 | GDF15 | THBS2 | | | | RSPO4 | | 0.739 |
| SVEP1 | GDF15 | | | RNASE1 | | RSPO4 | | 0.739 |
| SVEP1 | GDF15 | | SVEP1 | RNASE1 | | RSPO4 | WFDC2 | 0.739 |
| SVEP1 | GDF15 | | SVEP1 | RNASE1 | | RSPO4 | | 0.739 |

(continued)

| | | | | | | | | |
|---|---|---|---|---|---|---|---|---|
| ADAMTSL2 | | | | | | | | 0.658 |
| SVEP1 | GDF15 | | | RNASE1 | | RSPO4 | WFDC2 | 0.738 |
| SVEP1 | GDF15 | | SVEP1 | RNASE1 | TAGLN | RSPO4 | WFDC2 | 0.738 |
| SVEP1 | | THBS2 | SVEP1 | RNASE1 | TAGLN | RSPO4 | | 0.738 |
| SVEP1 | | THBS2 | | RNASE1 | TAGLN | RSPO4 | | 0.738 |
| SVEP1 | GDF15 | | | RNASE1 | TAGLN | RSPO4 | WFDC2 | 0.737 |
| SVEP1 | GDF15 | THBS2 | | | TAGLN | | | 0.737 |
| SVEP1 | GDF15 | | SVEP1 | | TAGLN | RSPO4 | | 0.737 |
| SVEP1 | GDF15 | THBS2 | SVEP1 | | TAGLN | | | 0.737 |
| SVEP1 | GDF15 | | SVEP1 | | TAGLN | RSPO4 | WFDC2 | 0.737 |
| SVEP1 | GDF15 | | | RNASE1 | TAGLN | RSPO4 | | 0.737 |
| SVEP1 | GDF15 | | SVEP1 | RNASE1 | TAGLN | RSPO4 | | 0.737 |
| SVEP1 | GDF15 | | | | TAGLN | RSPO4 | | 0.737 |
| SVEP1 | GDF15 | | | | TAGLN | RSPO4 | WFDC2 | 0.737 |
| SVEP1 | GDF15 | THBS2 | SVEP1 | | TAGLN | | WFDC2 | 0.737 |
| SVEP1 | | THBS2 | SVEP1 | RNASE1 | TAGLN | RSPO4 | WFDC2 | 0.737 |
| SVEP1 | GDF15 | THBS2 | | | TAGLN | | WFDC2 | 0.737 |
| SVEP1 | GDF15 | THBS2 | SVEP1 | RNASE1 | TAGLN | | WFDC2 | 0.737 |
| SVEP1 | GDF15 | THBS2 | SVEP1 | RNASE1 | TAGLN | | | 0.737 |
| SVEP1 | | THBS2 | | RNASE1 | TAGLN | RSPO4 | WFDC2 | 0.737 |
| SVEP1 | GDF15 | THBS2 | | RNASE1 | TAGLN | | | 0.737 |
| SVEP1 | GDF15 | THBS2 | | RNASE1 | TAGLN | | WFDC2 | 0.737 |
| SVEP1 | GDF15 | THBS2 | SVEP1 | RNASE1 | | | WFDC2 | 0.736 |
| SVEP1 | GDF15 | THBS2 | SVEP1 | RNASE1 | | | | 0.736 |
| SVEP1 | | THBS2 | | RNASE1 | | RSPO4 | WFDC2 | 0.736 |
| SVEP1 | GDF15 | THBS2 | | RNASE1 | | | | 0.736 |
| SVEP1 | GDF15 | THBS2 | | RNASE1 | | | WFDC2 | 0.736 |
| SVEP1 | | THBS2 | SVEP1 | RNASE1 | | RSPO4 | WFDC2 | 0.736 |
| SVEP1 | GDF15 | | SVEP1 | | | RSPO4 | | 0.736 |
| SVEP1 | GDF15 | | | | | RSPO4 | | 0.736 |
| SVEP1 | | THBS2 | SVEP1 | | TAGLN | RSPO4 | WFDC2 | 0.736 |
| SVEP1 | GDF15 | | | | | RSPO4 | WFDC2 | 0.736 |
| SVEP1 | GDF15 | | SVEP1 | | | RSPO4 | WFDC2 | 0.735 |
| SVEP1 | | THBS2 | | | TAGLN | RSPO4 | WFDC2 | 0.735 |
| SVEP1 | | THBS2 | | RNASE1 | | RSPO4 | | 0.735 |
| SVEP1 | | THBS2 | SVEP1 | RNASE1 | | RSPO4 | | 0.735 |
| SVEP1 | | THBS2 | SVEP1 | | TAGLN | RSPO4 | | 0.735 |
| SVEP1 | | THBS2 | | | TAGLN | RSPO4 | | 0.735 |
| SVEP1 | GDF15 | THBS2 | SVEP1 | | | | WFDC2 | 0.735 |
| SVEP1 | | THBS2 | | RNASE1 | TAGLN | | | 0.735 |

(continued)

| | | | | | | | | |
|---|---|---|---|---|---|---|---|---|
| ADAMTSL2 | | | | | | | | 0.658 |
| SVEP1 | GDF15 | THBS2 | | | | | WFDC2 | 0.734 |
| SVEP1 | | THBS2 | SVEP1 | RNASE1 | TAGLN | | | 0.734 |
| SVEP1 | GDF15 | THBS2 | | | | | | 0.734 |
| SVEP1 | GDF15 | THBS2 | SVEP1 | | | | | 0.734 |
| SVEP1 | GDF15 | | | | TAGLN | | | 0.734 |
| SVEP1 | GDF15 | | SVEP1 | | TAGLN | | | 0.734 |
| SVEP1 | GDF15 | | | | TAGLN | | WFDC2 | 0.734 |
| SVEP1 | GDF15 | | SVEP1 | | TAGLN | | WFDC2 | 0.733 |
| SVEP1 | GDF15 | | | RNASE1 | | | WFDC2 | 0.733 |
| SVEP1 | GDF15 | | SVEP1 | RNASE1 | | | | 0.733 |
| SVEP1 | GDF15 | | | RNASE1 | | | | 0.733 |
| SVEP1 | GDF15 | | SVEP1 | RNASE1 | | | WFDC2 | 0.733 |
| SVEP1 | GDF15 | | | RNASE1 | TAGLN | | WFDC2 | 0.733 |
| SVEP1 | GDF15 | | SVEP1 | RNASE1 | TAGLN | | WFDC2 | 0.733 |
| SVEP1 | GDF15 | | SVEP1 | RNASE1 | TAGLN | | | 0.733 |
| SVEP1 | GDF15 | | | RNASE1 | TAGLN | | | 0.732 |
| SVEP1 | | THBS2 | | | TAGLN | | | 0.732 |
| SVEP1 | | THBS2 | SVEP1 | | | RSPO4 | WFDC2 | 0.732 |
| SVEP1 | | THBS2 | | RNASE1 | TAGLN | | WFDC2 | 0.732 |
| SVEP1 | | THBS2 | SVEP1 | | TAGLN | | | 0.732 |
| SVEP1 | | THBS2 | SVEP1 | RNASE1 | TAGLN | | WFDC2 | 0.732 |
| SVEP1 | | THBS2 | | | | RSPO4 | WFDC2 | 0.732 |
| SVEP1 | GDF15 | | | | | | | 0.732 |
| SVEP1 | | THBS2 | | | TAGLN | | WFDC2 | 0.731 |
| SVEP1 | | THBS2 | SVEP1 | | TAGLN | | WFDC2 | 0.731 |
| SVEP1 | GDF15 | | SVEP1 | | | | | 0.731 |
| SVEP1 | GDF15 | | | | | | WFDC2 | 0.731 |
| SVEP1 | GDF15 | | SVEP1 | | | | WFDC2 | 0.731 |
| SVEP1 | | | SVEP1 | RNASE1 | TAGLN | RSPO4 | | 0.730 |
| SVEP1 | | | | RNASE1 | TAGLN | RSPO4 | | 0.730 |
| SVEP1 | | THBS2 | | RNASE1 | | | WFDC2 | 0.730 |
| SVEP1 | | THBS2 | SVEP1 | RNASE1 | | | WFDC2 | 0.730 |
| SVEP1 | | THBS2 | SVEP1 | RNASE1 | | | | 0.729 |
| SVEP1 | | THBS2 | | RNASE1 | | | | 0.729 |
| SVEP1 | | | SVEP1 | RNASE1 | | RSPO4 | WFDC2 | 0.729 |
| SVEP1 | | | SVEP1 | RNASE1 | TAGLN | RSPO4 | WFDC2 | 0.729 |
| SVEP1 | | | SVEP1 | RNASE1 | | RSPO4 | | 0.729 |
| SVEP1 | | | | RNASE1 | | RSPO4 | | 0.729 |
| SVEP1 | | | | RNASE1 | | RSPO4 | WFDC2 | 0.729 |

(continued)

| | | | | | | | | |
|---|---|---|---|---|---|---|---|---|
| ADAMTSL2 | | | | | | | | 0.658 |
| SVEP1 | | | | RNASE1 | TAGLN | RSPO4 | WFDC2 | 0.729 |
| SVEP1 | | THBS2 | SVEP1 | | | | WFDC2 | 0.727 |
| SVEP1 | | | | RNASE1 | TAGLN | | | 0.726 |
| SVEP1 | | THBS2 | | | | | WFDC2 | 0.726 |
| SVEP1 | | | SVEP1 | RNASE1 | TAGLN | | | 0.726 |
| SVEP1 | | | SVEP1 | | TAGLN | RSPO4 | WFDC2 | 0.725 |
| SVEP1 | | | | | TAGLN | RSPO4 | WFDC2 | 0.725 |
| SVEP1 | | | SVEP1 | RNASE1 | TAGLN | | WFDC2 | 0.724 |
| SVEP1 | | | | RNASE1 | TAGLN | | WFDC2 | 0.724 |
| SVEP1 | | | SVEP1 | RNASE1 | | | WFDC2 | 0.723 |
| SVEP1 | | | SVEP1 | RNASE1 | | | | 0.722 |
| SVEP1 | | | SVEP1 | | TAGLN | | WFDC2 | 0.722 |
| SVEP1 | | | | RNASE1 | | | WFDC2 | 0.722 |
| SVEP1 | | | SVEP1 | | TAGLN | RSPO4 | | 0.722 |
| SVEP1 | | | | RNASE1 | | | | 0.722 |
| SVEP1 | | | | | TAGLN | RSPO4 | | 0.722 |
| SVEP1 | | | SVEP1 | | | RSPO4 | WFDC2 | 0.722 |
| SVEP1 | | | | | TAGLN | | WFDC2 | 0.721 |
| SVEP1 | | | | | | RSPO4 | WFDC2 | 0.721 |
| SVEP1 | | | | | TAGLN | | | 0.719 |
| SVEP1 | | | S VEP1 | | TAGLN | | | 0.718 |
| SVEP1 | | THB S2 | | | | RSPO4 | | 0.716 |
| SVEP1 | | THB S2 | SVEP1 | | | RSPO4 | | 0.716 |
| SVEP1 | | | SVEP1 | | | | WFDC2 | 0.716 |
| SVEP1 | | | | | | | WFDC2 | 0.715 |
| SVEP1 | | THB S2 | | | | | | 0.708 |
| SVEP1 | | THBS2 | SVEP1 | | | | | 0.708 |
| SVEP1 | | | | | | RSPO4 | | 0.697 |
| SVEP1 | | | S VEP1 | | | RSPO4 | | 0.697 |
| SVEP1 | | | | | | | | 0.687 |
| SVEP1 | | | S VEP1 | | | | | 0.686 |

Example 4. HFpEF Model and Prediction of Cardiovascular Events

In order to predict the risk or likelihood that an individual with HFpEF will have a CV event within one year, a model containing a panel of 14 biomarker proteins was developed. The CV event was defined as death. The training analysis was developed using the BMS Penn Heart Failure Study (PHFS) data set. The PHFS includes 1,345 patients and 360 events. A portion of the University of Arizona Henry Ford data set (AZHF) and the ARIC visit 5 data set were used for verification. The validation data sets are holdouts from the PHFS, AZHF, and ARIC data sets.

Table 9A shows stratification of the datasets for HFpEF model training, verification and validation.

**Table 9A**

| Data Use (%) Dataset | Training | Verification | Hold-out Validation |
|---|---|---|---|
| PHFS | 80% | 0% | 20% |
| ARIC visit 5 | 0% | 50% | 50% |
| AZHF | 0% | 20% | 80% |

The HFpEF model is an accelerated failure time (AFT) survival model with a Weibull distribution. This model has 14 aptamers as its features. The 14 aptamers bind 14 different biomarker proteins, which are listed in Table 2. The output of this model is the probability of survival at one year, and traditional metrics are traditionally assessed as the inverse probability (i.e., probability of the event). The feature list was refined using univariate association in training and verification data sets, a cross-validated elastic net for feature selection models, and removal of analytes with high CV. The final model was trained using unpenalized AFT regression methods. The model metrics are shown in Table 9B below. The results in Table 9B show that the model exceeds the performance criteria for 1-year risk prediction on the training, verification, and validation data sets.

**Table 9B**

| Modeling Usage of Data | Data Set | C-Index (95% CI) | AUC at 365 days | AUC at 180 days |
|---|---|---|---|---|
| Training | 80% BMS Penn | 0.834 (0.791, 0.876) | 0.850 (0.787, 0.908) | 0.839 (0.768, 0.902) |
| Verification | 20% AZHF | 0.819 (0.742, 0.895) | 0.855 (0.696, 0.982) | 0.810 (0.5, 0.895) |
|  | 50% ARIC visit 5 with HFpEF | 0.750 (0.694, 0.797) | 0.747 (0.622, 0.844) | 0.732 (0.5, 1) |
| Validation | Combined dataset (20% BMS Penn, 80% AZHF, 50% ARIC visit 5 with HFpEF) | 0.761 (0.725, 0.792) | 0.807 (0.734, 0.871) | 0.800 (0.725, 0.876) |

Model Development

The demographics of the portion of the PHFS development cohort used in the training data set are shown in Table 10 below. The demographics for the portions of the AZHF cohort and ARIC visit 5 cohort used in the verification data sets are shown in Tables 11A and 11B, respectively. The demographics for the portions of the PHFS, AZHF, ARIC visit 5 cohorts used in the validation data sets are shown in Table 12, respectively.

**Table 10: PHFS development cohort**

| Covariate | Measure | Total | Death within 1 year | Survival at 1 year |
|---|---|---|---|---|
| Sample Size |  | 348 | 63 (18.1%) | 285 (81.9%) |
| Age | Mean (SD) | 72.1 (12.4) | 77.7 (10.2) | 70.4 (12.5) |
|  | Median | 74 | 79 | 73.5 |
|  | Range | 40 - 98 | 55 - 97 | 40 - 98 |
| Sex | Male | 36 (33.6%) | 14 (56%) | 22 (26.8%) |
|  | Female | 71 (66.4%) | 11 (44%) | 60 (73.2%) |
| Ethnicity | Caucasian | 51 (47.7%) | 17 (68%) | 34 (41.5%) |
|  | Black | 50 (46.7%) | 6 (24%) | 44 (53.7%) |
|  | Other | 6 (5.6%) | 2 (8%) | 4 (4.9%) |

(continued)

| Covariate | Measure | Total | Death within 1 year | Survival at 1 year |
|---|---|---|---|---|
| Diabetes | Yes | 49 (45.8%) | 10 (40%) | 39 (47.6%) |
|  | No | 58 (54.2%) | 15 (60%) | 43 (52.4%) |
| eGFR | Mean (SD) | 76.1 (48.1) | 56.6 (24.9) | 70.8 (31.3) |
|  | Median | 69.0 | 53.9 | 71.8 |
|  | Range | 8.3 - 403.4 | 55 - 97 | 8.3 - 164.3 |
| BMI | Mean (SD) | 31.1 (7.2) | 29.0 (4.8) | 31.7 (7.7) |
|  | Median | 29.7 | 27.9 | 31.2 |
|  | Range | 16.6-58.2 | 21.8-38.4 | 16.6-58.2 |

**Table 11A: AZHF verification cohort**

| Covariate | Measure | Total | Death within 1 year | Survival at 1 year |
|---|---|---|---|---|
| Sample Size |  | 107 | 25 (23.%) | 82 (76.6%) |
| Age | Mean (SD) | 72.1 (12.4) | 77.7 (10.2) | 70.0 (13.8) |
|  | Median | 74 | 79 | 58.1 |
|  | Range | 40-98 | 55-97 | 18.4-84.1 |
| Sex | Male | 36 (33.6%) | 14 (56%) | 146 (52.2%) |
|  | Female | 71 (66.4%) | 11 (44%) | 139 (48.8%) |
| Ethnicity | Caucasian | 51 (47.7%) | 17 (68%) | 206 (72.3%) |
|  | Black | 50 (46.7%) | 6 (24%) | 64 (22.5%) |
|  | Other | 6 (5.6%) | 2 (8%) | 15 (5.2%) |
| Diabetes | Yes | 49 (45.8%) | 10 (40%) | 76 (26.7%) |
|  | No | 58 (54.2%) | 15 (60%) | 209 (73.3%) |
| eGFR | Mean (SD) | 76.1 (48.1) | 56.6 (24.9) | 58.8 (22.6) |
|  | Median | 69.0 | 53.9 | 58.3 |
|  | Range | 8.3-403.4 | 55-97 | 4.6-139.6 |
| BMI | Mean (SD) | 31.1 (7.2) | 29.0 (4.8) | 32.3 (8.7) |
|  | Median | 29.7 | 27.9 | 30.1 |
|  | Range | 16.6-58.2 | 21.8-38.4 | 18.3-71.4 |

**Table 11B: ARIC visit 5 verification cohort**

| Covariate | Measure | Total | Death within 1 year | Survival at 1 year |
|---|---|---|---|---|
| Sample Size |  | 270 | 83 (30.7%) | 187 (69.3%) |
| Age | Mean (SD) | 76.9 (5.6) | 79.7 (5.8) | 75.6 (5.1) |
|  | Median | 77 | 81 | 75 |
|  | Range | 67-89 | 67-89 | 67-86 |
| Sex | Male | 134 (49.6%) | 52 (62.7%) | 82 (43.9%) |
|  | Female | 136 (50.4%) | 31 (37.5%) | 105 (56.2%) |
| Ethnicity | Caucasian | 203 (75.2%) | 72 (86.7%) | 131 (70.0%) |
|  | Black | 67 (24.8%) | 11 (13.3%) | 56 (30.0%) |
|  | Other | 0 (0%) | 0 (0%) | 0 (0%) |

(continued)

| Covariate | Measure | Total | Death within 1 year | Survival at 1 year |
|---|---|---|---|---|
| Diabetes | Yes | 113 (41.9%) | 34 (41%) | 79 (42.3%) |
| | No | 157 (58.2%) | 49 (59%) | 108 (57.8%) |
| eGFR | Mean (SD) | 62.6 (20.6) | 54.6 (20.0) | 66.2 (19.8) |
| | Median | 62.2 | 57.3 | 66.8 |
| | Range | 5.7-111.7 | 9.6-88.9 | 5.7-111.7 |
| BMI | Mean (SD) | 30.5 (6.7) | 29.3 (6.2) | 31.0 (6.9) |
| | Median | 29.4 | 28.4 | 29.7 |
| | Range | 19.6-76.1 | 19.6-52.5 | 19.8-76.1 |

Table 12: Combined validation cohort

| Covariate | Measure | Total | Death within 1 year | Survival at 1 year |
|---|---|---|---|---|
| Sample Size | | 785 | 225 (28.7%) | 560 (71.3%) |
| Age | Mean (SD) | 72.6 (11.1) | 76.7 (9.8) | 71.0 (11.1) |
| | Median | 74.0 | 77.0 | 72 |
| | Range | 24 - 100 | 37 - 100 | 24 - 94 |
| Sex | Male | 389 (49.6%) | 125 (55.6%) | 264 (47.1%) |
| | Female | 396 (50.5%) | 100 (44.4%) | 296 (52.9%) |
| Ethnicity | Caucasian | 489 (62.3%) | 150 (66.7%) | 339 (60.5%) |
| | Black | 268 (34.1%) | 60 (26.7%) | 208 (37.1%) |
| | Other | 28 (3.6%) | 15 (6.6%) | 13 (2.3%) |
| Diabetes | Yes | 332 (42.3%) | 113 (50.2%) | 219 (39.1%) |
| | No | 453 (57.7%) | 112 (49.8%) | 341 (60.9%) |
| eGFR | Mean (SD) | 65.1 (24.7) | 55.1 (23.2) | 69.1 (24.1) |
| | Median | 63.2 | 52.6 | 67.9 |
| | Range | 6.5 - 206.7 | 7.1 - 109.1 | 6.5 - 206.7 |
| BMI | Mean (SD) | 31.8 (7.6) | 30.4 (7.0) | 32.3 (7.8) |
| | Median | 30.5 | 29.9 | 30.8 |
| | Range | 15.7-70.7 | 15.7 - 59.6 | 17.3 - 70.7 |

To ensure quality of the data, pre-processing steps were performed before the data were analyzed. The pre-processing steps included data quality control (QC) and pre-analytics.

Data QC showed that 29 samples of the original combined data set failed row-check, meaning at least one of the hybridization or three median scale factors were outside the 0.4 to 2.5 range, indicating technical issues (*e.g.,* clogs) with that particular sample that would not be fixed by running the sample again. Additionally, there were 12 outlier samples with at least 5% of measurements more than 6 MADs from the median signal. These 41 samples in total (1.1%) were removed from further analyses. Finally, all analytes that did not pass target confirmation specificity testing were removed from the data set.

Pre-analytics did not show evidence of strong relationships between any of the clinical variables explored (age, sex, ethnicity, diabetes status, BMI, HFpEF/HFrEF status, event status, and eGFR) and the normalization scale factors.

After data quality control and pre-analytics, model development was completed in two steps, 1) proof of concept (POC), and 2) refinement.

Only the training data was used in the POC step. The preliminary models explored were Cox with elastic net regularization, and AFT with elastic net regularization using Weibull and log-logistic distributions all using 10 repeats of 5-fold cross-validation.

Initial model performance criteria were met for both the composite and the all-cause death endpoints. Furthermore, the HFpEF model trained on all-cause death performed almost as well as that trained on the composite endpoint at predicting the composite endpoint. The HFpEF pursued in refinement was trained on all-cause death so that it aligned with the HFrEF model refinement (see Example 2).

Models developed in refinement used the PFHS, AZHF, and ARIC visit 5 data sets. The PHFS data was split 80/20 training/validation, as in POC. AZHF was split 20/80 verification/validation, to preserve a sizeable candidate validation data set. ARIC data were split 50/50.

The final model is a Weibull AFT model and has 14 features. This model type was chosen because of its performance in POC, consistency with the HFrEF model, and ability to provide estimated risk probabilities.

POC Results

The POC results showed a number of analytes significant at different FDR levels for univariate Cox association with the endpoint. Those numbers and percentages are shown in Table 13 below for the HFpEF prognosis of all-cause death POC.

Table 13

| FDR | # analytes (%) ≤ FDR level |
|---|---|
| 0.10 | 780 (14.7%) |
| 0.05 | 593 (11.2%) |
| 0.01 | 323 (6.1%) |

The model that performed the best was an AFT Weibull model, which achieved a C-Index of 0.698, and an AUC of 0.81 at 90 days and an AUC of 0.685 at 365 days. Both of these models moved into refinement.

Although it was not one of the pre-defined models, assessment of HFpEF patients trained on all-cause-death and evaluated on an all-cause-death endpoint was also completed. The best model was an AFT Weibull distribution, which achieved a C-index of 0.718. Because this model passed the POC criterion for a C-index greater than 0.67, it also moved to refinement.

Refinement Results

The final model developed in refinement for the HFpEF population is a 14-aptamer AFT survival model using a Weibull distribution. The final model does not include regularization parameters (alpha and lambda). The model was trained on the 80% of PHFS data that was used for POC. The verification metrics were calculated on 20% of the AZHF patients with HFpEF, and 50% of the ARIC visit 5 patients with HFpEF. The rest of the data were held out for validation.

The model was built using a reduced feature list of overlapping univariate features from the derivation and AZHF verification data sets, further refined by LASSO feature selection. The best model had an alpha of 0.2, the minimum lambda (0.1) and the resulting best model with 15 features was observed to have a C-index difference of only 1% between training and verification, with both sets being above 0.80. A refined model was further assessed, and a feature with high CV was removed for a resulting 14-feature model. The final model was assessed for predicting an endpoint of all cause-death, using C-index and AUC at 1 year. Predictions were also assessed for concordance and AUC in predicting a composite endpoint of hospitalization or death. The results for training and verification data sets are shown in Table 14 below. The 95% Confidence Intervals (CI) are shown in parentheses.

Table 14

| Data set | C-index for death within 1 year | AUC for death endpoint at 1 year | AUC for death endpoint at 180 days | C-index for composite endpoint at 1 year | AUC for composite endpoint at 1 year |
|---|---|---|---|---|---|
| Training | 0.834 (0.791, 0.876) | 0.850 (0.787, 0.908) | 0.839 (0.768, 0.902) | 0.717 (0.675, 0.760) | 0.730 (0.661, 0.797) |
| Verification (AZHF) | 0.819 (0.742, 0.895) | 0.855 (0.696, 0.982) | 0.810 (0.5, 0.895) | 0.775 (0.705, 0.837) | 0.855 (0.605, 0.981) |

(continued)

| Data set | C-index for death within 1 year | AUC for death endpoint at 1 year | AUC for death endpoint at 180 days | C-index for composite endpoint at 1 year | AUC for composite endpoint at 1 year |
|---|---|---|---|---|---|
| Verification (ARIC) | 0.750 (0.694, 0.797) | 0.747 (0.622, 0.844) | 0.732 (0.5, 1) | 0.726 (0.674, 0.774) | 0.747 (0.654, 0.853) |

Validation

Validation of the model was assessed on the 20% of the PHFS and 80% of the AZHF data that were not used in the model development. A secondary validation data set is 50% of ARIV visit 5. The predictions are the survival probability at one year. Minimum values of 0.7 for the C-index and AUC are required in order to pass validation.

Validation results are shown in Table 15 below, along with training and verification results for comparison. The AUC at 1 year (365 days) and 6 months (180 days), and the C-Index are shown in Table 15 for the training, verification, and validation sets. All validation metrics are higher than required to pass validation (C-Index > 0.7 and AUC > 0.7 at one year and six months).

TABLE 15

| | Data set | C-Index (95% CI) | AUC (95% CI) (1 year) | AUC (95% CI) (180 days) |
|---|---|---|---|---|
| Training | 80% BMS Penn | 0.834 (0.791, 0.876) | 0.850 (0.787, 0.908) | 0.839 (0.768, 0.902) |
| Verification | 20% AZHF | 0.819 (0.742, 0.895) | 0.855 (0.696, 0.982) | 0.810 (0.5, 0.895) |
| | 50% ARIC visit 5 with HFpEF | 0.750 (0.694, 0.797) | 0.747 (0.622, 0.844) | 0.732 (0.5, 1) |
| Validation | Total Combined Validation Dataset | 0.761 (0.725, 0.792) | 0.807 (0.734, 0.871) | 0.800 (0.725, 0.876) |
| | 20% BMS Penn | 0.796 (0.667, 0.916) | 0.912 (0.791, 0.991) | 0.896 (0.733, 1) |
| | 80% AZHF | 0.789 (0.747, 0.830) | 0.825 (0.738, 0.883) | 0.830 (0.758, 0.902) |
| | 50% ARIC visit 5 with HFpEF | 0.701 (0.646, 0.763) | 0.682 (0.416, 0.900) | 0.626 (0.453, 0.901) |

In addition to discrimination metrics (C-Index and AUC), the fit of the predictive model on the observed event rate was observed in the training and validation datasets. Using the fitted AFT model, the survival probability was predicted for each respective sample at each discrete timepoint from 0 to 365 days.

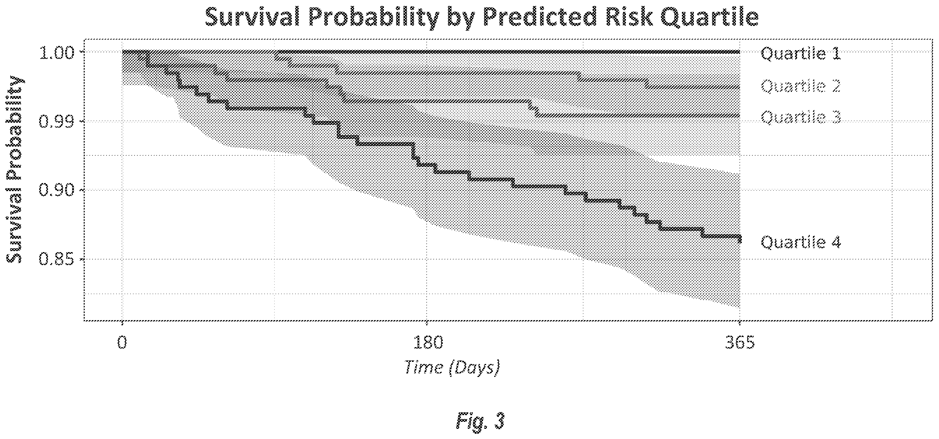

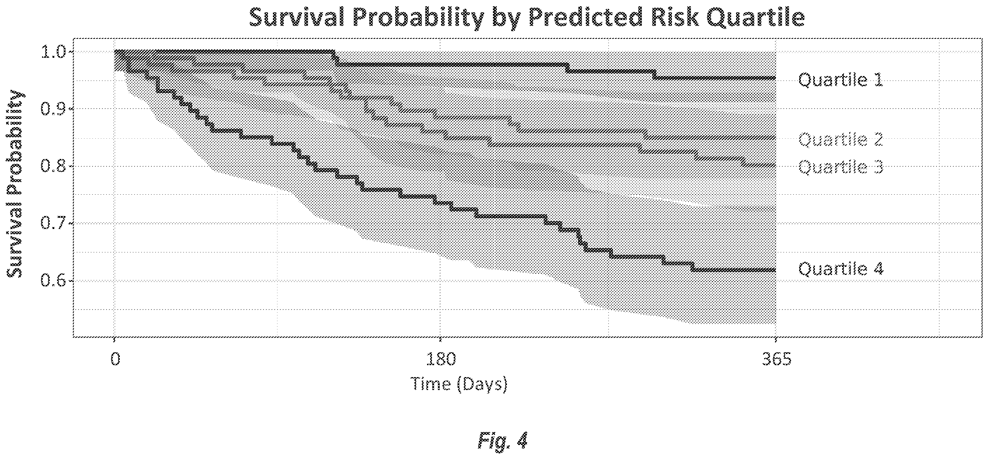

Figs. 3 and 4 show the observed Kaplan-Meier probability of the validation and training datasets, respectively, when stratified by predicted risk quartiles at 365 days. The lines are separated at 180 and 365 days as expected for a well-performing model. Furthermore, the lines do not cross after -45 days, which is another indicator of a model behaving as expected.

The interference testing data were evaluated for putative interference using the final model. Only albumin, hemoglobin, and valsartan failed the first step of interference testing at 365 days and 180 days, but none of them demonstrably affected the model output performance metrics for 180 or 365-days. The final model has 14 features and predicts either the 365-day or 180-day mortality risk in HFpEF patients. The model output is a risk probability from 0 to 1. It meets or exceeds validation criteria of C-Index and AUC at the respective timepoints in the validation dataset.

**Example 5: Analysis of HFpEF Biomarker Panel Model**

Model biomarker panels comprising various combinations of the biomarkers listed in Table 2 were analyzed to determine the C-index for all causes of death within one year of each panel. Tables 16A and 16B below show the model results when various combinations comprising 1 to 8 biomarker proteins were measured. The results in Table 16A show that panels comprising at least GDF15 and RET or CHRDL1 performed adequately, with a C-index above 0.700. Furthermore, panels comprising at least two to thirteen of GDF15, WFDC2, CLEC3B, GHR, MUC16, NPPB, IGFBP2, CCNS, TNNT2, HSPB6, SLPI, MMP12, RET, and CHRDL1 performed adequately. The results in Table 16A and 16B show that panels comprising at least three of GDF15, WFDC2, CLEC3B, GHR, MUC16, NPPB, IGFBP2, CCNS, TNNT2, HSPB6, SLPI, MMP12, RET, and CHRDL1 performed adequately, with a C-index above 0.700.

**Table 16A: Performance of panels comprising proteins expressed from the indicated genes**

| Gene | GDF15 | WFDC2 | CLEC3B | GHR | MUC16 | NPPB | IGFBP2 | CCN5 | TNNT2 | HSPB6 | SLPI | MMP12 | C-index |
|---|---|---|---|---|---|---|---|---|---|---|---|---|---|
| RET | GDF15 | WFDC2 | CLEC3B | GHR | MUC16 | NPPB | IGFBP2 | CCN5 | TNNT2 | HSPB6 | SLPI | MMP12 | 0.834 |
| RET | GDF15 | WFDC2 | CLEC3B | GHR | MUC16 | NPPB | IGFBP2 | CCN5 | TNNT2 | HSPB6 | SLPI | | 0.832 |
| RET | GDF15 | WFDC2 | CLEC3B | GHR | MUC16 | NPPB | IGFBP2 | CCN5 | TNNT2 | HSPB6 | | | 0.828 |
| RET | GDF15 | WFDC2 | CLEC3B | GHR | MUC16 | NPPB | IGFBP2 | CCN5 | TNNT2 | | | | 0.827 |
| RET | GDF15 | WFDC2 | CLEC3B | GHR | MUC16 | NPPB | | | | | | | 0.825 |
| RET | GDF15 | WFDC2 | CLEC3B | GHR | MUC16 | NPPB | IGFBP2 | | | | | | 0.825 |
| RET | GDF15 | WFDC2 | CLEC3B | GHR | MUC16 | NPPB | IGFBP2 | CCN5 | | | | | 0.823 |
| RET | GDF15 | WFDC2 | CLEC3B | GHR | MUC16 | | | | | | | | 0.816 |
| RET | GDF15 | WFDC2 | CLEC3B | GHR | | | | | | | | | 0.812 |
| RET | GDF15 | WFDC2 | CLEC3B | | | | | | | | | | 0.807 |
| RET | GDF15 | WFDC2 | | | | | | | | | | | 0.785 |
| RET | GDF15 | | | | | | | | | | | | 0.778 |
| CHRDL1 | GDF15 | WFDC2 | CLEC3B | GHR | MUC16 | NPPB | IGFBP2 | CCN5 | TNNT2 | HSPB6 | SLPI | MMP12 | 0.835 |
| CHRDL1 | GDF15 | WFDC2 | CLEC3B | GHR | MUC16 | NPPB | IGFBP2 | CCN5 | TNNT2 | HSPB6 | SLPI | | 0.834 |
| CHRDL1 | GDF15 | WFDC2 | CLEC3B | GHR | MUC16 | NPPB | IGFBP2 | CCN5 | TNNT2 | | | | 0.831 |
| CHRDL1 | GDF15 | WFDC2 | CLEC3B | GHR | MUC16 | NPPB | IGFBP2 | CCN5 | TNNT2 | HSPB6 | | | 0.830 |
| CHRDL1 | GDF15 | WFDC2 | CLEC3B | GHR | MUC16 | NPPB | IGFBP2 | | | | | | 0.826 |
| CHRDL1 | GDF15 | WFDC2 | CLEC3B | GHR | MUC16 | NPPB | IGFBP2 | CCN5 | | | | | 0.825 |
| CHRDL1 | GDF15 | WFDC2 | CLEC3B | GHR | MUC16 | NPPB | | | | | | | 0.825 |
| CHRDL1 | GDF15 | WFDC2 | CLEC3B | GHR | MUC16 | | | | | | | | 0.820 |
| CHRDL1 | GDF15 | WFDC2 | CLEC3B | GHR | | | | | | | | | 0.816 |
| CHRDL1 | GDF15 | WFDC2 | CLEC3B | | | | | | | | | | 0.811 |
| CHRDL1 | GDF15 | WFDC2 | | | | | | | | | | | 0.782 |
| CHRDL1 | GDF15 | | | | | | | | | | | | 0.775 |

**Table 16B: Performance of panels comprising proteins expressed from the indicated genes**

| RET | CHRDL1 | GDF15 | WFDC2 | CLEC3B | GHR | MUC16 | NPPB | IGFBP2 | CCN5 | TNNT2 | HSPB6 | SLPI | MMP12 | C-index |
|---|---|---|---|---|---|---|---|---|---|---|---|---|---|---|
| RET | CHRDL1 | GDF15 | WFDC2 | CLEC3B | GHR | MUC16 | NPPB | IGFBP2 | WISP2 | TNNT2 | HSPB6 | SLPI | MMP12 | 0.834 |
| RET | CHRDL1 | GDF15 | WFDC2 | CLEC3B | GHR | MUC16 | NPPB | IGFBP2 | WISP2 | TNNT2 | HSPB6 | SLPI | | 0.832 |
| RET | CHRDL1 | GDF15 | WFDC2 | CLEC3B | GHR | MUC16 | NPPB | IGFBP2 | WISP2 | TNNT2 | | | | 0.829 |
| RET | CHRDL1 | GDF15 | WFDC2 | CLEC3B | GHR | MUC16 | NPPB | IGFBP2 | WISP2 | TNNT2 | HSPB6 | | | 0.828 |
| RET | CHRDL1 | GDF15 | WFDC2 | CLEC3B | GHR | MUC16 | NPPB | IGFBP2 | | | | | | 0.824 |
| RET | CHRDL1 | GDF15 | WFDC2 | CLEC3B | GHR | MUC16 | NPPB | | | | | | | 0.824 |
| RET | CHRDL1 | GDF15 | WFDC2 | CLEC3B | GHR | MUC16 | NPPB | IGFBP2 | WISP2 | | | | | 0.823 |
| RET | CHRDL1 | GDF15 | WFDC2 | CLEC3B | GHR | MUC16 | | | | | | | | 0.816 |
| RET | CHRDL1 | GDF15 | WFDC2 | CLEC3B | GHR | | | | | | | | | 0.813 |
| RET | CHRDL1 | GDF15 | WFDC2 | CLEC3B | | | | | | | | | | 0.810 |
| RET | CHRDL1 | GDF15 | WFDC2 | | | | | | | | | | | 0.784 |
| RET | CHRDL1 | GDF15 | | | | | | | | | | | | 0.779 |

The following numbered paragraphs, describing aspects of our proposals, are part of the description:

1. A method for screening a subject for the risk of a cardiovascular event (CV) event, comprising forming a biomarker panel having N biomarker proteins, and detecting the level of each of the N biomarker proteins in a sample from the subject, wherein N is at least 2, and wherein a) at least two of the N biomarker proteins are selected from HCC-1, RNAS6, PAP1, SVEP1, and ATL2; or b) at least one of the N biomarker proteins is selected from HCC-1, RNAS6, PAP1, SVEP1, and ATL2, and at least one of the N biomarker proteins is selected from N-terminal pro-BNP, RSPO4, BNP, MIC-1, FABPA, ILRL1, ANGP2, HE4, TAGL, RNAS1, and TSP2.

2. A method of predicting the likelihood that a subject will have a CV event, comprising forming a biomarker panel having N biomarker proteins, and detecting the level of each of the N biomarker proteins in a sample from the subject, wherein N is at least 2, and wherein a) at least two of the N biomarker proteins are selected from HCC-1, RNAS6, PAP1, SVEP1, and ATL2; or b) at least one of the N biomarker proteins is selected from HCC-1, RNAS6, PAP1, SVEP1, and ATL2, and at least one of the N biomarker proteins is selected from N-terminal pro-BNP, RSPO4, BNP, MIC-1, FABPA, ILRL1, ANGP2, HE4, TAGL, RNAS1, and TSP2.

3. The method of clause 1 or clause 2, wherein at least two of the N biomarker proteins are RNAS6 and PAP1.

4. The method of clause 1 or clause 2, wherein at least two of the N biomarker proteins are RNAS6 and ATL2.

5. The method of clause 1 or clause 2, wherein at least two of the N biomarker proteins are HCC-1 and PAP1.

6. The method of clause 1 or clause 2, wherein at least two of the N biomarker proteins are HCC-1 and ATL2.

7. The method of clause 1 or clause 2, wherein at least two of the N biomarker proteins are HCC-1 and RNAS6.

8. The method of clause 1 or clause 2, wherein at least two of the N biomarker proteins are PAP1 and SVEP1.

9. The method of clause 1 or clause 2, wherein at least two of the N biomarker proteins are HCC-1 and SVEP1.

10. The method of clause 1 or clause 2, wherein at least two of the N biomarker proteins are RNAS6 and SVEP1.

11. The method of clause 1 or clause 2, wherein at least two of the N biomarker proteins are PAP1 and ATL2.

12. The method of any one of clauses 1-11, wherein all of the N biomarker proteins are selected from HCC-1, RNAS6, PAP1, SVEP1, ATL2, N-terminal pro-BNP, RSPO4, BNP, MIC-1, FABPA, ILRL1, ANGP2, HE4, TAGL, RNAS1, and TSP2.

13. The method of any one of clauses 1-12, wherein one of the N biomarker proteins is MIC-1.

14. The method of any one of clauses 1-13, wherein one of the N biomarker proteins is RNAS1.

15. The method of any one of clauses 1 to 14, wherein N is 2, N is 3, N is 4, N is 5, N is 6, N is 7, N is 8, N is 9, N is 10, N is is 11, N is is 12, N is is 13, N is is 14, N is is 15, or N is is 16.

16. The method of any one of clauses 1-15, wherein the subject has heart failure with reduced ejection fraction.

17. A method for screening a subject for the risk of a a cardiovascular event (CV) event, comprising forming a biomarker panel having N biomarker proteins, and detecting the level of each of the N biomarker proteins in a sample from the subject, wherein N is at least 2, wherein at least one of the N biomarker proteins is selected from RET and CRDL1, and at least one of the N biomarker proteins is selected from Tetranectin, N-terminal pro-BNP, TNNT2, CA125, MIC-1, SLPI, HE4, MMP-12, HSPB6, WISP-2, GHR, and IGFBP-2.

18. A method of predicting the likelihood that a subject will have a CV event, comprising forming a biomarker panel having N biomarker proteins, and detecting the level of each of the N biomarker proteins in a sample from the subject, wherein N is at least 2, wherein at least one of the N biomarker proteins is selected from RET and CRDL1, and at least one of the N biomarker proteins is selected from Tetranectin, N-terminal pro-BNP, TNNT2, CA125, MIC-1, SLPI, HE4, MMP-12, HSPB6, WISP-2, GHR, and IGFBP-2.

19. The method of clause 17 or clause 18, wherein at least two of the N biomarker proteins are RET and CRDL1.

20. The method of any one of clauses 17-19, wherein one of the N biomarker proteins is MIC-1.

21. The method of any one of clauses 17-20, wherein one of the N biomarker proteins is HE4.

22. The method of any one of clauses 17-21, wherein one of the N biomarker proteins is Tetranectin.

23. The method of any one of clauses 17-22, wherein one of the N biomarker proteins is GHR.

24. The method of any one of clauses 17-23, wherein one of the N biomarker proteins is CA125.

25. The method of any one of clauses 17-24, wherein one of the N biomarker proteins is N-terminal pro-BNP.

26. The method of any one of clauses 17-25, wherein one of the N biomarker proteins is IGFBP-2.

27. The method of any one of clauses 17-26, wherein one of the N biomarker proteins is WISP-2.

28. The method of any one of clauses 17-27, wherein one of the N biomarker proteins is TNNT2.

29. The method of any one of clauses 17-28, wherein one of the N biomarker proteins is HSPB6.

30. The method of any one of clauses 17-29, wherein one of the N biomarker proteins is SLPI.

31. The method of any one of clauses 17-30, wherein one of the N biomarker proteins is MMP-12.

32. The method of any one of clauses 17-31, wherein all of the N biomarker proteins are selected from RET, CRDL1, Tetranectin, N-terminal pro-BNP, TNNT2, CA125, MIC-1, SLPI, HE4, MMP-12, HSPB6, WISP-2, GHR, and IGFBP-2.

33. The method of any one of clauses 17 to 32, wherein N is 2, N is 3, N is 4, N is 5, N is 6, N is 7, N is 8, N is 9, N is 10, N is 11, N is 12, N is 13, or N is 14.

34. The method of any one of clauses 17-33, wherein the subject has heart failure with preserved ejection fraction.

35. The method of any one of clauses 1-34, wherein the CV event is death.

36. The method of any one of clauses 1-35, wherein the risk or likelihood of the subject having a CV event within 1 year from the date that the sample was taken from the subject is screened or predicted.

37. The method of any one of clauses 1-35, wherein the risk or likelihood of the subject having a CV event within 180 days from the date that the sample was taken from the subject is screened or predicted.

38. The method of any one of clauses 1-35, wherein the risk or likelihood of the subject having a CV event within 90 days from the date that the sample was taken from the subject is screened or predicted.

39. The method of any one of clauses 36-38, wherein the risk or likelihood of the subject having a CV within 1 year, 180 days, or 90 days from the date that the sample was taken from the subject is high if the levels of each of at least 2 of the N biomarker proteins are abnormal relative to a control level of the respective biomarker protein.

40. The method of any one of clauses 36-38, wherein the risk or likelihood of the subject having a CV within 1 year, 180 days, or 90 days from the date that the sample was taken from the subject is high if the levels of each of the N biomarker proteins are abnormal relative to a control level of the respective biomarker protein.

41. The method of clause any one of claims 36-38, wherein the risk or likelihood of the subject having a CV event within 1 year, 180 days, or 90 days from the date that the sample was taken from the subject is calculated as a probability of survival 1 year, 180 days, or 90 days from the date that the sample was taken from the subject.

42. The method of any one of clauses 1-41, wherein the sample is selected from a blood sample, a serum sample, a plasma sample, and a urine sample.

43. The method of clause 42, wherein the sample is a blood sample.

44. The method of any one of clauses 1-43, wherein the method is performed *in vitro.*

45. The method of any one of clauses 1-44, wherein the method comprises contacting biomarker proteins of the sample from the subject with a set of capture reagents, wherein each capture reagent of the set of capture reagents specifically binds to one biomarker protein being detected.

46. The method of clause 45, wherein two of the capture reagents bind to the same biomarker protein being detected.

47. The method of clause 46, wherein two capture reagents specifically bind to SVEP1, and wherein the two capture reagents are aptamers comprising different sequences.

48. The method of any one of clauses 1-47, wherein the method comprises contacting biomarker proteins of the sample from the subject with a set of capture reagents, wherein each capture reagent of the set of capture reagents specifically binds to a different biomarker protein being detected.

49. The method of any one of clauses 45-48, wherein each capture reagent is an antibody or an aptamer.

50. The method of clause 49, wherein each biomarker capture reagent is an aptamer.

51. The method of clause 50, wherein at least one aptamer is a slow off-rate aptamer.

52. The method of clause 51, wherein at least one slow off-rate aptamer comprises at least one, at least two, at least three, at least four, at least five, at least six, at least seven, at least eight, at least nine, or at least 10 nucleotides with modifications.

53. The method of clause 51 or clause 52, wherein each slow off-rate aptamer binds to its target protein with an off rate (ty2) of $\geq 30$ minutes, $\geq 60$ minutes, $\geq 90$ minutes, $\geq 120$ minutes, $\geq 150$ minutes, $\geq 180$ minutes, $\geq 210$ minutes, or $\geq 240$ minutes.

54. The method of any one of the preceding clauses, wherein the risk or likelihood of a CV event is based on the detected biomarker levels and at least one item of additional biomedical information selected from

a) information corresponding to physical descriptors of the subject,
b) information corresponding to a change in weight of the subject,
c) information corresponding to the ethnicity of the subject,
d) information corresponding to the gender of the subject,
e) information corresponding to the subject's smoking history,
f) information corresponding to the subject's alcohol use history,
g) information corresponding to the subject's occupational history,
h) information corresponding to the subject's family history of cardiovascular disease or other circulatory system conditions,
i) information corresponding to the presence or absence in the subject of at least one genetic marker correlating with a higher risk of cardiovascular disease in the subject or a family member of the subject,
j) information corresponding to clinical symptoms of the subject,
k) information corresponding to other laboratory tests,
l) information corresponding to gene expression values of the subject, and
m) information corresponding to the subject's consumption of known cardiovascular risk factors such as diet high in saturated fats, high salt, high cholesterol,

n) information corresponding to the subject's imaging results obtained by techniques selected from the group consisting of electrocardiogram, echocardiography, carotid ultrasound for intima-media thickness, flow mediated dilation, pulse wave velocity, ankle-brachial index, stress echocardiography, myocardial perfusion imaging, coronary calcium by CT, high resolution CT angiography, MRI imaging, and other imaging modalities,

o) information regarding the subject's medications,

p) information corresponding to the age of the subject, and

q) information regarding the subject's kidney function.

55. The method of clause 54, wherein the at least one item of additional biomedical information is information corresponding to the age of the subject.

56. The method of any one of the preceding claims, wherein the method comprises determining the risk or likelihood of a CV event for the purpose of determining a medical insurance premium or life insurance premium.

57. The method of clause 56, wherein the method further comprises determining coverage or premium for medical insurance or life insurance.

58. The method of any one of clauses 1-57, wherein the method further comprises using information resulting from the method to predict and/or manage the utilization of medical resources.

59. The method of any one of clauses 1-58, wherein the method further comprises using information resulting from the method to enable a decision to acquire or purchase a medical practice, hospital, or company.

60. A kit comprising N biomarker protein capture reagents, wherein N is at least 2, and wherein at least one of the capture reagents binds to HCC-1, RNAS6, PAP1, SVEP1, or ATL2, and at least one of the capture reagents binds to N-terminal pro-BNP, RSPO4, BNP, MIC-1, FABPA, ILRL1, ANGP2, HE4, TAGL, RNAS1, or TSP2.

61. The kit of clause 60, wherein two of the capture reagents bind to SVEP1 and each of the remaining capture reagents binds to a different protein selected from HCC-1, RNAS6, PAP1, ATL2, N-terminal pro-BNP, RSPO4, BNP, MIC-1, FABPA, ILRL1, ANGP2, HE4, TAGL, RNAS1, and TSP2.

62. A kit comprising N biomarker protein capture reagents, wherein N is at least 2, and wherein at least one of the capture reagents binds to RET or CRDL1, and at least one of the capture reagents binds to Tetranectin, N-terminal pro-BNP, TNNT2, CA125, MIC-1, SLPI, HE4, MMP-12, HSPB6, WISP-2, GHR, or IGFBP-2.

63. The kit of clause 62, wherein each capture reagent binds to a different biomarker protein.

64. The kit of clause 60 or clause 61, wherein N is 2, N is 3, N is 4, N is 5, N is 6, N is 7, N is 8, N is 9, N is 10, N is is 11, N is is 12, N is is 13, N is is 14, N is is 15, N is is 16, or N is 17.

65. The kit of clause 62 or clause 63, wherein N is 2, N is 3, or N is 4, or N is 5, or N is 6, or N is 7, or N is 8, or N is 9, or N is 10, or N is 11, or N is 12, or N is 13, or N is 14.

66. The kit of any one of clauses 60, 61, and 64, wherein each of the N biomarker protein capture reagents specifically binds to a biomarker protein selected from Table 1.

67. The kit of any one of clauses 62, 63, or 65, wherein each of the N biomarker protein capture reagents specifically binds to a biomarker protein selected form Table 2.

68. The kit of any one of clauses 60-67, wherein each of the N biomarker capture reagents is an antibody or an aptamer.

69. The kit of clause 68, wherein each biomarker capture reagent is an aptamer.

70. The kit of clause 69, wherein at least one aptamer is a slow off-rate aptamer.

71. The kit of clause 70, wherein at least one slow off-rate aptamer comprises at least one, at least two, at least three, at least four, at least five, at least six, at least seven, at least eight, at least nine, or at least 10 nucleotides with modifications.

72. The kit of clause 70 or clause 71, wherein each slow off-rate aptamer binds to its target protein with an off rate (tv2) of > 30 minutes, > 60 minutes, > 90 minutes, > 120 minutes, > 150 minutes, > 180 minutes, > 210 minutes, or > 240 minutes.

73. The kit of any one of clauses 60-72, for use in detecting the N biomarker proteins in a sample from a subject.

74. The kit of clause 73, for use in determining the subject's risk or likelihood of experiencing a CV event within 1 year from the date that the sample was taken from the subject, wherein the subject has heart failure.

75. The kit of clause 74, wherein the CV event is death.

76. The kit of clause 74 or 75, wherein the subject has heart failure with reduced ejection fraction.

77. The kit of clause 74 or 75, wherein the subject has heart failure with preserved ejection fraction.

## Claims

1. A method for screening a subject for the risk of a cardiovascular (CV) event or predicting the likelihood that a subject will have a CV event, comprising forming a biomarker panel having N biomarker proteins, and detecting the level of each of the N biomarker proteins in a sample from the subject, wherein N is at least 2, and wherein a) at least two of the N biomarker proteins are selected from RNAS6, PAP1, ATL2, SVEP1, and HCC-1; or b) at least one of the N biomarker proteins is selected from RNAS6, PAP1, ATL2, SVEP1, and HCC-1 and at least one of the N biomarker proteins is selected from N-terminal pro-BNP, RSPO4, BNP, MIC-1, FABPA, ILRL1, ANGP2, HE4, TAGL, RNAS1, and TSP2.

2. The method of claim 1, wherein at least two of the N biomarker proteins are RNAS6 and PAP1; or

   wherein at least two of the N biomarker proteins are RNAS6 and ATL2; or
   wherein at least two of the N biomarker proteins are HCC-1 and PAP1; or
   wherein at least two of the N biomarker proteins are HCC-1 and ATL2; or
   wherein at least two of the N biomarker proteins are HCC-1 and RNAS6; or
   wherein at least two of the N biomarker proteins are PAP1 and SVEP1; or
   wherein at least two of the N biomarker proteins are HCC-1 and SVEP1; or
   wherein at least two of the N biomarker proteins are RNAS6 and SVEP1; or
   wherein at least two of the N biomarker proteins are PAP1 and ATL2; or
   wherein one of the N biomarker proteins is MIC-1; or
   wherein one of the N biomarker proteins is RNAS1.

3. The method of claim 1 or claim 2, wherein all of the N biomarker proteins are selected from HCC-1, RNAS6, PAP1, SVEP1, ATL2, N-terminal pro-BNP, RSPO4, BNP, MIC-1, FABPA, ILRL1, ANGP2, HE4, TAGL, RNAS1, and TSP2.

4. The method of any one of claims 1-3, wherein N is 2, N is 3, N is 4, N is 5, N is 6, N is 7, N is 8, N is 9, N is 10, N is 11, N is 12, N is 13, N is 14, N is 15, or N is 16.

5. The method of any one of claims 1-4, wherein the subject has heart failure with reduced ejection fraction.

6. The method of any one of claims 1-5, wherein the risk or likelihood of the subject having a CV event within 1 year from the date that the sample was taken from the subject is screened or predicted; or wherein the risk or likelihood of the subject having a CV event within 180 days from the date that the sample was taken from the subject is screened or predicted; or wherein the risk or likelihood of the subject having a CV event within 90 days from the date that the sample was taken from the subject is screened or predicted.

7. The method of claim 6, wherein the risk or likelihood of the subject having a CV within 1 year, 180 days, or 90 days from the date that the sample was taken from the subject is high if the levels of each of at least 2 of the N biomarker proteins are abnormal relative to a control level of the respective biomarker protein, optionally wherein the risk or likelihood of the subject having a CV within 1 year, 180 days, or 90 days from the date that the sample was taken from the subject is high if the levels of each of the N biomarker proteins are abnormal relative to a control level of the respective biomarker protein.

8. The method of claim 6 or claim 7, wherein the risk or likelihood of the subject having a CV event within 1 year, 180 days, or 90 days from the date that the sample was taken from the subject is calculated as a probability of survival 1 year, 180 days, or 90 days from the date that the sample was taken from the subject.

9. The method of any one of claims 1-8, wherein the sample is selected from a blood sample, a serum sample, a plasma sample, and a urine sample.

10. The method of any one of claims 1-9, wherein the method comprises contacting biomarker proteins of the sample from the subject with a set of capture reagents, wherein each capture reagent of the set of capture reagents specifically binds to one biomarker protein being detected.

11. The method of claim 10, wherein each capture reagent is an antibody or an aptamer.

12. The method of claim 11, wherein each biomarker capture reagent is an aptamer.

13. The method of claim 12, wherein at least one aptamer is a slow off-rate aptamer.

14. The method of claim 13, wherein at least one slow off-rate aptamer comprises at least one, at least two, at least three, at least four, at least five, at least six, at least seven, at least eight, at least nine, or at least ten nucleotides with modifications.

15. The method of claim 13 or claim 14, wherein each slow off-rate aptamer binds to its target protein with an off rate (t½) of ? 30 minutes, ? 60 minutes, ? 90 minutes, ? 120 minutes, ? 150 minutes, ? 180 minutes, ? 210 minutes, or ? 240 minutes.

Survival Probability by Predicted Risk Quartile

Fig. 1

EP 4 614 156 A2

*Fig. 2A*

EP 4 614 156 A2

**Survival Probability by Predicted Risk Quartile**

*Fig. 2B*

EP 4 614 156 A2

Fig. 3

EP 4 614 156 A2

**Survival Probability by Predicted Risk Quartile**

*Fig. 4*

Fig. 5

* *Aptamer binds protein in solution*

* *Aptamer/protein complex captured on beads*
* **Protein tagging reaction**
* **Anionic kinetic challenge**
* **Removes free proteins**

* **Photocleavage release from beads**

* **Protein capture magnetic beads**
* **Removes free aptamers**

* **Release bound aptamer and detect**

Fig. 6

CYTOSINE

URACIL

~~~~ - denotes attachment to backbone of nucleic acid molecules (e.g., phosphate deoxyribose backbone)

X - denotes a linker attached to the 5-position of the pyrimidine molecule (uracil or cytosine)

R' – denotes a moiety attached to the 5-position of the pyrimidine molecule via linker (X)

*Fig. 7*

Exemplary Linkers (Denoted as X in Figure 7)
n = 0, 1, 2, 3, 4, 5, 6, 7, 8, 9 or 10; R' denotes a moiety
∿∿∿ -denotes attachment to the 5-position of the pyrimidine molecule (uracil or cytosine)

*Fig. 7 (cont.)*

Exemplary Linkers (Denoted as X in Figure 7)
n = 0, 1, 2, 3, 4, 5, 6, 7, 8, 9 or 10; R' denotes a moiety
-denotes attachment to the 5-position of the pyrimidine molecule (uracil or cytosine)

*Fig. 7 (cont.)*

| Group | R' Structures (* denotes the point of attachment of the R' group to X (linker)) |
|-------|-------------------------------------------------------------------------------------|
| I | |
| II | |
| III | |
| IV | |
| V | |
| VI | |
| VII | |
| VIII | |
| IX | |
| X | |

*Fig. 7 (cont.)*

| Group | R' Structures (* denotes the point of attachment of the R' group to X (linker)) |
|-------|----------------------------------------------------------------------------------|
| XI | |
| XII | |
| XIII | |
| XIV | |
| XV | |
| XVI | |
| XVII | |
| XVIII | |
| XIX | |
| XX | |

R" and R'" are, independently, selected from a halogen (-F, -Cl, -Br, -I); nitrile (-CN); -NH$_2$; -OH; branched lower alkyl (C1-C20); linear lower alkyl (C1-C20); primary amide; secondary amide; tertiary amide; alkoxy group.

*Fig. 7 (cont.)*

| Abbreviation | 5-dU Modification Attached via Amide Linkage | Chemical Structure |
|---|---|---|
| Bn | benzylmethyl | |
| Nap | 1-naphthylmethyl | |
| PE | 2-phenylethyl | |
| PP | 3-phenylpropyl | |
| iBu | iso-butyl | |
| FBn | 4-fluorobenzylmethyl | |
| 2Nap | 2-naphthylmethyl | |
| Tyr | tyrosyl | |
| NE | 1-naphthylethyl | |
| MBn | 3,4-methylenedioxy benzyl | |
| MOE | morpholinoethyl | |

*Fig. 8*

| Abbreviation | 5-dU Modification Attached via Amide Linkage | Chemical Structure |
|---|---|---|
| *BF* | 3-benzofuranylethyl | |
| *BT* | 3-benzothiophenylethyl | |
| *Trp* | 3-indole-2-ethyl | |
| *Thr* | (R)-2-hydroxypropyl | |

*Fig. 8 (cont.)*

Fig. 8 (cont.)

NEdU

MBndU

MOEdU

BFdU

BTdU

TrpdU

ThrdU

*Fig. 8 (cont.)*

| Abbreviation | 5-dC Modification Attached via Amide Linkage | Chemical Structure |
|---|---|---|
| Bn | benzylmethyl | |
| Nap | 1-naphthylmethyl | |
| PE | 2-phenylethyl | |
| PP | 3-phenylpropyl | |
| 2Nap | 2-naphthylmethyl | |
| Tyr | tyrosyl | |
| NE | 1-naphthylethyl | |
| 2NE | 2-naphthyl-2-ethyl | |

Fig. 9

Fig. 9 (cont.)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5475096 A
- US 6242246 B
- US 6458543 B
- US 6503715 B
- US 5705337 A
- US 5660985 A
- US 5580737 A
- US 20090098549
- US 20090004667 A
- US 6544776 B
- US 5763177 A
- US 6001577 A
- US 6291184 B
- US 6458539 B
- US 20090042206 A
- US 20120101002 A
- US 20120077695 A

**Non-patent literature cited in the description**

- **D'AGOSTINO, R et al.** General Cardiovascular Risk Profile for Use in Primary Care: The Framingham Heart Study. *Circulation*, 2008, vol. 117, 743-53
- **RIDKER, P. et al.** Development and Validation of Improved Algorithms for the Assessment of Global Cardiovascular Risk in Women. *JAMA*, 2007, vol. 297 (6), 611-619
- **SHLIPAK, M. et al.** Biomarkers to Predict Recurrent Cardiovascular Disease: The Heart & Soul Study. *Am. J. Med.*, 2008, vol. 121, 50-57
- **WANG et al.** Multiple Biomarkers for the Prediction of First Major Cardiovascular Events and Death. *N. Eng. J. Med.*, 2006, vol. 355, 2631-2637
- **YANCY et al.** 2013 ACCF/AHA guideline for the management of heart failure: A report of the American college of cardiology foundation/American heart association task force on practice guidelines. *J Amer College Cardiol*, 2013, vol. 62, e147-e239
- **PONIKOWSKI P** ; **VOORS A** ; **ANKER S et al.** 2016 ESC Guidelines for the diagnosis and treatment of acute and chronic heart failure. *Eur J Heart Fail*, 2016, vol. 37, 2129-200
- **AMOS et al.** *Nature Genetics*, 2009, vol. 40, 616622
- Principles of Fluorescence Spectroscopy. Springer Science + Business Media, Inc., 2004
- Bioluminescence & Chemiluminescence: Progress & Current Applications. World Scientific Publishing Company, January 2002
- **KRAEMER et al.** *PLoS One*, 2011, vol. 6 (10), e26332
- ImmunoAssay: A Practical Guide. Taylor & Francis, Ltd., 2005
- Gene Expression Profiling: Methods and Protocols. Humana Press, 2004
- **N. BLOW**. *Nature Methods*, 2009, vol. 6, 465-469
- **BURLINGAME et al.** *Anal. Chem.*, 1998, vol. 70, 647 R-716R
- Pattern Classification. John Wiley & Sons, 2001
- The Elements of Statistical Learning - Data Mining, Inference, and Prediction. Springer Science+Business Media, 2009
- **FAWCETT T**. An introduction to ROC analysis. *Pattern Recognition Letters*, 2006, vol. 27, 861-874
- **HANLEY, J.A.** ; **MCNEIL, B.J.** The meaning and use of the area under a receiver operating characteristic (ROC) curve. *Radiology*, 1982, vol. 143, 29-36
- **PENCINA et al.** *Stat. Med.*, 2011, vol. 30, 11-21
- **SAMBROOK et al.** Molecular Cloning: A Laboratory Manual. Cold Spring Harbor Laboratory Press, 2001
- **KRAEMER et al.** *PLoS One*, vol. 6 (10), e26332